# EUROPEAN PATENT APPLICATION

(11) **EP 3 954 393 A1**
(43) Date of publication of application: **16.02.2022**
(21) Application number: 21191109.4
(22) Date of filing: 12.08.2021
(51) Int. Cl.: A61K 47/64, A61K 47/68, A61P 35/00, A61P 29/00, A61P 37/00, A61K 39/395

(54) **COMBINATION THERAPIES FOR DELIVERY ACROSS THE BLOOD BRAIN BARRIER**

(30) Priority: 13.08.2020 US 202063065492 P
(71) Applicant: Bioasis Technologies Inc., Vancouver, British Columbia V6C 3L2 (CA)
(72) Inventor: GABATHULER, Reinhard, London (GB); TIAN, Mei Mei, Maple Ridge (CA)
(74) Representative: Prüfer & Partner mbB Patentanwälte · Rechtsanwälte

(57) **Abstract**

Provided are compositions and therapeutic uses related to the co-administration of an immunoglobulin Fc region-containing polypeptide and a central nervous system (CNS)-targeted antibody or Fc-fusion polypeptide conjugate, to improve pharmacokinetics and/or delivery of the conjugate across the blood-brain barrier (BBB). Also provided are uses of treatment of indications and diseases of the CNS and indications and diseases with a CNS component.

SEQUENCE LISTING

<110> BIOASIS TECHNOLOGIES, INC.

<120> COMBINATION THERAPIES FOR DELIVERY ACROSS THE BLOOD BRAIN BARRIER

<130> 1569553.111EP2

<140>
<141>

<150> 63/065,492
<151> 2020-08-13

<160> 278

<170> PatentIn version 3.5

<210> 1
<211> 738
<212> PRT
<213> Homo sapiens

<400> 1
Met Arg Gly Pro Ser Gly Ala Leu Trp Leu Leu Leu Ala Leu Arg Thr
1               5                   10                  15

Val Leu Gly Gly Met Glu Val Arg Trp Cys Ala Thr Ser Asp Pro Glu
            20                  25                  30

Gln His Lys Cys Gly Asn Met Ser Glu Ala Phe Arg Glu Ala Gly Ile
        35                  40                  45

Gln Pro Ser Leu Leu Cys Val Arg Gly Thr Ser Ala Asp His Cys Val
    50                  55                  60

Gln Leu Ile Ala Ala Gln Glu Ala Asp Ala Ile Thr Leu Asp Gly Gly
65                  70                  75                  80

Ala Ile Tyr Glu Ala Gly Lys Glu His Gly Leu Lys Pro Val Val Gly
                85                  90                  95

Glu Val Tyr Asp Gln Glu Val Gly Thr Ser Tyr Tyr Ala Val Ala Val
            100                 105                 110

Val Arg Arg Ser Ser His Val Thr Ile Asp Thr Leu Lys Gly Val Lys
        115                 120                 125

Ser Cys His Thr Gly Ile Asn Arg Thr Val Gly Trp Asn Val Pro Val
    130                 135                 140

Gly Tyr Leu Val Glu Ser Gly Arg Leu Ser Val Met Gly Cys Asp Val
145                 150                 155                 160

Leu Lys Ala Val Ser Asp Tyr Phe Gly Gly Ser Cys Val Pro Gly Ala
                165                 170                 175

Gly Glu Thr Ser Tyr Ser Glu Ser Leu Cys Arg Leu Cys Arg Gly Asp
            180                 185                 190

Ser Ser Gly Glu Gly Val Cys Asp Lys Ser Pro Leu Glu Arg Tyr Tyr
        195                 200                 205

Asp Tyr Ser Gly Ala Phe Arg Cys Leu Ala Glu Gly Ala Gly Asp Val
    210                 215                 220

Ala Phe Val Lys His Ser Thr Val Leu Glu Asn Thr Asp Gly Lys Thr
225                 230                 235                 240

Leu Pro Ser Trp Gly Gln Ala Leu Leu Ser Gln Asp Phe Glu Leu Leu
                245                 250                 255

Cys Arg Asp Gly Ser Arg Ala Asp Val Thr Glu Trp Arg Gln Cys His
            260                 265                 270

Leu Ala Arg Val Pro Ala His Ala Val Val Val Arg Ala Asp Thr Asp
        275                 280                 285

Gly Gly Leu Ile Phe Arg Leu Leu Asn Glu Gly Gln Arg Leu Phe Ser
    290                 295                 300

His Glu Gly Ser Ser Phe Gln Met Phe Ser Ser Glu Ala Tyr Gly Gln
305                 310                 315                 320

Lys Asp Leu Leu Phe Lys Asp Ser Thr Ser Glu Leu Val Pro Ile Ala
                325                 330                 335

Thr Gln Thr Tyr Glu Ala Trp Leu Gly His Glu Tyr Leu His Ala Met
            340                 345                 350

Lys Gly Leu Leu Cys Asp Pro Asn Arg Leu Pro Pro Tyr Leu Arg Trp
        355                 360                 365

Cys Val Leu Ser Thr Pro Glu Ile Gln Lys Cys Gly Asp Met Ala Val
    370                 375                 380

Ala Phe Arg Arg Gln Arg Leu Lys Pro Glu Ile Gln Cys Val Ser Ala
385                 390                 395                 400

Lys Ser Pro Gln His Cys Met Glu Arg Ile Gln Ala Glu Gln Val Asp

                405                 410                 415

Ala Val Thr Leu Ser Gly Glu Asp Ile Tyr Thr Ala Gly Lys Thr Tyr
            420                 425                 430

Gly Leu Val Pro Ala Ala Gly Glu His Tyr Ala Pro Glu Asp Ser Ser
        435                 440                 445

Asn Ser Tyr Tyr Val Val Ala Val Val Arg Arg Asp Ser Ser His Ala
    450                 455                 460

Phe Thr Leu Asp Glu Leu Arg Gly Lys Arg Ser Cys His Ala Gly Phe
465                 470                 475                 480

Gly Ser Pro Ala Gly Trp Asp Val Pro Val Gly Ala Leu Ile Gln Arg
                485                 490                 495

Gly Phe Ile Arg Pro Lys Asp Cys Asp Val Leu Thr Ala Val Ser Glu
            500                 505                 510

Phe Phe Asn Ala Ser Cys Val Pro Val Asn Asn Pro Lys Asn Tyr Pro
        515                 520                 525

Ser Ser Leu Cys Ala Leu Cys Val Gly Asp Glu Gln Gly Arg Asn Lys
    530                 535                 540

Cys Val Gly Asn Ser Gln Glu Arg Tyr Tyr Gly Tyr Arg Gly Ala Phe
545                 550                 555                 560

Arg Cys Leu Val Glu Asn Ala Gly Asp Val Ala Phe Val Arg His Thr
                565                 570                 575

Thr Val Phe Asp Asn Thr Asn Gly His Asn Ser Glu Pro Trp Ala Ala
            580                 585                 590

Glu Leu Arg Ser Glu Asp Tyr Glu Leu Leu Cys Pro Asn Gly Ala Arg
        595                 600                 605

Ala Glu Val Ser Gln Phe Ala Ala Cys Asn Leu Ala Gln Ile Pro Pro
    610                 615                 620

His Ala Val Met Val Arg Pro Asp Thr Asn Ile Phe Thr Val Tyr Gly
625                 630                 635                 640

Leu Leu Asp Lys Ala Gln Asp Leu Phe Gly Asp Asp His Asn Lys Asn
                645                 650                 655

Gly Phe Lys Met Phe Asp Ser Ser Asn Tyr His Gly Gln Asp Leu Leu
            660                 665                 670

Phe Lys Asp Ala Thr Val Arg Ala Val Pro Val Gly Glu Lys Thr Thr
        675                 680                 685

Tyr Arg Gly Trp Leu Gly Leu Asp Tyr Val Ala Ala Leu Glu Gly Met
    690                 695                 700

Ser Ser Gln Gln Cys Ser Gly Ala Ala Ala Pro Ala Pro Gly Ala Pro
705                 710                 715                 720

Leu Leu Pro Leu Leu Leu Pro Ala Leu Ala Ala Arg Leu Leu Pro Pro
                725                 730                 735

Ala Leu

<210> 2
<211> 692
<212> PRT
<213> Homo sapiens

<400> 2
Gly Met Glu Val Arg Trp Cys Ala Thr Ser Asp Pro Glu Gln His Lys
1               5                   10                  15

Cys Gly Asn Met Ser Glu Ala Phe Arg Glu Ala Gly Ile Gln Pro Ser
            20                  25                  30

Leu Leu Cys Val Arg Gly Thr Ser Ala Asp His Cys Val Gln Leu Ile
        35                  40                  45

Ala Ala Gln Glu Ala Asp Ala Ile Thr Leu Asp Gly Gly Ala Ile Tyr
    50                  55                  60

Glu Ala Gly Lys Glu His Gly Leu Lys Pro Val Val Gly Glu Val Tyr
65                  70                  75                  80

Asp Gln Glu Val Gly Thr Ser Tyr Tyr Ala Val Ala Val Val Arg Arg
                85                  90                  95

Ser Ser His Val Thr Ile Asp Thr Leu Lys Gly Val Lys Ser Cys His
            100                 105                 110

Thr Gly Ile Asn Arg Thr Val Gly Trp Asn Val Pro Val Gly Tyr Leu
        115                 120                 125

Val Glu Ser Gly Arg Leu Ser Val Met Gly Cys Asp Val Leu Lys Ala

130                 135                 140

Val Ser Asp Tyr Phe Gly Gly Ser Cys Val Pro Gly Ala Gly Glu Thr
145                 150                 155                 160

Ser Tyr Ser Glu Ser Leu Cys Arg Leu Cys Arg Gly Asp Ser Ser Gly
                165                 170                 175

Glu Gly Val Cys Asp Lys Ser Pro Leu Glu Arg Tyr Tyr Asp Tyr Ser
            180                 185                 190

Gly Ala Phe Arg Cys Leu Ala Glu Gly Ala Gly Asp Val Ala Phe Val
        195                 200                 205

Lys His Ser Thr Val Leu Glu Asn Thr Asp Gly Lys Thr Leu Pro Ser
    210                 215                 220

Trp Gly Gln Ala Leu Leu Ser Gln Asp Phe Glu Leu Leu Cys Arg Asp
225                 230                 235                 240

Gly Ser Arg Ala Asp Val Thr Glu Trp Arg Gln Cys His Leu Ala Arg
                245                 250                 255

Val Pro Ala His Ala Val Val Val Arg Ala Asp Thr Asp Gly Gly Leu
            260                 265                 270

Ile Phe Arg Leu Leu Asn Glu Gly Gln Arg Leu Phe Ser His Glu Gly
        275                 280                 285

Ser Ser Phe Gln Met Phe Ser Ser Glu Ala Tyr Gly Gln Lys Asp Leu
    290                 295                 300

Leu Phe Lys Asp Ser Thr Ser Glu Leu Val Pro Ile Ala Thr Gln Thr
305                 310                 315                 320

Tyr Glu Ala Trp Leu Gly His Glu Tyr Leu His Ala Met Lys Gly Leu
                325                 330                 335

Leu Cys Asp Pro Asn Arg Leu Pro Pro Tyr Leu Arg Trp Cys Val Leu
            340                 345                 350

Ser Thr Pro Glu Ile Gln Lys Cys Gly Asp Met Ala Val Ala Phe Arg
        355                 360                 365

Arg Gln Arg Leu Lys Pro Glu Ile Gln Cys Val Ser Ala Lys Ser Pro
    370                 375                 380

Gln His Cys Met Glu Arg Ile Gln Ala Glu Gln Val Asp Ala Val Thr
385                 390                 395                 400

Leu Ser Gly Glu Asp Ile Tyr Thr Ala Gly Lys Thr Tyr Gly Leu Val
                405                 410                 415

Pro Ala Ala Gly Glu His Tyr Ala Pro Glu Asp Ser Ser Asn Ser Tyr
            420                 425                 430

Tyr Val Val Ala Val Val Arg Arg Asp Ser Ser His Ala Phe Thr Leu
        435                 440                 445

Asp Glu Leu Arg Gly Lys Arg Ser Cys His Ala Gly Phe Gly Ser Pro
    450                 455                 460

Ala Gly Trp Asp Val Pro Val Gly Ala Leu Ile Gln Arg Gly Phe Ile
465                 470                 475                 480

Arg Pro Lys Asp Cys Asp Val Leu Thr Ala Val Ser Glu Phe Phe Asn
                485                 490                 495

Ala Ser Cys Val Pro Val Asn Asn Pro Lys Asn Tyr Pro Ser Ser Leu
            500                 505                 510

Cys Ala Leu Cys Val Gly Asp Glu Gln Gly Arg Asn Lys Cys Val Gly
        515                 520                 525

Asn Ser Gln Glu Arg Tyr Tyr Gly Tyr Arg Gly Ala Phe Arg Cys Leu
    530                 535                 540

Val Glu Asn Ala Gly Asp Val Ala Phe Val Arg His Thr Thr Val Phe
545                 550                 555                 560

Asp Asn Thr Asn Gly His Asn Ser Glu Pro Trp Ala Ala Glu Leu Arg
                565                 570                 575

Ser Glu Asp Tyr Glu Leu Leu Cys Pro Asn Gly Ala Arg Ala Glu Val
            580                 585                 590

Ser Gln Phe Ala Ala Cys Asn Leu Ala Gln Ile Pro Pro His Ala Val
        595                 600                 605

Met Val Arg Pro Asp Thr Asn Ile Phe Thr Val Tyr Gly Leu Leu Asp
    610                 615                 620

Lys Ala Gln Asp Leu Phe Gly Asp Asp His Asn Lys Asn Gly Phe Lys
625                 630                 635                 640

Met Phe Asp Ser Ser Asn Tyr His Gly Gln Asp Leu Leu Phe Lys Asp
                645                 650                 655

Ala Thr Val Arg Ala Val Pro Val Gly Glu Lys Thr Thr Tyr Arg Gly
            660                 665                 670

Trp Leu Gly Leu Asp Tyr Val Ala Ala Leu Glu Gly Met Ser Ser Gln
        675                 680                 685

Gln Cys Ser Gly
    690

<210> 3
<211> 11
<212> PRT
<213> Homo sapiens

<400> 3
Trp Cys Ala Thr Ser Asp Pro Glu Gln His Lys
1               5                   10

<210> 4
<211> 11
<212> PRT
<213> Homo sapiens

<400> 4
Arg Ser Ser His Val Thr Ile Asp Thr Leu Lys
1               5                   10

<210> 5
<211> 13
<212> PRT
<213> Homo sapiens

<400> 5
Ser Ser His Val Thr Ile Asp Thr Leu Lys Gly Val Lys
1               5                   10

<210> 6
<211> 14
<212> PRT
<213> Homo sapiens

<400> 6
Leu Cys Arg Gly Asp Ser Ser Gly Glu Gly Val Cys Asp Lys
1               5                   10

<210> 7
<211> 16
<212> PRT
<213> Homo sapiens

<400> 7

Gly Asp Ser Ser Gly Glu Gly Val Cys Asp Lys Ser Pro Leu Glu Arg
1               5                   10                  15

<210> 8
<211> 9
<212> PRT
<213> Homo sapiens

<400> 8
Tyr Tyr Asp Tyr Ser Gly Ala Phe Arg
1               5

<210> 9
<211> 7
<212> PRT
<213> Homo sapiens

<400> 9
Ala Asp Val Thr Glu Trp Arg
1               5

<210> 10
<211> 9
<212> PRT
<213> Homo sapiens

<400> 10
Val Pro Ala His Ala Val Val Val Arg
1               5

<210> 11
<211> 10
<212> PRT
<213> Homo sapiens

<400> 11
Ala Asp Thr Asp Gly Gly Leu Ile Phe Arg
1               5                   10

<210> 12
<211> 9
<212> PRT
<213> Homo sapiens

<400> 12
Cys Gly Asp Met Ala Val Ala Phe Arg
1               5

<210> 13
<211> 11
<212> PRT
<213> Homo sapiens

<400> 13
Leu Lys Pro Glu Ile Gln Cys Val Ser Ala Lys
1               5                   10

<210> 14
<211> 12
<212> PRT
<213> Unknown

<220>
<221> source
<223> /note="Description of Unknown:
      Various organism designations"

<400> 14
Asp Ser Ser His Ala Phe Thr Leu Asp Glu Leu Arg
1               5                   10


<210> 15
<211> 13
<212> PRT
<213> Homo sapiens

<400> 15
Ser Glu Asp Tyr Glu Leu Leu Cys Pro Asn Gly Ala Arg
1               5                   10


<210> 16
<211> 15
<212> PRT
<213> Homo sapiens

<400> 16
Ala Gln Asp Leu Phe Gly Asp Asp His Asn Lys Asn Gly Phe Lys
1               5                   10                  15


<210> 17
<211> 40
<212> PRT
<213> Homo sapiens

<400> 17
Phe Ser Ser Glu Ala Tyr Gly Gln Lys Asp Leu Leu Phe Lys Asp Ser
1               5                   10                  15


Thr Ser Glu Leu Val Pro Ile Ala Thr Gln Thr Tyr Glu Ala Trp Leu
            20                  25                  30


Gly His Glu Tyr Leu His Ala Met
        35                  40


<210> 18
<211> 221
<212> PRT
<213> Homo sapiens

<400> 18
Glu Arg Ile Gln Ala Glu Gln Val Asp Ala Val Thr Leu Ser Gly Glu
1               5                   10                  15

Asp Ile Tyr Thr Ala Gly Lys Thr Tyr Gly Leu Val Pro Ala Ala Gly
            20                  25                  30

Glu His Tyr Ala Pro Glu Asp Ser Ser Asn Ser Tyr Tyr Val Val Ala
        35                  40                  45

Val Val Arg Arg Asp Ser Ser His Ala Phe Thr Leu Asp Glu Leu Arg
    50                  55                  60

Gly Lys Arg Ser Cys His Ala Gly Phe Gly Ser Pro Ala Gly Trp Asp
65                  70                  75                  80

Val Pro Val Gly Ala Leu Ile Gln Arg Gly Phe Ile Arg Pro Lys Asp
                85                  90                  95

Cys Asp Val Leu Thr Ala Val Ser Glu Phe Phe Asn Ala Ser Cys Val
            100                 105                 110

Pro Val Asn Asn Pro Lys Asn Tyr Pro Ser Ser Leu Cys Ala Leu Cys
        115                 120                 125

Val Gly Asp Glu Gln Gly Arg Asn Lys Cys Val Gly Asn Ser Gln Glu
    130                 135                 140

Arg Tyr Tyr Gly Tyr Arg Gly Ala Phe Arg Cys Leu Val Glu Asn Ala
145                 150                 155                 160

Gly Asp Val Ala Phe Val Arg His Thr Thr Val Phe Asp Asn Thr Asn
                165                 170                 175

Gly His Asn Ser Glu Pro Trp Ala Ala Glu Leu Arg Ser Glu Asp Tyr
            180                 185                 190

Glu Leu Leu Cys Pro Asn Gly Ala Arg Ala Glu Val Ser Gln Phe Ala
        195                 200                 205

Ala Cys Asn Leu Ala Gln Ile Pro Pro His Ala Val Met
    210                 215                 220

<210> 19
<211> 32
<212> PRT
<213> Homo sapiens

<400> 19
Val Arg Pro Asp Thr Asn Ile Phe Thr Val Tyr Gly Leu Leu Asp Lys
1               5                   10                  15

Ala Gln Asp Leu Phe Gly Asp Asp His Asn Lys Asn Gly Phe Lys Met
            20                  25                  30

<210> 20
<211> 323
<212> PRT
<213> Artificial Sequence

<220>
<221> source
<223> /note="Description of Artificial Sequence: Synthetic
      polypeptide"

<400> 20
Tyr Ser Arg Glu Lys Asn Gln Pro Lys Pro Ser Pro Lys Arg Glu Ser
1               5                   10                  15

Gly Glu Glu Phe Arg Met Glu Lys Leu Asn Gln Leu Trp Glu Lys Ala
            20                  25                  30

Gln Arg Leu His Leu Pro Pro Val Arg Leu Ala Glu Leu His Ala Asp
        35                  40                  45

Leu Lys Ile Gln Glu Arg Asp Glu Leu Ala Trp Lys Lys Leu Lys Leu
    50                  55                  60

Asp Gly Leu Asp Glu Asp Gly Glu Lys Glu Ala Arg Leu Ile Arg Asn
65                  70                  75                  80

Leu Asn Val Ile Leu Ala Lys Tyr Gly Leu Asp Gly Lys Lys Asp Ala
                85                  90                  95

Arg Gln Val Thr Ser Asn Ser Leu Ser Gly Thr Gln Glu Asp Gly Leu
            100                 105                 110

Asp Asp Pro Arg Leu Glu Lys Leu Trp His Lys Ala Lys Thr Ser Gly
        115                 120                 125

Lys Phe Ser Gly Glu Glu Leu Asp Lys Leu Trp Arg Glu Phe Leu His
    130                 135                 140

His Lys Glu Lys Val His Glu Tyr Asn Val Leu Leu Glu Thr Leu Ser
145                 150                 155                 160

Arg Thr Glu Glu Ile His Glu Asn Val Ile Ser Pro Ser Asp Leu Ser
                165                 170                 175

Asp Ile Lys Gly Ser Val Leu His Ser Arg His Thr Glu Leu Lys Glu
            180                 185                 190

Lys Leu Arg Ser Ile Asn Gln Gly Leu Asp Arg Leu Arg Arg Val Ser
        195                 200                 205

His Gln Gly Tyr Ser Thr Glu Ala Glu Phe Glu Glu Pro Arg Val Ile
    210                 215                 220

Asp Leu Trp Asp Leu Ala Gln Ser Ala Asn Leu Thr Asp Lys Glu Leu
225                 230                 235                 240

Glu Ala Phe Arg Glu Glu Leu Lys His Phe Glu Ala Lys Ile Glu Lys
                245                 250                 255

His Asn His Tyr Gln Lys Gln Leu Glu Ile Ala His Glu Lys Leu Arg
            260                 265                 270

His Ala Glu Ser Val Gly Asp Gly Glu Arg Val Ser Arg Ser Arg Glu
        275                 280                 285

Lys His Ala Leu Leu Glu Gly Arg Thr Lys Glu Leu Gly Tyr Thr Val
    290                 295                 300

Lys Lys His Leu Gln Asp Leu Ser Gly Arg Ile Ser Arg Ala Arg His
305                 310                 315                 320

Asn Glu Leu

<210> 21
<211> 58
<212> PRT
<213> Artificial Sequence

<220>
<221> source
<223> /note="Description of Artificial Sequence: Synthetic
      polypeptide"

<400> 21

<210> 22
<211> 19
<212> PRT
<213> Artificial Sequence

<220>
<221> source
<223> /note="Description of Artificial Sequence: Synthetic
      peptide"

<400> 22
Thr Phe Val Tyr Gly Gly Cys Arg Ala Lys Arg Asn Asn Phe Lys Ser
1               5                   10                  15

Ala Glu Asp

<210> 23
<211> 19
<212> PRT
<213> Artificial Sequence

<220>
<221> source
<223> /note="Description of Artificial Sequence: Synthetic
      peptide"

<400> 23
Thr Phe Phe Tyr Gly Gly Cys Arg Gly Lys Arg Asn Asn Phe Lys Thr
1               5                   10                  15

Glu Glu Tyr

<210> 24
<211> 19
<212> PRT
<213> Artificial Sequence

<220>
<221> source
<223> /note="Description of Artificial Sequence: Synthetic
      peptide"

<400> 24
Thr Phe Phe Tyr Gly Gly Ser Arg Gly Lys Arg Asn Asn Phe Lys Thr
1               5                   10                  15

Glu Glu Tyr

<210> 25
<211> 19
<212> PRT
<213> Artificial Sequence

<220>

<221> source
<223> /note="Description of Artificial Sequence: Synthetic
      peptide"

<400> 25
Thr Phe Val Tyr Gly Gly Cys Arg Ala Lys Arg Asn Asn Phe Lys Ser
1               5                   10                  15

Ala Glu Asp

<210> 26
<211> 19
<212> PRT
<213> Artificial Sequence

<220>
<221> source
<223> /note="Description of Artificial Sequence: Synthetic
      peptide"

<400> 26
Thr Phe Gln Tyr Gly Gly Cys Met Gly Asn Gly Asn Asn Phe Val Thr
1               5                   10                  15

Glu Lys Glu

<210> 27
<211> 19
<212> PRT
<213> Artificial Sequence

<220>
<221> source
<223> /note="Description of Artificial Sequence: Synthetic
      peptide"

<400> 27
Pro Phe Phe Tyr Gly Gly Cys Gly Gly Asn Arg Asn Asn Phe Asp Thr
1               5                   10                  15

Glu Glu Tyr

<210> 28
<211> 19
<212> PRT
<213> Artificial Sequence

<220>
<221> source
<223> /note="Description of Artificial Sequence: Synthetic
      peptide"

<400> 28
Ser Phe Tyr Tyr Gly Gly Cys Leu Gly Asn Lys Asn Asn Tyr Leu Arg

1               5                   10                  15

Glu Glu Glu

<210> 29
<211> 19
<212> PRT
<213> Artificial Sequence

<220>
<221> source
<223> /note="Description of Artificial Sequence: Synthetic
      peptide"

<400> 29
Thr Phe Phe Tyr Gly Gly Cys Arg Ala Lys Arg Asn Asn Phe Lys Arg
1               5                   10                  15

Ala Lys Tyr

<210> 30
<211> 19
<212> PRT
<213> Artificial Sequence

<220>
<221> source
<223> /note="Description of Artificial Sequence: Synthetic
      peptide"

<400> 30
Thr Phe Phe Tyr Gly Gly Cys Arg Gly Lys Arg Asn Asn Phe Lys Arg
1               5                   10                  15

Ala Lys Tyr

<210> 31
<211> 19
<212> PRT
<213> Artificial Sequence

<220>
<221> source
<223> /note="Description of Artificial Sequence: Synthetic
      peptide"

<400> 31
Thr Phe Phe Tyr Gly Gly Cys Arg Ala Lys Lys Asn Asn Tyr Lys Arg
1               5                   10                  15

Ala Lys Tyr

<210> 32
<211> 19
<212> PRT
<213> Artificial Sequence

<220>
<221> source
<223> /note="Description of Artificial Sequence: Synthetic
      peptide"

<400> 32
Thr Phe Phe Tyr Gly Gly Cys Arg Gly Lys Lys Asn Asn Phe Lys Arg
1               5                   10                  15

Ala Lys Tyr

<210> 33
<211> 19
<212> PRT
<213> Artificial Sequence

<220>
<221> source
<223> /note="Description of Artificial Sequence: Synthetic
      peptide"

<400> 33
Thr Phe Gln Tyr Gly Gly Cys Arg Ala Lys Arg Asn Asn Phe Lys Arg
1               5                   10                  15

Ala Lys Tyr

<210> 34
<211> 19
<212> PRT
<213> Artificial Sequence

<220>
<221> source
<223> /note="Description of Artificial Sequence: Synthetic
      peptide"

<400> 34
Thr Phe Gln Tyr Gly Gly Cys Arg Gly Lys Lys Asn Asn Phe Lys Arg
1               5                   10                  15

Ala Lys Tyr

<210> 35
<211> 19
<212> PRT
<213> Artificial Sequence

<220>
<221> source
<223> /note="Description of Artificial Sequence: Synthetic
      peptide"

<400> 35
Thr Phe Phe Tyr Gly Gly Cys Leu Gly Lys Arg Asn Asn Phe Lys Arg
1               5                   10                  15

Ala Lys Tyr

<210> 36
<211> 19
<212> PRT
<213> Artificial Sequence

<220>
<221> source
<223> /note="Description of Artificial Sequence: Synthetic
      peptide"

<400> 36
Thr Phe Phe Tyr Gly Gly Ser Leu Gly Lys Arg Asn Asn Phe Lys Arg
1               5                   10                  15

Ala Lys Tyr

<210> 37
<211> 19
<212> PRT
<213> Artificial Sequence

<220>
<221> source
<223> /note="Description of Artificial Sequence: Synthetic
      peptide"

<400> 37
Pro Phe Phe Tyr Gly Gly Cys Gly Gly Lys Lys Asn Asn Phe Lys Arg
1               5                   10                  15

Ala Lys Tyr

<210> 38
<211> 19
<212> PRT
<213> Artificial Sequence

<220>
<221> source
<223> /note="Description of Artificial Sequence: Synthetic
      peptide"

<400> 38

Thr Phe Phe Tyr Gly Gly Cys Arg Gly Lys Gly Asn Asn Tyr Lys Arg
1               5                   10                  15

Ala Lys Tyr

<210> 39
<211> 19
<212> PRT
<213> Artificial Sequence

<220>
<221> source
<223> /note="Description of Artificial Sequence: Synthetic
      peptide"

<400> 39
Pro Phe Phe Tyr Gly Gly Cys Arg Gly Lys Arg Asn Asn Phe Leu Arg
1               5                   10                  15

Ala Lys Tyr

<210> 40
<211> 19
<212> PRT
<213> Artificial Sequence

<220>
<221> source
<223> /note="Description of Artificial Sequence: Synthetic
      peptide"

<400> 40
Thr Phe Phe Tyr Gly Gly Cys Arg Gly Lys Arg Asn Asn Phe Lys Arg
1               5                   10                  15

Glu Lys Tyr

<210> 41
<211> 19
<212> PRT
<213> Artificial Sequence

<220>
<221> source
<223> /note="Description of Artificial Sequence: Synthetic
      peptide"

<400> 41
Pro Phe Phe Tyr Gly Gly Cys Arg Ala Lys Lys Asn Asn Phe Lys Arg
1               5                   10                  15

Ala Lys Glu

<210> 42
<211> 19
<212> PRT
<213> Artificial Sequence

<220>
<221> source
<223> /note="Description of Artificial Sequence: Synthetic
      peptide"

<400> 42
Thr Phe Phe Tyr Gly Gly Cys Arg Gly Lys Arg Asn Asn Phe Lys Arg
1               5                   10                  15

Ala Lys Asp

<210> 43
<211> 19
<212> PRT
<213> Artificial Sequence

<220>
<221> source
<223> /note="Description of Artificial Sequence: Synthetic
      peptide"

<400> 43
Thr Phe Phe Tyr Gly Gly Cys Arg Ala Lys Arg Asn Asn Phe Asp Arg
1               5                   10                  15

Ala Lys Tyr

<210> 44
<211> 19
<212> PRT
<213> Artificial Sequence

<220>
<221> source
<223> /note="Description of Artificial Sequence: Synthetic
      peptide"

<400> 44
Thr Phe Phe Tyr Gly Gly Cys Arg Gly Lys Lys Asn Asn Phe Lys Arg
1               5                   10                  15

Ala Glu Tyr

<210> 45
<211> 19
<212> PRT
<213> Artificial Sequence

<220>
<221> source
<223> /note="Description of Artificial Sequence: Synthetic
      peptide"

<400> 45
Pro Phe Phe Tyr Gly Gly Cys Gly Ala Asn Arg Asn Asn Phe Lys Arg
1               5                   10                  15

Ala Lys Tyr

<210> 46
<211> 19
<212> PRT
<213> Artificial Sequence

<220>
<221> source
<223> /note="Description of Artificial Sequence: Synthetic
      peptide"

<400> 46
Thr Phe Phe Tyr Gly Gly Cys Gly Gly Lys Lys Asn Asn Phe Lys Thr
1               5                   10                  15

Ala Lys Tyr

<210> 47
<211> 19
<212> PRT
<213> Artificial Sequence

<220>
<221> source
<223> /note="Description of Artificial Sequence: Synthetic
      peptide"

<400> 47
Thr Phe Phe Tyr Gly Gly Cys Arg Gly Asn Arg Asn Asn Phe Leu Arg
1               5                   10                  15

Ala Lys Tyr

<210> 48
<211> 19
<212> PRT
<213> Artificial Sequence

<220>
<221> source
<223> /note="Description of Artificial Sequence: Synthetic
      peptide"

<400> 48
Thr Phe Phe Tyr Gly Gly Cys Arg Gly Asn Arg Asn Asn Phe Lys Thr
1               5                   10                  15

Ala Lys Tyr

<210> 49
<211> 19
<212> PRT
<213> Artificial Sequence

<220>
<221> source
<223> /note="Description of Artificial Sequence: Synthetic
      peptide"

<400> 49
Thr Phe Phe Tyr Gly Gly Ser Arg Gly Asn Arg Asn Asn Phe Lys Thr
1               5                   10                  15

Ala Lys Tyr

<210> 50
<211> 19
<212> PRT
<213> Artificial Sequence

<220>
<221> source
<223> /note="Description of Artificial Sequence: Synthetic
      peptide"

<400> 50
Thr Phe Phe Tyr Gly Gly Cys Leu Gly Asn Gly Asn Asn Phe Lys Arg
1               5                   10                  15

Ala Lys Tyr

<210> 51
<211> 19
<212> PRT
<213> Artificial Sequence

<220>
<221> source
<223> /note="Description of Artificial Sequence: Synthetic
      peptide"

<400> 51
Thr Phe Phe Tyr Gly Gly Cys Leu Gly Asn Arg Asn Asn Phe Leu Arg
1               5                   10                  15

Ala Lys Tyr

<210> 52
<211> 19
<212> PRT
<213> Artificial Sequence

<220>
<221> source
<223> /note="Description of Artificial Sequence: Synthetic
      peptide"

<400> 52
Thr Phe Phe Tyr Gly Gly Cys Leu Gly Asn Arg Asn Asn Phe Lys Thr
1               5                   10                  15

Ala Lys Tyr

<210> 53
<211> 19
<212> PRT
<213> Artificial Sequence

<220>
<221> source
<223> /note="Description of Artificial Sequence: Synthetic
      peptide"

<400> 53
Thr Phe Phe Tyr Gly Gly Cys Arg Gly Asn Gly Asn Asn Phe Lys Ser
1               5                   10                  15

Ala Lys Tyr

<210> 54
<211> 19
<212> PRT
<213> Artificial Sequence

<220>
<221> source
<223> /note="Description of Artificial Sequence: Synthetic
      peptide"

<400> 54
Thr Phe Phe Tyr Gly Gly Cys Arg Gly Lys Lys Asn Asn Phe Asp Arg
1               5                   10                  15

Glu Lys Tyr

<210> 55
<211> 19
<212> PRT

<213> Artificial Sequence

<220>
<221> source
<223> /note="Description of Artificial Sequence: Synthetic
      peptide"

<400> 55
Thr Phe Phe Tyr Gly Gly Cys Arg Gly Lys Arg Asn Asn Phe Leu Arg
1               5                   10                  15

Glu Lys Glu

<210> 56
<211> 19
<212> PRT
<213> Artificial Sequence

<220>
<221> source
<223> /note="Description of Artificial Sequence: Synthetic
      peptide"

<400> 56
Thr Phe Phe Tyr Gly Gly Cys Arg Gly Lys Gly Asn Asn Phe Asp Arg
1               5                   10                  15

Ala Lys Tyr

<210> 57
<211> 19
<212> PRT
<213> Artificial Sequence

<220>
<221> source
<223> /note="Description of Artificial Sequence: Synthetic
      peptide"

<400> 57
Thr Phe Phe Tyr Gly Gly Ser Arg Gly Lys Gly Asn Asn Phe Asp Arg
1               5                   10                  15

Ala Lys Tyr

<210> 58
<211> 19
<212> PRT
<213> Artificial Sequence

<220>
<221> source
<223> /note="Description of Artificial Sequence: Synthetic
      peptide"

<400> 58
Thr Phe Phe Tyr Gly Gly Cys Arg Gly Asn Gly Asn Asn Phe Val Thr
1               5                   10                  15

Ala Lys Tyr

<210> 59
<211> 19
<212> PRT
<213> Artificial Sequence

<220>
<221> source
<223> /note="Description of Artificial Sequence: Synthetic
      peptide"

<400> 59
Pro Phe Phe Tyr Gly Gly Cys Gly Gly Lys Gly Asn Asn Tyr Val Thr
1               5                   10                  15

Ala Lys Tyr

<210> 60
<211> 19
<212> PRT
<213> Artificial Sequence

<220>
<221> source
<223> /note="Description of Artificial Sequence: Synthetic
      peptide"

<400> 60
Thr Phe Phe Tyr Gly Gly Cys Leu Gly Lys Gly Asn Asn Phe Leu Thr
1               5                   10                  15

Ala Lys Tyr

<210> 61
<211> 19
<212> PRT
<213> Artificial Sequence

<220>
<221> source
<223> /note="Description of Artificial Sequence: Synthetic
      peptide"

<400> 61
Ser Phe Phe Tyr Gly Gly Cys Leu Gly Asn Lys Asn Asn Phe Leu Thr
1               5                   10                  15

Ala Lys Tyr

<210> 62
<211> 19
<212> PRT
<213> Artificial Sequence

<220>
<221> source
<223> /note="Description of Artificial Sequence: Synthetic
      peptide"

<400> 62
Thr Phe Phe Tyr Gly Gly Cys Gly Gly Asn Lys Asn Asn Phe Val Arg
1               5                   10                  15

Glu Lys Tyr

<210> 63
<211> 12
<212> PRT
<213> Artificial Sequence

<220>
<221> source
<223> /note="Description of Artificial Sequence: Synthetic
      peptide"

<400> 63
Leu Gly Asn Lys Asn Asn Tyr Val Arg Glu Lys Tyr
1               5                   10

<210> 64
<211> 19
<212> PRT
<213> Artificial Sequence

<220>
<221> source
<223> /note="Description of Artificial Sequence: Synthetic
      peptide"

<400> 64
Thr Phe Phe Tyr Gly Gly Cys Gly Gly Asn Gly Asn Asn Phe Leu Thr
1               5                   10                  15

Ala Lys Tyr

<210> 65
<211> 19
<212> PRT
<213> Artificial Sequence

<220>

<221> source
<223> /note="Description of Artificial Sequence: Synthetic
      peptide"

<400> 65
Thr Phe Phe Tyr Gly Gly Cys Arg Gly Asn Arg Asn Asn Phe Leu Thr
1               5                   10                  15

Ala Glu Tyr

<210> 66
<211> 19
<212> PRT
<213> Artificial Sequence

<220>
<221> source
<223> /note="Description of Artificial Sequence: Synthetic
      peptide"

<400> 66
Thr Phe Phe Tyr Gly Gly Cys Arg Gly Asn Gly Asn Asn Phe Lys Ser
1               5                   10                  15

Ala Glu Tyr

<210> 67
<211> 19
<212> PRT
<213> Artificial Sequence

<220>
<221> source
<223> /note="Description of Artificial Sequence: Synthetic
      peptide"

<400> 67
Pro Phe Phe Tyr Gly Gly Cys Leu Gly Asn Lys Asn Asn Phe Lys Thr
1               5                   10                  15

Ala Glu Tyr

<210> 68
<211> 19
<212> PRT
<213> Artificial Sequence

<220>
<221> source
<223> /note="Description of Artificial Sequence: Synthetic
      peptide"

<400> 68
Thr Phe Phe Tyr Gly Gly Cys Arg Gly Asn Arg Asn Asn Phe Lys Thr

1               5                   10                  15

Glu Glu Tyr

<210> 69
<211> 19
<212> PRT
<213> Artificial Sequence

<220>
<221> source
<223> /note="Description of Artificial Sequence: Synthetic
      peptide"

<400> 69
Thr Phe Phe Tyr Gly Gly Cys Arg Gly Lys Arg Asn Asn Phe Lys Thr
1               5                   10                  15

Glu Glu Asp

<210> 70
<211> 19
<212> PRT
<213> Artificial Sequence

<220>
<221> source
<223> /note="Description of Artificial Sequence: Synthetic
      peptide"

<400> 70
Pro Phe Phe Tyr Gly Gly Cys Gly Gly Asn Gly Asn Asn Phe Val Arg
1               5                   10                  15

Glu Lys Tyr

<210> 71
<211> 19
<212> PRT
<213> Artificial Sequence

<220>
<221> source
<223> /note="Description of Artificial Sequence: Synthetic
      peptide"

<400> 71
Ser Phe Phe Tyr Gly Gly Cys Met Gly Asn Gly Asn Asn Phe Val Arg
1               5                   10                  15

Glu Lys Tyr

<210> 72
<211> 19
<212> PRT
<213> Artificial Sequence

<220>
<221> source
<223> /note="Description of Artificial Sequence: Synthetic
      peptide"

<400> 72
Pro Phe Phe Tyr Gly Gly Cys Gly Gly Asn Gly Asn Asn Phe Leu Arg
1               5                   10                  15

Glu Lys Tyr

<210> 73
<211> 19
<212> PRT
<213> Artificial Sequence

<220>
<221> source
<223> /note="Description of Artificial Sequence: Synthetic
      peptide"

<400> 73
Thr Phe Phe Tyr Gly Gly Cys Leu Gly Asn Gly Asn Asn Phe Val Arg
1               5                   10                  15

Glu Lys Tyr

<210> 74
<211> 19
<212> PRT
<213> Artificial Sequence

<220>
<221> source
<223> /note="Description of Artificial Sequence: Synthetic
      peptide"

<400> 74
Ser Phe Phe Tyr Gly Gly Cys Leu Gly Asn Gly Asn Asn Tyr Leu Arg
1               5                   10                  15

Glu Lys Tyr

<210> 75
<211> 19
<212> PRT
<213> Artificial Sequence

<220>
<221> source
<223> /note="Description of Artificial Sequence: Synthetic
      peptide"

<400> 75
Thr Phe Phe Tyr Gly Gly Ser Leu Gly Asn Gly Asn Asn Phe Val Arg
1               5                   10                  15

Glu Lys Tyr

<210> 76
<211> 19
<212> PRT
<213> Artificial Sequence

<220>
<221> source
<223> /note="Description of Artificial Sequence: Synthetic
      peptide"

<400> 76
Thr Phe Phe Tyr Gly Gly Cys Arg Gly Asn Gly Asn Asn Phe Val Thr
1               5                   10                  15

Ala Glu Tyr

<210> 77
<211> 19
<212> PRT
<213> Artificial Sequence

<220>
<221> source
<223> /note="Description of Artificial Sequence: Synthetic
      peptide"

<400> 77
Thr Phe Phe Tyr Gly Gly Cys Leu Gly Lys Gly Asn Asn Phe Val Ser
1               5                   10                  15

Ala Glu Tyr

<210> 78
<211> 19
<212> PRT
<213> Artificial Sequence

<220>
<221> source
<223> /note="Description of Artificial Sequence: Synthetic
      peptide"

<400> 78

Thr Phe Phe Tyr Gly Gly Cys Leu Gly Asn Arg Asn Asn Phe Asp Arg
1               5                   10                  15

Ala Glu Tyr

<210> 79
<211> 19
<212> PRT
<213> Artificial Sequence

<220>
<221> source
<223> /note="Description of Artificial Sequence: Synthetic
      peptide"

<400> 79
Thr Phe Phe Tyr Gly Gly Cys Leu Gly Asn Arg Asn Asn Phe Leu Arg
1               5                   10                  15

Glu Glu Tyr

<210> 80
<211> 19
<212> PRT
<213> Artificial Sequence

<220>
<221> source
<223> /note="Description of Artificial Sequence: Synthetic
      peptide"

<400> 80
Thr Phe Phe Tyr Gly Gly Cys Leu Gly Asn Lys Asn Asn Tyr Leu Arg
1               5                   10                  15

Glu Glu Tyr

<210> 81
<211> 19
<212> PRT
<213> Artificial Sequence

<220>
<221> source
<223> /note="Description of Artificial Sequence: Synthetic
      peptide"

<400> 81
Pro Phe Phe Tyr Gly Gly Cys Gly Gly Asn Arg Asn Asn Tyr Leu Arg
1               5                   10                  15

Glu Glu Tyr

<210> 82
<211> 19
<212> PRT
<213> Artificial Sequence

<220>
<221> source
<223> /note="Description of Artificial Sequence: Synthetic
      peptide"

<400> 82
Pro Phe Phe Tyr Gly Gly Ser Gly Gly Asn Arg Asn Asn Tyr Leu Arg
1               5                   10                  15

Glu Glu Tyr

<210> 83
<211> 19
<212> PRT
<213> Artificial Sequence

<220>
<221> source
<223> /note="Description of Artificial Sequence: Synthetic
      peptide"

<400> 83
Met Arg Pro Asp Phe Cys Leu Glu Pro Pro Tyr Thr Gly Pro Cys Val
1               5                   10                  15

Ala Arg Ile

<210> 84
<211> 21
<212> PRT
<213> Artificial Sequence

<220>
<221> source
<223> /note="Description of Artificial Sequence: Synthetic
      peptide"

<400> 84
Ala Arg Ile Ile Arg Tyr Phe Tyr Asn Ala Lys Ala Gly Leu Cys Gln
1               5                   10                  15

Thr Phe Val Tyr Gly
            20

<210> 85
<211> 22
<212> PRT
<213> Artificial Sequence

<220>
<221> source
<223> /note="Description of Artificial Sequence: Synthetic
      peptide"

<400> 85
Tyr Gly Gly Cys Arg Ala Lys Arg Asn Asn Tyr Lys Ser Ala Glu Asp
1               5                   10                  15

Cys Met Arg Thr Cys Gly
            20

<210> 86
<211> 22
<212> PRT
<213> Artificial Sequence

<220>
<221> source
<223> /note="Description of Artificial Sequence: Synthetic
      peptide"

<400> 86
Pro Asp Phe Cys Leu Glu Pro Pro Tyr Thr Gly Pro Cys Val Ala Arg
1               5                   10                  15

Ile Ile Arg Tyr Phe Tyr
            20

<210> 87
<211> 19
<212> PRT
<213> Artificial Sequence

<220>
<221> source
<223> /note="Description of Artificial Sequence: Synthetic
      peptide"

<400> 87
Thr Phe Phe Tyr Gly Gly Cys Arg Gly Lys Arg Asn Asn Phe Lys Thr
1               5                   10                  15

Glu Glu Tyr

<210> 88
<211> 19
<212> PRT
<213> Artificial Sequence

<220>
<221> source
<223> /note="Description of Artificial Sequence: Synthetic
      peptide"

<400> 88
Lys Phe Phe Tyr Gly Gly Cys Arg Gly Lys Arg Asn Asn Phe Lys Thr
1               5                   10                  15

Glu Glu Tyr

<210> 89
<211> 19
<212> PRT
<213> Artificial Sequence

<220>
<221> source
<223> /note="Description of Artificial Sequence: Synthetic
      peptide"

<400> 89
Thr Phe Tyr Tyr Gly Gly Cys Arg Gly Lys Arg Asn Asn Tyr Lys Thr
1               5                   10                  15

Glu Glu Tyr

<210> 90
<211> 19
<212> PRT
<213> Artificial Sequence

<220>
<221> source
<223> /note="Description of Artificial Sequence: Synthetic
      peptide"

<400> 90
Thr Phe Phe Tyr Gly Gly Ser Arg Gly Lys Arg Asn Asn Phe Lys Thr
1               5                   10                  15

Glu Glu Tyr

<210> 91
<211> 20
<212> PRT
<213> Artificial Sequence

<220>
<221> source
<223> /note="Description of Artificial Sequence: Synthetic
      peptide"

<400> 91
Cys Thr Phe Phe Tyr Gly Cys Cys Arg Gly Lys Arg Asn Asn Phe Lys
1               5                   10                  15

Thr Glu Glu Tyr

            20

<210> 92
<211> 20
<212> PRT
<213> Artificial Sequence

<220>
<221> source
<223> /note="Description of Artificial Sequence: Synthetic
      peptide"

<400> 92
Thr Phe Phe Tyr Gly Gly Cys Arg Gly Lys Arg Asn Asn Phe Lys Thr
1               5                   10                  15

Glu Glu Tyr Cys
            20

<210> 93
<211> 19
<212> PRT
<213> Artificial Sequence

<220>
<221> source
<223> /note="Description of Artificial Sequence: Synthetic
      peptide"

<400> 93
Thr Phe Phe Tyr Gly Gly Lys Arg Gly Lys Arg Asn Asn Phe Lys Thr
1               5                   10                  15

Glu Glu Tyr

<210> 94
<211> 19
<212> PRT
<213> Artificial Sequence

<220>
<221> source
<223> /note="Description of Artificial Sequence: Synthetic
      peptide"

<400> 94
Thr Phe Phe Tyr Gly Gly Cys Arg Gly Lys Arg Asn Asn Phe Lys Thr
1               5                   10                  15

Lys Arg Tyr

<210> 95
<211> 19
<212> PRT

<213> Artificial Sequence

<220>
<221> source
<223> /note="Description of Artificial Sequence: Synthetic
      peptide"

<400> 95
Thr Phe Phe Tyr Gly Gly Lys Arg Gly Lys Arg Asn Asn Phe Lys Thr
1               5                   10                  15

Ala Glu Tyr

<210> 96
<211> 19
<212> PRT
<213> Artificial Sequence

<220>
<221> source
<223> /note="Description of Artificial Sequence: Synthetic
      peptide"

<400> 96
Thr Phe Phe Tyr Gly Gly Lys Arg Gly Lys Arg Asn Asn Phe Lys Arg
1               5                   10                  15

Ala Gly Tyr

<210> 97
<211> 19
<212> PRT
<213> Artificial Sequence

<220>
<221> source
<223> /note="Description of Artificial Sequence: Synthetic
      peptide"

<400> 97
Thr Phe Phe Tyr Gly Gly Lys Arg Gly Lys Arg Asn Asn Phe Lys Arg
1               5                   10                  15

Glu Lys Tyr

<210> 98
<211> 19
<212> PRT
<213> Artificial Sequence

<220>
<221> source
<223> /note="Description of Artificial Sequence: Synthetic
      peptide"

<400> 98
Thr Phe Phe Tyr Gly Gly Lys Arg Gly Lys Arg Asn Asn Phe Lys Thr
1               5                   10                  15

Ala Lys Tyr

<210> 99
<211> 19
<212> PRT
<213> Artificial Sequence

<220>
<221> source
<223> /note="Description of Artificial Sequence: Synthetic
      peptide"

<400> 99
Thr Phe Phe Tyr Gly Gly Cys Leu Gly Asn Arg Asn Asn Phe Lys Thr
1               5                   10                  15

Glu Glu Tyr

<210> 100
<211> 19
<212> PRT
<213> Artificial Sequence

<220>
<221> source
<223> /note="Description of Artificial Sequence: Synthetic
      peptide"

<400> 100
Thr Phe Phe Tyr Gly Cys Gly Arg Gly Lys Arg Asn Asn Phe Lys Thr
1               5                   10                  15

Glu Glu Tyr

<210> 101
<211> 19
<212> PRT
<213> Artificial Sequence

<220>
<221> source
<223> /note="Description of Artificial Sequence: Synthetic
      peptide"

<400> 101
Thr Phe Phe Tyr Gly Gly Arg Cys Gly Lys Arg Asn Asn Phe Lys Thr
1               5                   10                  15

Glu Glu Tyr

<210> 102
<211> 20
<212> PRT
<213> Artificial Sequence

<220>
<221> source
<223> /note="Description of Artificial Sequence: Synthetic
      peptide"

<400> 102
Thr Phe Phe Tyr Gly Gly Cys Leu Gly Asn Gly Asn Asn Phe Asp Thr
1               5                   10                  15

Glu Glu Glu Gln
            20

<210> 103
<211> 19
<212> PRT
<213> Artificial Sequence

<220>
<221> source
<223> /note="Description of Artificial Sequence: Synthetic
      peptide"

<400> 103
Thr Phe Gln Tyr Gly Gly Cys Arg Gly Lys Arg Asn Asn Phe Lys Thr
1               5                   10                  15

Glu Glu Tyr

<210> 104
<211> 19
<212> PRT
<213> Artificial Sequence

<220>
<221> source
<223> /note="Description of Artificial Sequence: Synthetic
      peptide"

<400> 104
Tyr Asn Lys Glu Phe Gly Thr Phe Asn Thr Lys Gly Cys Glu Arg Gly
1               5                   10                  15

Tyr Arg Phe

<210> 105
<211> 19

<212> PRT
<213> Artificial Sequence

<220>
<221> source
<223> /note="Description of Artificial Sequence: Synthetic
      peptide"

<400> 105
Arg Phe Lys Tyr Gly Gly Cys Leu Gly Asn Met Asn Asn Phe Glu Thr
1               5                   10                  15

Leu Glu Glu

<210> 106
<211> 19
<212> PRT
<213> Artificial Sequence

<220>
<221> source
<223> /note="Description of Artificial Sequence: Synthetic
      peptide"

<400> 106
Arg Phe Lys Tyr Gly Gly Cys Leu Gly Asn Lys Asn Asn Phe Leu Arg
1               5                   10                  15

Leu Lys Tyr

<210> 107
<211> 19
<212> PRT
<213> Artificial Sequence

<220>
<221> source
<223> /note="Description of Artificial Sequence: Synthetic
      peptide"

<400> 107
Arg Phe Lys Tyr Gly Gly Cys Leu Gly Asn Lys Asn Asn Tyr Leu Arg
1               5                   10                  15

Leu Lys Tyr

<210> 108
<211> 22
<212> PRT
<213> Artificial Sequence

<220>
<221> source
<223> /note="Description of Artificial Sequence: Synthetic

peptide"

<400> 108
Lys Thr Lys Arg Lys Arg Lys Lys Gln Arg Val Lys Ile Ala Tyr Glu
1               5                   10                  15

Glu Ile Phe Lys Asn Tyr
            20

<210> 109
<211> 15
<212> PRT
<213> Artificial Sequence

<220>
<221> source
<223> /note="Description of Artificial Sequence: Synthetic
      peptide"

<400> 109
Lys Thr Lys Arg Lys Arg Lys Lys Gln Arg Val Lys Ile Ala Tyr
1               5                   10                  15

<210> 110
<211> 17
<212> PRT
<213> Artificial Sequence

<220>
<221> source
<223> /note="Description of Artificial Sequence: Synthetic
      peptide"

<400> 110
Arg Gly Gly Arg Leu Ser Tyr Ser Arg Arg Phe Ser Thr Ser Thr Gly
1               5                   10                  15

Arg

<210> 111
<211> 10
<212> PRT
<213> Artificial Sequence

<220>
<221> source
<223> /note="Description of Artificial Sequence: Synthetic
      peptide"

<400> 111
Arg Arg Leu Ser Tyr Ser Arg Arg Arg Phe
1               5                   10

<210> 112
<211> 16
<212> PRT

<213> Artificial Sequence

<220>
<221> source
<223> /note="Description of Artificial Sequence: Synthetic
      peptide"

<400> 112
Arg Gln Ile Lys Ile Trp Phe Gln Asn Arg Arg Met Lys Trp Lys Lys
1               5                   10                  15


<210> 113
<211> 19
<212> PRT
<213> Artificial Sequence

<220>
<221> source
<223> /note="Description of Artificial Sequence: Synthetic
      peptide"

<400> 113
Thr Phe Phe Tyr Gly Gly Ser Arg Gly Lys Arg Asn Asn Phe Lys Thr
1               5                   10                  15


Glu Glu Tyr



<210> 114
<211> 59
<212> PRT
<213> Artificial Sequence

<220>
<221> source
<223> /note="Description of Artificial Sequence: Synthetic
      polypeptide"

<400> 114
Met Arg Pro Asp Phe Cys Leu Glu Pro Pro Tyr Thr Gly Pro Cys Val
1               5                   10                  15


Ala Arg Ile Ile Arg Tyr Phe Tyr Asn Ala Lys Ala Gly Leu Cys Gln
            20                  25                  30


Thr Phe Val Tyr Gly Gly Cys Arg Ala Lys Arg Asn Asn Phe Lys Ser
        35                  40                  45


Ala Glu Asp Cys Met Arg Thr Cys Gly Gly Ala
    50                  55


<210> 115
<211> 19
<212> PRT
<213> Artificial Sequence

<220>
<221> source
<223> /note="Description of Artificial Sequence: Synthetic
      peptide"

<400> 115
Thr Phe Phe Tyr Gly Gly Cys Arg Gly Lys Arg Asn Asn Phe Lys Thr
1               5                   10                  15

Lys Glu Tyr

<210> 116
<211> 19
<212> PRT
<213> Artificial Sequence

<220>
<221> source
<223> /note="Description of Artificial Sequence: Synthetic
      peptide"

<400> 116
Thr Phe Phe Tyr Gly Gly Cys Arg Ala Lys Arg Asn Asn Phe Lys Arg
1               5                   10                  15

Ala Lys Tyr

<210> 117
<211> 35
<212> PRT
<213> Artificial Sequence

<220>
<221> source
<223> /note="Description of Artificial Sequence: Synthetic
      polypeptide"

<400> 117
Asn Ala Lys Ala Gly Leu Cys Gln Thr Phe Val Tyr Gly Gly Cys Leu
1               5                   10                  15

Ala Lys Arg Asn Asn Phe Glu Ser Ala Glu Asp Cys Met Arg Thr Cys
            20                  25                  30

Gly Gly Ala
        35

<210> 118
<211> 24
<212> PRT
<213> Artificial Sequence

<220>
<221> source

<223> /note="Description of Artificial Sequence: Synthetic
      peptide"

<400> 118
Tyr Gly Gly Cys Arg Ala Lys Arg Asn Asn Phe Lys Ser Ala Glu Asp
1               5                   10                  15

Cys Met Arg Thr Cys Gly Gly Ala
            20

<210> 119
<211> 22
<212> PRT
<213> Artificial Sequence

<220>
<221> source
<223> /note="Description of Artificial Sequence: Synthetic
      peptide"

<400> 119
Gly Leu Cys Gln Thr Phe Val Tyr Gly Gly Cys Arg Ala Lys Arg Asn
1               5                   10                  15

Asn Phe Lys Ser Ala Glu
            20

<210> 120
<211> 20
<212> PRT
<213> Artificial Sequence

<220>
<221> source
<223> /note="Description of Artificial Sequence: Synthetic
      peptide"

<400> 120
Leu Cys Gln Thr Phe Val Tyr Gly Gly Cys Glu Ala Lys Arg Asn Asn
1               5                   10                  15

Phe Lys Ser Ala
            20

<210> 121
<211> 19
<212> PRT
<213> Artificial Sequence

<220>
<221> source
<223> /note="Description of Artificial Sequence: Synthetic
      peptide"

<400> 121
Thr Phe Phe Tyr Gly Gly Ser Arg Gly Lys Arg Asn Asn Phe Lys Thr
1               5                   10                  15

Glu Glu Tyr

<210> 122
<211> 19
<212> PRT
<213> Artificial Sequence

<220>
<221> source
<223> /note="Description of Artificial Sequence: Synthetic
      peptide"

<400> 122
Arg Phe Phe Tyr Gly Gly Ser Arg Gly Lys Arg Asn Asn Phe Lys Thr
1               5                   10                  15

Glu Glu Tyr

<210> 123
<211> 19
<212> PRT
<213> Artificial Sequence

<220>
<221> source
<223> /note="Description of Artificial Sequence: Synthetic
      peptide"

<400> 123
Arg Phe Phe Tyr Gly Gly Ser Arg Gly Lys Arg Asn Asn Phe Lys Thr
1               5                   10                  15

Glu Glu Tyr

<210> 124
<211> 19
<212> PRT
<213> Artificial Sequence

<220>
<221> source
<223> /note="Description of Artificial Sequence: Synthetic
      peptide"

<400> 124
Arg Phe Phe Tyr Gly Gly Ser Arg Gly Lys Arg Asn Asn Phe Arg Thr
1               5                   10                  15

Glu Glu Tyr

<210> 125
<211> 19
<212> PRT
<213> Artificial Sequence

<220>
<221> source
<223> /note="Description of Artificial Sequence: Synthetic
      peptide"

<400> 125
Thr Phe Phe Tyr Gly Gly Ser Arg Gly Lys Arg Asn Asn Phe Arg Thr
1               5                   10                  15

Glu Glu Tyr

<210> 126
<211> 19
<212> PRT
<213> Artificial Sequence

<220>
<221> source
<223> /note="Description of Artificial Sequence: Synthetic
      peptide"

<400> 126
Thr Phe Phe Tyr Gly Gly Ser Arg Gly Arg Arg Asn Asn Phe Arg Thr
1               5                   10                  15

Glu Glu Tyr

<210> 127
<211> 20
<212> PRT
<213> Artificial Sequence

<220>
<221> source
<223> /note="Description of Artificial Sequence: Synthetic
      peptide"

<400> 127
Cys Thr Phe Phe Tyr Gly Gly Ser Arg Gly Lys Arg Asn Asn Phe Lys
1               5                   10                  15

Thr Glu Glu Tyr
            20

<210> 128
<211> 20
<212> PRT
<213> Artificial Sequence

<220>

<221> source
<223> /note="Description of Artificial Sequence: Synthetic
      peptide"

<400> 128
Thr Phe Phe Tyr Gly Gly Ser Arg Gly Lys Arg Asn Asn Phe Lys Thr
1               5                   10                  15

Glu Glu Tyr Cys
            20

<210> 129
<211> 20
<212> PRT
<213> Artificial Sequence

<220>
<221> source
<223> /note="Description of Artificial Sequence: Synthetic
      peptide"

<400> 129
Cys Thr Phe Phe Tyr Gly Gly Ser Arg Gly Arg Arg Asn Asn Phe Arg
1               5                   10                  15

Thr Glu Glu Tyr
            20

<210> 130
<211> 20
<212> PRT
<213> Artificial Sequence

<220>
<221> source
<223> /note="Description of Artificial Sequence: Synthetic
      peptide"

<400> 130
Thr Phe Phe Tyr Gly Gly Ser Arg Gly Arg Arg Asn Asn Phe Arg Thr
1               5                   10                  15

Glu Glu Tyr Cys
            20

<210> 131
<211> 86
<212> PRT
<213> Artificial Sequence

<220>
<221> source
<223> /note="Description of Artificial Sequence: Synthetic
      polypeptide"

<400> 131
Met Glu Pro Val Asp Pro Arg Leu Glu Pro Trp Lys His Pro Gly Ser

1               5                   10                  15

Gln Pro Lys Thr Ala Cys Thr Thr Cys Tyr Cys Lys Lys Cys Cys Phe
            20                  25                  30

His Cys Gln Val Cys Phe Thr Thr Lys Ala Leu Gly Ile Ser Tyr Gly
        35                  40                  45

Arg Lys Lys Arg Arg Gln Arg Arg Arg Pro Pro Gln Gly Ser Gln Thr
    50                  55                  60

His Gln Val Ser Leu Ser Lys Gln Pro Thr Ser Gln Pro Arg Gly Asp
65                  70                  75                  80

Pro Thr Gly Pro Lys Glu
                85

<210> 132
<211> 20
<212> PRT
<213> Artificial Sequence

<220>
<221> source
<223> /note="Description of Artificial Sequence: Synthetic
      peptide"

<400> 132
Phe Thr Thr Lys Ala Leu Gly Ile Ser Tyr Gly Arg Lys Lys Arg Arg
1               5                   10                  15

Gln Arg Arg Arg
            20

<210> 133
<211> 16
<212> PRT
<213> Artificial Sequence

<220>
<221> source
<223> /note="Description of Artificial Sequence: Synthetic
      peptide"

<400> 133
Arg Gln Ile Lys Ile Trp Phe Gln Asn Arg Arg Met Lys Trp Lys Lys
1               5                   10                  15

<210> 134
<211> 18
<212> PRT
<213> Artificial Sequence

<220>

<221> source
<223> /note="Description of Artificial Sequence: Synthetic
      peptide"

<400> 134
Arg Gly Gly Arg Leu Cys Tyr Cys Arg Arg Arg Phe Cys Val Cys Val
1               5                   10                  15

Gly Arg

<210> 135
<211> 19
<212> PRT
<213> Artificial Sequence

<220>
<221> source
<223> /note="Description of Artificial Sequence: Synthetic
      peptide"

<400> 135
Arg Gly Gly Arg Leu Ser Tyr Ser Arg Arg Arg Phe Ser Thr Ser Thr
1               5                   10                  15

Gly Arg Ala

<210> 136
<211> 10
<212> PRT
<213> Artificial Sequence

<220>
<221> source
<223> /note="Description of Artificial Sequence: Synthetic
      peptide"

<400> 136
Arg Arg Leu Ser Tyr Ser Arg Arg Arg Phe
1               5                   10

<210> 137
<211> 17
<212> PRT
<213> Artificial Sequence

<220>
<221> source
<223> /note="Description of Artificial Sequence: Synthetic
      peptide"

<400> 137
Arg Gly Gly Arg Leu Ala Tyr Leu Arg Arg Arg Trp Ala Val Leu Gly
1               5                   10                  15

Arg

<210> 138
<211> 12
<212> PRT
<213> Artificial Sequence

<220>
<221> source
<223> /note="Description of Artificial Sequence: Synthetic
      peptide"

<400> 138
His Leu Asp Cys Met Pro Arg Gly Cys Phe Arg Asn
1               5                   10


<210> 139
<211> 10
<212> PRT
<213> Artificial Sequence

<220>
<221> source
<223> /note="Description of Artificial Sequence: Synthetic
      peptide"

<400> 139
Ala Cys Gln Val Lys Ser Met Pro Arg Cys
1               5                   10


<210> 140
<211> 10
<212> PRT
<213> Artificial Sequence

<220>
<221> source
<223> /note="Description of Artificial Sequence: Synthetic
      peptide"

<400> 140
Ala Cys Thr Thr Pro Met Pro Arg Leu Cys
1               5                   10


<210> 141
<211> 10
<212> PRT
<213> Artificial Sequence

<220>
<221> source
<223> /note="Description of Artificial Sequence: Synthetic
      peptide"

<400> 141
Ala Cys Lys Ala Pro Gln Met Pro Arg Cys
1               5                   10

<210> 142
<211> 10
<212> PRT
<213> Artificial Sequence

<220>
<221> source
<223> /note="Description of Artificial Sequence: Synthetic
      peptide"

<400> 142
Ala Cys Leu Asn Pro Ser Met Pro Arg Cys
1               5                   10


<210> 143
<211> 10
<212> PRT
<213> Artificial Sequence

<220>
<221> source
<223> /note="Description of Artificial Sequence: Synthetic
      peptide"

<400> 143
Ala Cys Leu Val Ser Ser Met Pro Arg Cys
1               5                   10


<210> 144
<211> 10
<212> PRT
<213> Artificial Sequence

<220>
<221> source
<223> /note="Description of Artificial Sequence: Synthetic
      peptide"

<400> 144
Ala Cys Leu Gln Pro Met Pro Arg Leu Cys
1               5                   10


<210> 145
<211> 10
<212> PRT
<213> Artificial Sequence

<220>
<221> source
<223> /note="Description of Artificial Sequence: Synthetic
      peptide"

<400> 145
Ala Cys Pro Val Ser Ser Met Pro Arg Cys
1               5                   10


<210> 146
<211> 10
<212> PRT

<213> Artificial Sequence

<220>
<221> source
<223> /note="Description of Artificial Sequence: Synthetic
      peptide"

<400> 146
Ala Cys Gln Ser Pro Met Pro Arg Leu Cys
1               5                   10


<210> 147
<211> 10
<212> PRT
<213> Artificial Sequence

<220>
<221> source
<223> /note="Description of Artificial Sequence: Synthetic
      peptide"

<400> 147
Ala Cys Leu Thr Pro Met Pro Arg Leu Cys
1               5                   10


<210> 148
<211> 15
<212> PRT
<213> Artificial Sequence

<220>
<221> source
<223> /note="Description of Artificial Sequence: Synthetic
      peptide"

<400> 148
Asp Ser Gly Leu Cys Met Pro Arg Leu Arg Gly Cys Asp Pro Arg
1               5                   10                  15


<210> 149
<211> 10
<212> PRT
<213> Artificial Sequence

<220>
<221> source
<223> /note="Description of Artificial Sequence: Synthetic
      peptide"

<400> 149
Ala Cys Met Pro Arg Leu Arg Gly Cys Ala
1               5                   10


<210> 150
<211> 15
<212> PRT
<213> Artificial Sequence

<220>

<221> source
<223> /note="Description of Artificial Sequence: Synthetic
      peptide"

<400> 150
Ala Ser Gly Leu Cys Met Pro Arg Leu Arg Gly Cys Asp Pro Arg
1               5                   10                  15


<210> 151
<211> 15
<212> PRT
<213> Artificial Sequence

<220>
<221> source
<223> /note="Description of Artificial Sequence: Synthetic
      peptide"

<400> 151
Asp Ala Gly Leu Cys Met Pro Arg Leu Arg Gly Cys Asp Pro Arg
1               5                   10                  15


<210> 152
<211> 15
<212> PRT
<213> Artificial Sequence

<220>
<221> source
<223> /note="Description of Artificial Sequence: Synthetic
      peptide"

<400> 152
Asp Ser Ala Leu Cys Met Pro Arg Leu Arg Gly Cys Asp Pro Arg
1               5                   10                  15


<210> 153
<211> 15
<212> PRT
<213> Artificial Sequence

<220>
<221> source
<223> /note="Description of Artificial Sequence: Synthetic
      peptide"

<400> 153
Asp Ser Gly Ala Cys Met Pro Arg Leu Arg Gly Cys Asp Pro Arg
1               5                   10                  15


<210> 154
<211> 15
<212> PRT
<213> Artificial Sequence

<220>
<221> source
<223> /note="Description of Artificial Sequence: Synthetic
      peptide"

<400> 154
Asp Ser Gly Leu Cys Met Pro Arg Ala Arg Gly Cys Asp Pro Arg
1               5                   10                  15


<210> 155
<211> 15
<212> PRT
<213> Artificial Sequence

<220>
<221> source
<223> /note="Description of Artificial Sequence: Synthetic
      peptide"

<400> 155
Asp Ser Gly Leu Cys Met Pro Arg Leu Ala Gly Cys Asp Pro Arg
1               5                   10                  15


<210> 156
<211> 15
<212> PRT
<213> Artificial Sequence

<220>
<221> source
<223> /note="Description of Artificial Sequence: Synthetic
      peptide"

<400> 156
Asp Ser Gly Leu Cys Met Pro Arg Leu Arg Ala Cys Asp Pro Arg
1               5                   10                  15


<210> 157
<211> 15
<212> PRT
<213> Artificial Sequence

<220>
<221> source
<223> /note="Description of Artificial Sequence: Synthetic
      peptide"

<400> 157
Asp Ser Gly Leu Cys Met Pro Arg Leu Arg Gly Cys Ala Pro Arg
1               5                   10                  15


<210> 158
<211> 15
<212> PRT
<213> Artificial Sequence

<220>
<221> source
<223> /note="Description of Artificial Sequence: Synthetic
      peptide"

<400> 158
Asp Ser Gly Leu Cys Met Pro Arg Leu Arg Gly Cys Asp Ala Arg

1                 5                   10                  15


<210> 159
<211> 15
<212> PRT
<213> Artificial Sequence

<220>
<221> source
<223> /note="Description of Artificial Sequence: Synthetic
      peptide"

<400> 159
Asp Ser Gly Leu Cys Met Pro Arg Leu Arg Gly Cys Asp Pro Ala
1               5                   10                  15


<210> 160
<211> 14
<212> PRT
<213> Artificial Sequence

<220>
<221> source
<223> /note="Description of Artificial Sequence: Synthetic
      peptide"

<400> 160
Ser Gly Leu Cys Met Pro Arg Leu Arg Gly Cys Asp Pro Arg
1               5                   10


<210> 161
<211> 13
<212> PRT
<213> Artificial Sequence

<220>
<221> source
<223> /note="Description of Artificial Sequence: Synthetic
      peptide"

<400> 161
Gly Leu Cys Met Pro Arg Leu Arg Gly Cys Asp Pro Arg
1               5                   10


<210> 162
<211> 8
<212> PRT
<213> Artificial Sequence

<220>
<221> source
<223> /note="Description of Artificial Sequence: Synthetic
      peptide"

<400> 162
Cys Met Pro Arg Leu Arg Gly Cys
1               5

<210> 163
<211> 8
<212> PRT
<213> Artificial Sequence

<220>
<221> source
<223> /note="Description of Artificial Sequence: Synthetic
      peptide"

<400> 163
Cys Met Pro Arg Ala Arg Gly Cys
1               5


<210> 164
<211> 8
<212> PRT
<213> Artificial Sequence

<220>
<221> source
<223> /note="Description of Artificial Sequence: Synthetic
      peptide"

<400> 164
Cys Met Pro Arg Leu Ala Gly Cys
1               5


<210> 165
<211> 8
<212> PRT
<213> Artificial Sequence

<220>
<221> source
<223> /note="Description of Artificial Sequence: Synthetic
      peptide"

<400> 165
Cys Met Pro Arg Leu Arg Ala Cys
1               5


<210> 166
<211> 8
<212> PRT
<213> Artificial Sequence

<220>
<221> source
<223> /note="Description of Artificial Sequence: Synthetic
      peptide"

<400> 166
Cys Met Pro Arg Leu Lys Gly Cys
1               5


<210> 167
<211> 8
<212> PRT

<213> Artificial Sequence

<220>
<221> source
<223> /note="Description of Artificial Sequence: Synthetic
      peptide"


<220>
<221> MOD_RES
<222> (1)..(1)
<223> D-Cys

<400> 167
Xaa Met Pro Arg Leu Arg Gly Cys
1               5


<210> 168
<211> 8
<212> PRT
<213> Artificial Sequence

<220>
<221> source
<223> /note="Description of Artificial Sequence: Synthetic
      peptide"


<220>
<221> MOD_RES
<222> (5)..(5)
<223> D-Leu

<400> 168
Cys Met Pro Arg Xaa Arg Gly Cys
1               5


<210> 169
<211> 8
<212> PRT
<213> Artificial Sequence

<220>
<221> source
<223> /note="Description of Artificial Sequence: Synthetic
      peptide"


<220>
<221> MOD_RES
<222> (6)..(6)
<223> D-Arg

<400> 169
Cys Met Pro Arg Leu Xaa Gly Cys
1               5


<210> 170
<211> 8
<212> PRT

<213> Artificial Sequence

<220>
<221> source
<223> /note="Description of Artificial Sequence: Synthetic
      peptide"


<220>
<221> MOD_RES
<222> (8)..(8)
<223> D-Cys

<400> 170
Cys Met Pro Arg Leu Arg Gly Xaa
1               5


<210> 171
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (8)..(8)
<223> D-Cys

<400> 171
Xaa Met Pro Arg Leu Arg Gly Xaa
1               5


<210> 172
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (7)..(7)

<223> Sar

<400> 172
Xaa Met Pro Arg Leu Arg Xaa Cys
1               5


<210> 173
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<400> 173
Xaa Met Pro Arg Lys Arg Gly Cys
1               5


<210> 174
<211> 7
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<400> 174
Xaa Met Pro Arg Leu Arg Cys
1               5


<210> 175
<211> 7
<212> PRT
<213> Artificial Sequence

<220>
<221> source
<223> /note="Description of Artificial Sequence: Synthetic
      peptide"


<220>
<221> MOD_RES
<222> (1)..(1)

<223> D-Cys

<400> 175
Xaa Met Pro Arg Leu Cys Arg
1               5


<210> 176
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<400> 176
Xaa Met Pro Arg Cys Leu Arg Gly
1               5


<210> 177
<211> 6
<212> PRT
<213> Artificial Sequence

<220>
<221> source
<223> /note="Description of Artificial Sequence: Synthetic
      peptide"

<400> 177
Cys Met Pro Arg Gly Cys
1               5


<210> 178
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> Pen

<400> 178
Xaa Met Pro Arg Leu Arg Gly Cys
1               5

<210> 179
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Pen

<400> 179
Xaa Met Pro Arg Leu Arg Gly Cys
1               5


<210> 180
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (8)..(8)
<223> Pen

<400> 180
Xaa Met Pro Arg Leu Arg Gly Xaa
1               5


<210> 181
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> Pen

<220>
<221> MOD_RES
<222> (8)..(8)
<223> Pen

<400> 181
Xaa Met Pro Arg Leu Arg Gly Xaa
1               5


<210> 182
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Pen

<220>
<221> MOD_RES
<222> (8)..(8)
<223> Pen

<400> 182
Xaa Met Pro Arg Leu Arg Gly Xaa
1               5


<210> 183
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Pen

<220>
<221> MOD_RES
<222> (8)..(8)
<223> D-Pen

<400> 183
Xaa Met Pro Arg Leu Arg Gly Xaa
1               5

<210> 184
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> Mpa

<400> 184
Xaa Met Pro Arg Leu Arg Gly Cys
1               5


<210> 185
<211> 15
<212> PRT
<213> Artificial Sequence

<220>
<221> source
<223> /note="Description of Artificial Sequence: Synthetic
      peptide"

<400> 185
Met Thr Val Met Pro Thr Gly Leu Trp Asn Pro Leu Ile Pro Ser
1               5                   10                  15


<210> 186
<211> 15
<212> PRT
<213> Artificial Sequence

<220>
<221> source
<223> /note="Description of Artificial Sequence: Synthetic
      peptide"

<400> 186
Ser Ala Ser Trp Phe Ala Val Pro Ile Pro Pro Leu Arg Leu Glu
1               5                   10                  15


<210> 187
<211> 15
<212> PRT
<213> Artificial Sequence

<220>
<221> source
<223> /note="Description of Artificial Sequence: Synthetic
      peptide"

<400> 187
Met Thr Pro Met Ser Thr Pro Arg Met Leu Pro Val Tyr Val Ala

1               5                   10                  15


<210> 188
<211> 15
<212> PRT
<213> Artificial Sequence

<220>
<221> source
<223> /note="Description of Artificial Sequence: Synthetic
      peptide"

<400> 188
Met Thr Ala Thr His Leu Ser Thr Leu Phe Gln Pro Leu Thr Tyr
1               5                   10                  15


<210> 189
<211> 15
<212> PRT
<213> Artificial Sequence

<220>
<221> source
<223> /note="Description of Artificial Sequence: Synthetic
      peptide"

<400> 189
Met Ser Pro Ile Pro Pro Ala Ala Ser Thr Trp Ala Asn Thr Leu
1               5                   10                  15


<210> 190
<211> 15
<212> PRT
<213> Artificial Sequence

<220>
<221> source
<223> /note="Description of Artificial Sequence: Synthetic
      peptide"

<400> 190
Met Thr Ala Asn Pro Leu Gln Asn Ala Pro Gly Pro Leu Ser Leu
1               5                   10                  15


<210> 191
<211> 15
<212> PRT
<213> Artificial Sequence

<220>
<221> source
<223> /note="Description of Artificial Sequence: Synthetic
      peptide"

<400> 191
Met Gln Thr Ala Pro Pro Pro Pro Leu Thr Arg Val Gln Trp Ser
1               5                   10                  15

<210> 192
<211> 15
<212> PRT
<213> Artificial Sequence

<220>
<221> source
<223> /note="Description of Artificial Sequence: Synthetic
      peptide"

<400> 192
Gly Thr Pro Arg Met His Ile Pro Leu Asn Val Asp His Leu Pro
1               5                   10                  15


<210> 193
<211> 15
<212> PRT
<213> Artificial Sequence

<220>
<221> source
<223> /note="Description of Artificial Sequence: Synthetic
      peptide"

<400> 193
Leu Thr Leu Pro Pro Ile Ser Gly Leu Ser Ser Tyr Pro Leu Pro
1               5                   10                  15


<210> 194
<211> 15
<212> PRT
<213> Artificial Sequence

<220>
<221> source
<223> /note="Description of Artificial Sequence: Synthetic
      peptide"

<400> 194
Thr Pro Ser Ala His Ala Met Ala Leu Gln Ser Leu Ser Val Gly
1               5                   10                  15


<210> 195
<211> 15
<212> PRT
<213> Artificial Sequence

<220>
<221> source
<223> /note="Description of Artificial Sequence: Synthetic
      peptide"

<400> 195
Leu Thr Leu Pro Pro Ile Ser Gly Leu Ser Ser Tyr Pro Leu Pro
1               5                   10                  15


<210> 196
<211> 15
<212> PRT

<213> Artificial Sequence

<220>
<221> source
<223> /note="Description of Artificial Sequence: Synthetic
      peptide"

<400> 196
Met Gly Thr Leu Asn Ala Pro Thr Ala Tyr Pro Gln Asp Ser Leu
1               5                   10                  15


<210> 197
<211> 15
<212> PRT
<213> Artificial Sequence

<220>
<221> source
<223> /note="Description of Artificial Sequence: Synthetic
      peptide"

<400> 197
Leu Thr Asn Pro Pro Ala Tyr Leu Pro Gln Asn Thr Asp Pro His
1               5                   10                  15


<210> 198
<211> 15
<212> PRT
<213> Artificial Sequence

<220>
<221> source
<223> /note="Description of Artificial Sequence: Synthetic
      peptide"

<400> 198
Met Gly Leu Pro Leu Pro Tyr Ile Gln Thr Ile Leu His Thr Pro
1               5                   10                  15


<210> 199
<211> 15
<212> PRT
<213> Artificial Sequence

<220>
<221> source
<223> /note="Description of Artificial Sequence: Synthetic
      peptide"

<400> 199
Ser Ala Ala Leu Ile Ala Met Ser Ser Phe Lys Ser Ile Thr Ala
1               5                   10                  15


<210> 200
<211> 15
<212> PRT
<213> Artificial Sequence

<220>

<221> source
<223> /note="Description of Artificial Sequence: Synthetic
      peptide"

<400> 200
Ser Gly Phe Ala Phe Ala Arg Ser Val Pro Thr Glu Ser Arg Arg
1               5                   10                  15


<210> 201
<211> 14
<212> PRT
<213> Artificial Sequence

<220>
<221> source
<223> /note="Description of Artificial Sequence: Synthetic
      peptide"

<400> 201
Met Thr Ser Pro Tyr Met Ser Leu Pro Pro Ser Thr Asp Asp
1               5                   10


<210> 202
<211> 15
<212> PRT
<213> Artificial Sequence

<220>
<221> source
<223> /note="Description of Artificial Sequence: Synthetic
      peptide"

<400> 202
Leu Thr Asn Pro Pro Ala Tyr Leu Pro Gln Asn Thr Asp Pro His
1               5                   10                  15


<210> 203
<211> 8
<212> PRT
<213> Artificial Sequence

<220>
<221> source
<223> /note="Description of Artificial Sequence: Synthetic
      peptide"


<220>
<221> VARIANT
<222> (1)..(1)
<223> /replace="an analogue or isostere of Cys"

<220>
<221> VARIANT
<222> (3)..(3)
<223> /replace="an analogue or isostere of Pro"

<220>
<221> VARIANT
<222> (6)..(6)

<223> /replace="Arg"

<220>
<221> VARIANT
<222> (7)..(7)
<223> /replace="an analogue or isostere of Gly"

<220>
<221> VARIANT
<222> (8)..(8)
<223> /replace="an analogue or isostere of Cys"

<220>
<221> SITE
<222> (1)..(8)
<223> /note="Variant residues given in the sequence have no
      preference with respect to those in the annotations
      for variant positions"

<400> 203
Cys Met Pro Arg Leu Ala Gly Cys
1               5


<210> 204
<211> 8
<212> PRT
<213> Artificial Sequence

<220>
<221> source
<223> /note="Description of Artificial Sequence: Synthetic
      peptide"


<220>
<221> VARIANT
<222> (1)..(1)
<223> /replace="D-Cys" or "Pen" or "D-Pen"

<220>
<221> VARIANT
<222> (3)..(3)
<223> /replace="Pip" or "Thz"

<220>
<221> SITE
<222> (1)..(8)
<223> /note="Variant residues given in the sequence have no
      preference with respect to those in the annotations
      for variant positions"

<400> 204
Cys Met Pro Arg Leu Arg Gly Cys
1               5


<210> 205
<211> 8
<212> PRT
<213> Artificial Sequence

<220>

<221> source
<223> /note="Description of Artificial Sequence: Synthetic
      peptide"

<220>
<221> MOD_RES
<222> (1)..(1)
<223> D-Cys

<220>
<221> MOD_RES
<222> (3)..(3)
<223> Pip

<400> 205
Xaa Met Xaa Arg Leu Arg Gly Cys
1               5

<210> 206
<211> 8
<212> PRT
<213> Artificial Sequence

<220>
<221> source
<223> /note="Description of Artificial Sequence: Synthetic
      peptide"

<220>
<221> MOD_RES
<222> (1)..(1)
<223> D-pen

<220>
<221> MOD_RES
<222> (3)..(3)
<223> Pip

<400> 206
Xaa Met Xaa Arg Leu Arg Gly Cys
1               5

<210> 207
<211> 8
<212> PRT
<213> Artificial Sequence

<220>
<221> source
<223> /note="Description of Artificial Sequence: Synthetic
      peptide"

<220>
<221> MOD_RES
<222> (1)..(1)
<223> Pen

<220>

<221> MOD_RES
<222> (3)..(3)
<223> Pip

<400> 207
Xaa Met Xaa Arg Leu Arg Gly Cys
1               5


<210> 208
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (3)..(3)
<223> Thz

<400> 208
Xaa Met Xaa Arg Leu Arg Gly Cys
1               5


<210> 209
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-pen

<220>
<221> MOD_RES
<222> (3)..(3)
<223> Thz

<400> 209
Xaa Met Xaa Arg Leu Arg Gly Cys
1               5


<210> 210
<211> 8

<212> PRT
<213> Artificial Sequence

<220>
<221> source
<223> /note="Description of Artificial Sequence: Synthetic
      peptide"


<220>
<221> MOD_RES
<222> (1)..(1)
<223> Pen

<220>
<221> MOD_RES
<222> (3)..(3)
<223> Thz

<400> 210
Xaa Met Xaa Arg Leu Arg Gly Cys
1               5


<210> 211
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (3)..(3)
<223> Thz

<220>
<221> MOD_RES
<222> (7)..(7)
<223> Sar

<400> 211
Xaa Met Xaa Arg Leu Arg Xaa Cys
1               5


<210> 212
<211> 8
<212> PRT
<213> Artificial Sequence

<220>
<221> source
<223> /note="Description of Artificial Sequence: Synthetic

peptide"

<220>
<221> MOD_RES
<222> (1)..(1)
<223> D-Cys

<220>
<221> MOD_RES
<222> (3)..(3)
<223> Thz

<220>
<221> MOD_RES
<222> (8)..(8)
<223> Pen

<400> 212
Xaa Met Xaa Arg Leu Arg Gly Xaa
1               5

<210> 213
<211> 8
<212> PRT
<213> Artificial Sequence

<220>
<221> source
<223> /note="Description of Artificial Sequence: Synthetic
      peptide"

<220>
<221> MOD_RES
<222> (1)..(1)
<223> D-Cys

<220>
<221> MOD_RES
<222> (3)..(3)
<223> Thz

<220>
<221> MOD_RES
<222> (7)..(7)
<223> Sar

<220>
<221> MOD_RES
<222> (8)..(8)
<223> Pen

<400> 213
Xaa Met Xaa Arg Leu Arg Xaa Xaa
1               5

<210> 214
<211> 8
<212> PRT
<213> Artificial Sequence

<220>
<221> source
<223> /note="Description of Artificial Sequence: Synthetic
      peptide"

<220>
<221> MOD_RES
<222> (1)..(1)
<223> D-Cys

<220>
<221> MOD_RES
<222> (3)..(3)
<223> Pip

<220>
<221> MOD_RES
<222> (7)..(7)
<223> Sar

<400> 214
Xaa Met Xaa Arg Leu Arg Xaa Cys
1               5

<210> 215
<211> 8
<212> PRT
<213> Artificial Sequence

<220>
<221> source
<223> /note="Description of Artificial Sequence: Synthetic
      peptide"

<220>
<221> MOD_RES
<222> (1)..(1)
<223> D-Cys

<220>
<221> MOD_RES
<222> (3)..(3)
<223> Pip

<220>
<221> MOD_RES
<222> (8)..(8)
<223> Pen

<400> 215
Xaa Met Xaa Arg Leu Arg Gly Xaa
1               5

<210> 216
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (3)..(3)
<223> Pip

<220>
<221> MOD_RES
<222> (7)..(7)
<223> Sar

<220>
<221> MOD_RES
<222> (8)..(8)
<223> Pen

<400> 216
Xaa Met Xaa Arg Leu Arg Xaa Xaa
1               5


<210> 217
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (4)..(4)
<223> hArg

<400> 217
Xaa Met Pro Xaa Leu Arg Gly Cys
1               5


<210> 218
<211> 8
<212> PRT
<213> Artificial Sequence

<220>

<221> source
<223> /note="Description of Artificial Sequence: Synthetic
      peptide"


<220>
<221> MOD_RES
<222> (1)..(1)
<223> D-Cys

<220>
<221> MOD_RES
<222> (4)..(4)
<223> Agb

<400> 218
Xaa Met Pro Xaa Leu Arg Gly Cys
1               5


<210> 219
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (4)..(4)
<223> Agp

<400> 219
Xaa Met Pro Xaa Leu Arg Gly Cys
1               5


<210> 220
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>

<221> MOD_RES
<222> (4)..(4)
<223> Cit

<400> 220
Xaa Met Pro Xaa Leu Arg Gly Cys
1               5


<210> 221
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (6)..(6)
<223> Cit

<400> 221
Xaa Met Pro Arg Leu Xaa Gly Cys
1               5


<210> 222
<211> 8
<212> PRT
<213> Artificial Sequence

<220>
<221> source
<223> /note="Description of Artificial Sequence: Synthetic
      peptide"


<220>
<221> MOD_RES
<222> (1)..(1)
<223> D-Cys

<220>
<221> MOD_RES
<222> (6)..(6)
<223> Arg(NO2)

<400> 222
Xaa Met Pro Arg Leu Xaa Gly Cys
1               5


<210> 223
<211> 8

<212> PRT
<213> Artificial Sequence

<220>
<221> source
<223> /note="Description of Artificial Sequence: Synthetic
      peptide"

<220>
<221> MOD_RES
<222> (1)..(1)
<223> D-Cys

<400> 223
Xaa Met Pro Arg Leu Arg Gly Cys
1               5

<210> 224
<211> 8
<212> PRT
<213> Artificial Sequence

<220>
<221> source
<223> /note="Description of Artificial Sequence: Synthetic
      peptide"

<400> 224
Cys Met Pro Arg Leu Arg Gly Cys
1               5

<210> 225
<211> 19
<212> PRT
<213> Homo sapiens

<400> 225
Val Pro Ser Thr Pro Pro Thr Pro Ser Pro Ser Thr Pro Pro Thr Pro
1               5                   10                  15

Ser Pro Ser

<210> 226
<211> 101
<212> PRT
<213> Homo sapiens

<400> 226
Cys Cys His Pro Arg Leu Ser Leu His Arg Pro Ala Leu Glu Asp Leu
1               5                   10                  15

Leu Leu Gly Ser Glu Ala Asn Leu Thr Cys Thr Leu Thr Gly Leu Arg
            20                  25                  30

Asp Ala Ser Gly Val Thr Phe Thr Trp Thr Pro Ser Ser Gly Lys Ser

35                  40                  45

Ala Val Gln Gly Pro Pro Glu Arg Asp Leu Cys Gly Cys Tyr Ser Val
    50                  55                  60

Ser Ser Val Leu Pro Gly Cys Ala Glu Pro Trp Asn His Gly Lys Thr
65                  70                  75                  80

Phe Thr Cys Thr Ala Ala Tyr Pro Glu Ser Lys Thr Pro Leu Thr Ala
                85                  90                  95

Thr Leu Ser Lys Ser
            100

<210> 227
<211> 131
<212> PRT
<213> Homo sapiens

<400> 227
Gly Asn Thr Phe Arg Pro Glu Val His Leu Leu Pro Pro Pro Ser Glu
1               5                   10                  15

Glu Leu Ala Leu Asn Glu Leu Val Thr Leu Thr Cys Leu Ala Arg Gly
            20                  25                  30

Phe Ser Pro Lys Asp Val Leu Val Arg Trp Leu Gln Gly Ser Gln Glu
        35                  40                  45

Leu Pro Arg Glu Lys Tyr Leu Thr Trp Ala Ser Arg Gln Glu Pro Ser
    50                  55                  60

Gln Gly Thr Thr Thr Phe Ala Val Thr Ser Ile Leu Arg Val Ala Ala
65                  70                  75                  80

Glu Asp Trp Lys Lys Gly Asp Thr Phe Ser Cys Met Val Gly His Glu
                85                  90                  95

Ala Leu Pro Leu Ala Phe Thr Gln Lys Thr Ile Asp Arg Leu Ala Gly
            100                 105                 110

Lys Pro Thr His Val Asn Val Ser Val Val Met Ala Glu Val Asp Gly
        115                 120                 125

Thr Cys Tyr
    130

<210> 228
<211> 6

<212> PRT
<213> Homo sapiens

<400> 228
Val Pro Pro Pro Pro Pro
1               5


<210> 229
<211> 101
<212> PRT
<213> Homo sapiens

<400> 229
Cys Cys His Pro Arg Leu Ser Leu His Arg Pro Ala Leu Glu Asp Leu
1               5                   10                  15

Leu Leu Gly Ser Glu Ala Asn Leu Thr Cys Thr Leu Thr Gly Leu Arg
            20                  25                  30

Asp Ala Ser Gly Ala Thr Phe Thr Trp Thr Pro Ser Ser Gly Lys Ser
        35                  40                  45

Ala Val Gln Gly Pro Pro Glu Arg Asp Leu Cys Gly Cys Tyr Ser Val
    50                  55                  60

Ser Ser Val Leu Pro Gly Cys Ala Gln Pro Trp Asn His Gly Glu Thr
65                  70                  75                  80

Phe Thr Cys Thr Ala Ala His Pro Glu Leu Lys Thr Pro Leu Thr Ala
                85                  90                  95

Asn Ile Thr Lys Ser
            100


<210> 230
<211> 131
<212> PRT
<213> Homo sapiens

<400> 230
Gly Asn Thr Phe Arg Pro Glu Val His Leu Leu Pro Pro Pro Ser Glu
1               5                   10                  15

Glu Leu Ala Leu Asn Glu Leu Val Thr Leu Thr Cys Leu Ala Arg Gly
            20                  25                  30

Phe Ser Pro Lys Asp Val Leu Val Arg Trp Leu Gln Gly Ser Gln Glu
        35                  40                  45

Leu Pro Arg Glu Lys Tyr Leu Thr Trp Ala Ser Arg Gln Glu Pro Ser
    50                  55                  60

Gln Gly Thr Thr Thr Phe Ala Val Thr Ser Ile Leu Arg Val Ala Ala
65                  70                  75                  80

Glu Asp Trp Lys Lys Gly Asp Thr Phe Ser Cys Met Val Gly His Glu
                85                  90                  95

Ala Leu Pro Leu Ala Phe Thr Gln Lys Thr Ile Asp Arg Leu Ala Gly
            100                 105                 110

Lys Pro Thr His Val Asn Val Ser Val Val Met Ala Glu Val Asp Gly
        115                 120                 125

Thr Cys Tyr
    130

<210> 231
<211> 58
<212> PRT
<213> Homo sapiens

<400> 231
Glu Ser Pro Lys Ala Gln Ala Ser Ser Val Pro Thr Ala Gln Pro Gln
1               5                   10                  15

Ala Glu Gly Ser Leu Ala Lys Ala Thr Thr Ala Pro Ala Thr Thr Arg
            20                  25                  30

Asn Thr Gly Arg Gly Gly Glu Glu Lys Lys Lys Glu Lys Glu Lys Glu
        35                  40                  45

Glu Gln Glu Glu Arg Glu Thr Lys Thr Pro
    50                  55

<210> 232
<211> 108
<212> PRT
<213> Homo sapiens

<400> 232
Glu Cys Pro Ser His Thr Gln Pro Leu Gly Val Tyr Leu Leu Thr Pro
1               5                   10                  15

Ala Val Gln Asp Leu Trp Leu Arg Asp Lys Ala Thr Phe Thr Cys Phe
            20                  25                  30

Val Val Gly Ser Asp Leu Lys Asp Ala His Leu Thr Trp Glu Val Ala
        35                  40                  45

Gly Lys Val Pro Thr Gly Gly Val Glu Glu Gly Leu Leu Glu Arg His
    50                  55                  60

Ser Asn Gly Ser Gln Ser Gln His Ser Arg Leu Thr Leu Pro Arg Ser
65                  70                  75                  80

Leu Trp Asn Ala Gly Thr Ser Val Thr Cys Thr Leu Asn His Pro Ser
                85                  90                  95

Leu Pro Pro Gln Arg Leu Met Ala Leu Arg Glu Pro
            100                 105

<210> 233
<211> 117
<212> PRT
<213> Homo sapiens

<400> 233
Ala Ala Gln Ala Pro Val Lys Leu Ser Leu Asn Leu Leu Ala Ser Ser
1               5                   10                  15

Asp Pro Pro Glu Ala Ala Ser Trp Leu Leu Cys Glu Val Ser Gly Phe
            20                  25                  30

Ser Pro Pro Asn Ile Leu Leu Met Trp Leu Glu Asp Gln Arg Glu Val
        35                  40                  45

Asn Thr Ser Gly Phe Ala Pro Ala Arg Pro Pro Pro Gln Pro Arg Ser
    50                  55                  60

Thr Thr Phe Trp Ala Trp Ser Val Leu Arg Val Pro Ala Pro Pro Ser
65                  70                  75                  80

Pro Gln Pro Ala Thr Tyr Thr Cys Val Val Ser His Glu Asp Ser Arg
                85                  90                  95

Thr Leu Leu Asn Ala Ser Arg Ser Leu Glu Val Ser Tyr Val Thr Asp
            100                 105                 110

His Gly Pro Met Lys
        115

<210> 234
<211> 107
<212> PRT
<213> Homo sapiens

<400> 234
Val Cys Ser Arg Asp Phe Thr Pro Pro Thr Val Lys Ile Leu Gln Ser
1               5                   10                  15

Ser Cys Asp Gly Gly Gly His Phe Pro Pro Thr Ile Gln Leu Leu Cys

            20                  25                  30

Leu Val Ser Gly Tyr Thr Pro Gly Thr Ile Asn Ile Thr Trp Leu Glu
        35                  40                  45

Asp Gly Gln Val Met Asp Val Asp Leu Ser Thr Ala Ser Thr Thr Gln
    50                  55                  60

Glu Gly Glu Leu Ala Ser Thr Gln Ser Glu Leu Thr Leu Ser Gln Lys
65                  70                  75                  80

His Trp Leu Ser Asp Arg Thr Tyr Thr Cys Gln Val Thr Tyr Gln Gly
                85                  90                  95

His Thr Phe Glu Asp Ser Thr Lys Lys Cys Ala
            100                 105

<210> 235
<211> 108
<212> PRT
<213> Homo sapiens

<400> 235
Asp Ser Asn Pro Arg Gly Val Ser Ala Tyr Leu Ser Arg Pro Ser Pro
1               5                   10                  15

Phe Asp Leu Phe Ile Arg Lys Ser Pro Thr Ile Thr Cys Leu Val Val
            20                  25                  30

Asp Leu Ala Pro Ser Lys Gly Thr Val Asn Leu Thr Trp Ser Arg Ala
        35                  40                  45

Ser Gly Lys Pro Val Asn His Ser Thr Arg Lys Glu Glu Lys Gln Arg
    50                  55                  60

Asn Gly Thr Leu Thr Val Thr Ser Thr Leu Pro Val Gly Thr Arg Asp
65                  70                  75                  80

Trp Ile Glu Gly Glu Thr Tyr Gln Cys Arg Val Thr His Pro His Leu
                85                  90                  95

Pro Arg Ala Leu Met Arg Ser Thr Thr Lys Thr Ser
            100                 105

<210> 236
<211> 110
<212> PRT
<213> Homo sapiens

<400> 236

Gly Pro Arg Ala Ala Pro Glu Val Tyr Ala Phe Ala Thr Pro Glu Trp
1               5                   10                  15

Pro Gly Ser Arg Asp Lys Arg Thr Leu Ala Cys Leu Ile Gln Asn Phe
            20                  25                  30

Met Pro Glu Asp Ile Ser Val Gln Trp Leu His Asn Glu Val Gln Leu
        35                  40                  45

Pro Asp Ala Arg His Ser Thr Thr Gln Pro Arg Lys Thr Lys Gly Ser
    50                  55                  60

Gly Phe Phe Val Phe Ser Arg Leu Glu Val Thr Arg Ala Glu Trp Glu
65                  70                  75                  80

Gln Lys Asp Glu Phe Ile Cys Arg Ala Val His Glu Ala Ala Ser Pro
                85                  90                  95

Ser Gln Thr Val Gln Arg Ala Val Ser Val Asn Pro Gly Lys
            100                 105                 110

<210> 237
<211> 15
<212> PRT
<213> Homo sapiens

<400> 237
Glu Pro Lys Ser Cys Asp Lys Thr His Thr Cys Pro Pro Cys Pro
1               5                   10                  15

<210> 238
<211> 11
<212> PRT
<213> Artificial Sequence

<220>
<221> source
<223> /note="Description of Artificial Sequence: Synthetic
      peptide"

<400> 238
Ser Asp Lys Thr His Thr Cys Pro Pro Cys Pro
1               5                   10

<210> 239
<211> 110
<212> PRT
<213> Homo sapiens

<400> 239
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110

<210> 240
<211> 107
<212> PRT
<213> Homo sapiens

<400> 240
Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210> 241
<211> 229
<212> PRT
<213> Homo sapiens

<400> 241
Met Ser Asp Lys Thr His Thr Cys Pro Pro Cys Pro Ala Pro Glu Leu
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser His Glu Asp Pro Glu Val Lys Phe Asn Trp Tyr Val Asp Gly Val
    50                  55                  60

Glu Val His Asn Ala Lys Thr Lys Pro Arg Glu Glu Gln Tyr Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Ala Leu Pro Ala
            100                 105                 110

Pro Ile Glu Lys Thr Ile Ser Lys Ala Lys Gly Gln Pro Arg Glu Pro
        115                 120                 125

Gln Val Tyr Thr Leu Pro Pro Ser Arg Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Lys Leu
            180                 185                 190

Thr Val Asp Lys Ser Arg Trp Gln Gln Gly Asn Val Phe Ser Cys Ser
        195                 200                 205

Val Met His Glu Ala Leu His Asn His Tyr Thr Gln Lys Ser Leu Ser
    210                 215                 220

Leu Ser Pro Gly Lys
225

<210> 242
<211> 12

<212> PRT
<213> Homo sapiens

<400> 242
Glu Arg Lys Cys Cys Val Glu Cys Pro Pro Cys Pro
1               5                   10

<210> 243
<211> 109
<212> PRT
<213> Homo sapiens

<400> 243
Ala Pro Pro Val Ala Gly Pro Ser Val Phe Leu Phe Pro Pro Lys Pro
1               5                   10                  15

Lys Asp Thr Leu Met Ile Ser Arg Thr Pro Glu Val Thr Cys Val Val
            20                  25                  30

Val Asp Val Ser His Glu Asp Pro Glu Val Gln Phe Asn Trp Tyr Val
        35                  40                  45

Asp Gly Val Glu Val His Asn Ala Lys Thr Lys Pro Arg Glu Glu Gln
    50                  55                  60

Phe Asn Ser Thr Phe Arg Val Val Ser Val Leu Thr Val Val His Gln
65                  70                  75                  80

Asp Trp Leu Asn Gly Lys Glu Tyr Lys Cys Lys Val Ser Asn Lys Gly
                85                  90                  95

Leu Pro Ala Pro Ile Glu Lys Thr Ile Ser Lys Thr Lys
            100                 105

<210> 244
<211> 107
<212> PRT
<213> Homo sapiens

<400> 244
Gly Gln Pro Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Met Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210> 245
<211> 62
<212> PRT
<213> Homo sapiens

<400> 245
Glu Leu Lys Thr Pro Leu Gly Asp Thr Thr His Thr Cys Pro Arg Cys
1               5                   10                  15

Pro Glu Pro Lys Ser Cys Asp Thr Pro Pro Pro Cys Pro Arg Cys Pro
            20                  25                  30

Glu Pro Lys Ser Cys Asp Thr Pro Pro Pro Cys Pro Arg Cys Pro Glu
        35                  40                  45

Pro Lys Ser Cys Asp Thr Pro Pro Pro Cys Pro Arg Cys Pro
    50                  55                  60

<210> 246
<211> 110
<212> PRT
<213> Homo sapiens

<400> 246
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Gln Phe Lys Trp Tyr
        35                  40                  45

Val Asp Gly Val Glu Val His Asn Ala Lys Thr Lys Pro Arg Glu Glu
    50                  55                  60

Gln Tyr Asn Ser Thr Phe Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Thr Lys
            100                 105                 110

<210> 247
<211> 107
<212> PRT
<213> Homo sapiens

<400> 247
Gly Gln Pro Arg Glu Pro Gln Val Tyr Thr Leu Pro Pro Ser Arg Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Ser Gly Gln Pro Glu
        35                  40                  45

Asn Asn Tyr Asn Thr Thr Pro Pro Met Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Ile Phe Ser Cys Ser Val Met His Glu Ala Leu His Asn Arg Phe
                85                  90                  95

Thr Gln Lys Ser Leu Ser Leu Ser Pro Gly Lys
            100                 105

<210> 248
<211> 12
<212> PRT
<213> Homo sapiens

<400> 248
Glu Ser Lys Tyr Gly Pro Pro Cys Pro Ser Cys Pro
1               5                   10

<210> 249
<211> 110
<212> PRT
<213> Homo sapiens

<400> 249
Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys
            100                 105                 110

<210> 250
<211> 107
<212> PRT
<213> Homo sapiens

<400> 250
Gly Gln Pro Arg Glu Pro Gln Val Tyr Thr Leu Pro Pro Ser Gln Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Arg Leu Thr Val Asp Lys Ser Arg Trp Gln Glu Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Leu Gly Lys
            100                 105

<210> 251
<211> 112
<212> PRT
<213> Homo sapiens

<400> 251
Val Ile Ala Glu Leu Pro Pro Lys Val Ser Val Phe Val Pro Pro Arg
1               5                   10                  15

Asp Gly Phe Phe Gly Asn Pro Arg Lys Ser Lys Leu Ile Cys Gln Ala
            20                  25                  30

Thr Gly Phe Ser Pro Arg Gln Ile Gln Val Ser Trp Leu Arg Glu Gly
        35                  40                  45

Lys Gln Val Gly Ser Gly Val Thr Thr Asp Gln Val Gln Ala Glu Ala
    50                  55                  60

Lys Glu Ser Gly Pro Thr Thr Tyr Lys Val Thr Ser Thr Leu Thr Ile
65                  70                  75                  80

Lys Glu Ser Asp Trp Leu Gly Gln Ser Met Phe Thr Cys Arg Val Asp
                85                  90                  95

His Arg Gly Leu Thr Phe Gln Gln Asn Ala Ser Ser Met Cys Val Pro
            100                 105                 110

<210> 252
<211> 106
<212> PRT
<213> Homo sapiens

<400> 252
Asp Gln Asp Thr Ala Ile Arg Val Phe Ala Ile Pro Pro Ser Phe Ala
1               5                   10                  15

Ser Ile Phe Leu Thr Lys Ser Thr Lys Leu Thr Cys Leu Val Thr Asp
            20                  25                  30

Leu Thr Thr Tyr Asp Ser Val Thr Ile Ser Trp Thr Arg Gln Asn Gly
        35                  40                  45

Glu Ala Val Lys Thr His Thr Asn Ile Ser Glu Ser His Pro Asn Ala
    50                  55                  60

Thr Phe Ser Ala Val Gly Glu Ala Ser Ile Cys Glu Asp Asp Trp Asn
65                  70                  75                  80

Ser Gly Glu Arg Phe Thr Cys Thr Val Thr His Thr Asp Leu Pro Ser
                85                  90                  95

Pro Leu Lys Gln Thr Ile Ser Arg Pro Lys
            100                 105

<210> 253
<211> 131
<212> PRT

<213> Homo sapiens

<400> 253
Gly Val Ala Leu His Arg Pro Asp Val Tyr Leu Leu Pro Pro Ala Arg
1               5                   10                  15

Glu Gln Leu Asn Leu Arg Glu Ser Ala Thr Ile Thr Cys Leu Val Thr
            20                  25                  30

Gly Phe Ser Pro Ala Asp Val Phe Val Gln Trp Met Gln Arg Gly Gln
        35                  40                  45

Pro Leu Ser Pro Glu Lys Tyr Val Thr Ser Ala Pro Met Pro Glu Pro
    50                  55                  60

Gln Ala Pro Gly Arg Tyr Phe Ala His Ser Ile Leu Thr Val Ser Glu
65                  70                  75                  80

Glu Glu Trp Asn Thr Gly Glu Thr Tyr Thr Cys Val Val Ala His Glu
                85                  90                  95

Ala Leu Pro Asn Arg Val Thr Glu Arg Thr Val Asp Lys Ser Thr Gly
            100                 105                 110

Lys Pro Thr Leu Tyr Asn Val Ser Leu Val Met Ser Asp Thr Ala Gly
        115                 120                 125

Thr Cys Tyr
    130


<210> 254
<211> 12
<212> PRT
<213> Unknown

<220>
<221> source
<223> /note="Description of Unknown:
      Various organism designations"

<400> 254
Asp Ser Ser Asp Ala Phe Thr Leu Asp Glu Leu Arg
1               5                   10


<210> 255
<211> 12
<212> PRT
<213> Otolemur garnettii

<400> 255
Asp Ser Ser His Ser Phe Thr Leu Asp Glu Leu Arg
1               5                   10

<210> 256
<211> 12
<212> PRT
<213> Ictidomys tridecemlineatus

<400> 256
Asp Ser Ser Tyr Ala Phe Thr Leu Asp Glu Leu Arg
1               5                   10


<210> 257
<211> 12
<212> PRT
<213> Ceratotherium simum

<400> 257
Asn Ser Ser His Ala Phe Thr Leu Asp Glu Leu Arg
1               5                   10


<210> 258
<211> 12
<212> PRT
<213> Unknown

<220>
<221> source
<223> /note="Description of Unknown:
      Various organism designations"

<400> 258
Asn Ser Ser Tyr Ala Phe Thr Leu Asp Glu Leu Arg
1               5                   10


<210> 259
<211> 12
<212> PRT
<213> Ochotona princeps

<400> 259
Asp Ser Ser Tyr Ala Phe Pro Leu Asp Glu Leu Arg
1               5                   10


<210> 260
<211> 12
<212> PRT
<213> Tupaia belangeri

<400> 260
Asp Ser Thr His Ala Phe Thr Val Asp Glu Leu Arg
1               5                   10


<210> 261
<211> 12
<212> PRT
<213> Unknown

<220>
<221> source

<223> /note="Description of Unknown:
      Various organism designations"

<400> 261
Asp Ser Ser Tyr Ser Phe Thr Leu Asp Glu Leu Arg
1               5                   10


<210> 262
<211> 12
<212> PRT
<213> Orcinus orca

<400> 262
Asn Ser Ser Asn Ala Phe Thr Leu Asp Glu Leu Arg
1               5                   10


<210> 263
<211> 12
<212> PRT
<213> Dasypus novemcinctus

<400> 263
Asp Ser Ser Tyr Ala Phe Thr Leu Asp Glu Leu Trp
1               5                   10


<210> 264
<211> 12
<212> PRT
<213> Odobenus rosmarus

<400> 264
Asn Ser Ser Ser Ala Phe Thr Leu Asp Glu Leu Arg
1               5                   10


<210> 265
<211> 12
<212> PRT
<213> Unknown

<220>
<221> source
<223> /note="Description of Unknown:
      Cyphellophora europaea sequence"

<400> 265
Ala Thr Ser His Ala Ile Thr Leu Asp Glu Leu Arg
1               5                   10


<210> 266
<211> 12
<212> PRT
<213> Unknown

<220>
<221> source
<223> /note="Description of Unknown:
      Various organism designations"

<400> 266
Asn Ser Ser Tyr Ala Phe Thr Met Asp Glu Leu Arg
1               5                   10


<210> 267
<211> 12
<212> PRT
<213> Unknown

<220>
<221> source
<223> /note="Description of Unknown:
      Various organism designations"

<400> 267
Asp Arg Ser Tyr Ser Phe Thr Leu Asp Glu Leu Arg
1               5                   10


<210> 268
<211> 12
<212> PRT
<213> Oryctolagus cuniculus

<400> 268
Asp Ser Ala Tyr Ala Phe Thr Val Asp Glu Leu Arg
1               5                   10


<210> 269
<211> 12
<212> PRT
<213> Octodon degus

<400> 269
Asp Ser Ser Ser Ala Phe Asn Leu Asn Glu Leu Arg
1               5                   10


<210> 270
<211> 12
<212> PRT
<213> Unknown

<220>
<221> source
<223> /note="Description of Unknown:
      Various organism designations"

<400> 270
Asn Ser Ser Asp Ala Phe Ser Leu Asp Glu Leu Arg
1               5                   10


<210> 271
<211> 12
<212> PRT
<213> Cavia porcellus

<400> 271
Asp Ser Ser Ser Ala Phe Ser Leu Asn Glu Leu Arg
1               5                   10

<210> 272
<211> 12
<212> PRT
<213> Monodelphis domestica

<400> 272
Asn Ser Ser Tyr Ser Phe Thr Leu Asp Glu Leu Arg
1               5                   10


<210> 273
<211> 12
<212> PRT
<213> Unknown

<220>
<221> source
<223> /note="Description of Unknown:
      Various organism designations"

<400> 273
Asn Ser Ser Tyr Ala Phe Thr Val Asp Glu Leu Arg
1               5                   10


<210> 274
<211> 12
<212> PRT
<213> Condylura cristata

<400> 274
Asn Ser Ser Tyr Ala Phe Ser Leu Asp Glu Leu Arg
1               5                   10


<210> 275
<211> 11
<212> PRT
<213> Homo sapiens

<400> 275
Ser Ala Ser Asp Leu Thr Trp Asp Asn Leu Lys
1               5                   10


<210> 276
<211> 11
<212> PRT
<213> Homo sapiens

<400> 276
Ser Asp Thr Ser Leu Thr Trp Asn Ser Val Lys
1               5                   10


<210> 277
<211> 12
<212> PRT
<213> Chinchilla lanigera

<400> 277

Asp Ser Ser Ser Ala Phe Thr Leu Asn Glu Leu Arg
1               5                   10

<210> 278
<211> 4
<212> PRT
<213> Unknown

<220>
<221> source
<223> /note="Description of Unknown:
      Various organism designations"

<400> 278
Leu Asp Glu Leu
1

## Description

### SEQUENCE LISTING

The Sequence Listing associated with this application is provided in tables listed below.

### CROSS-REFERENCE TO RELATED APPLICATIONS

The present application is related to U.S. Provisional Patent Application No. 62/865,166, filed June 22, 2019, U.S. Provisional Patent Application No. US 62/677,008, filed May 27, 2018, U.S. Provisional Patent Application No. 62/506,316, filed May 15, 2017, and U.S. Provisional Patent Application No. 62/335,285, filed May 12, 2016, each of which is incorporated herein by reference in its entirety.

### BACKGROUND

### Technical Field

Embodiments of the present invention relate to compositions and methods for the co-administration of an immunoglobulin Fc region-containing polypeptide and central nervous system (CNS)-targeted antibody or Fc-fusion polypeptide conjugate, for example, to improve pharmacokinetics and/or delivery of the conjugate across the blood-brain barrier (BBB) and into tissues of the central nervous system (CNS).

### Description of the Related Art

Overcoming the difficulties of delivering therapeutic or diagnostic agents to specific regions of the brain represents a major challenge to treatment or diagnosis of many central nervous system (CNS) disorders, including those of the brain. In its neuroprotective role, the blood-brain barrier (BBB) functions to hinder the delivery of many potentially important diagnostic and therapeutic agents to the brain.

Therapeutic molecules and genes that might otherwise be effective in diagnosis and therapy do not cross the BBB in adequate amounts. It is reported that over 95% of all therapeutic molecules do not cross the blood-brain barrier.

Accordingly, there is a need for compositions and methods that facilitate the delivery of therapeutic agents and other molecules across the blood-brain-barrier, for instance, to effectively treat certain diseases of the central nervous system (CNS) such as cancers, particularly those that have metastasized to the CNS. The present invention addresses these needs and offers other related advantages.

### BRIEF SUMMARY OF THE INVENTION

Embodiments of the present invention include pharmaceutical compositions, comprising:
(a) a polypeptide that comprises an immunoglobulin Fc region; and
(b) a conjugate, comprising a blood-brain barrier (BBB)-transport moiety linked to (i) a therapeutic antibody or antigen binding fragment thereof or (ii) a therapeutic Fc-fusion polypeptide.

In certain embodiments, (a) is an antibody, which optionally differs from the antibody of part (b). In certain embodiments, the variable region of the antibody of (a) does not substantially bind to a human antigen.

In certain embodiments, (a) comprises a human immunoglobulin Fc region. In certain embodiments, the human immunoglobulin Fc region comprises, consists, or consists essentially of a sequence from Table A1, including combinations, variants, and fragments thereof. In certain embodiments, the human immunoglobulin Fc region comprises, consists, or consists essentially of an Fc region derived from human IgA1, human IgA2, human IgD, human IgE, human IgG1, human IgG2, human IgG3, human IgG4, or human IgM, optionally as defined by the sequences in Table A1.

In certain embodiments, (b) is a human IgA1 antibody, a human IgA2 antibody, a human IgD antibody, a human IgE antibody, a human IgG1 antibody, a human IgG2 antibody, a human IgG3 antibody, a human IgG4 antibody, or a human IgM antibody, or a fragment thereof that comprise an Fc region or a portion of an Fc region.

In certain embodiments, the BBB-transport moiety is selected from one or more of a p97 (melanotransferrin) polypeptide, a Receptor Associated Protein (RAP), an aprotinin peptide or an analog thereof, a protein transduction domain (PTD), a human low-density lipoprotein receptor (hLDLR) binding peptide or an analog thereof, an antibody or natural ligand that binds to a BBB-associated receptor, and glutathione (GSH).

In certain embodiments, the p97 polypeptide comprises, consists, or consists essentially of a sequence in **Table B1 or B2,** or an active variant or fragment thereof. In specific embodiments, the p97 polypeptide comprises DSSHAFTLDELR (SEQ ID NO:14) or is a soluble human p97 polypeptide.

In certain embodiments, the RAP comprises or consists of a sequence in Table B3, or an active variant or fragment thereof. In certain embodiments, the aprotinin peptide comprises or consists of a sequence in Table B4, or an active variant or fragment thereof. In certain embodiments, the PTD comprises or consists of a sequence in Table B5, or an active variant or fragment. In certain embodiments, the hLDLR binding peptide comprises or consists of a sequence in Table B6, or an active variant or fragment. In certain embodiments, the BBB-associated receptor is selected from one or more of the insulin receptor, the transferrin receptor, the leptin receptor, lipoprotein receptors such as the lipoprotein receptor-related protein (LRP-1) receptor, insulin-like growth factor (IGF) receptors such as IGF1R and IGF2R, the low-density lipoprotein receptor, the diptheria toxin receptor, and TMEM 30A (Flippase). In certain embodiments, the BBB-associated receptor ligand is selected from one or more of insulin, transferrin and transferrin fragments, lactoferrin and lactoferrin fragments, apolipoprotein A (Apo A), apolipoprotein B (Apo B), apolipoprotein E (Apo E), and diptheria toxin (including non-toxic mutants thereof such as CRM45 and CRM197).

In certain embodiments, the antibody or antigen-binding fragment of (b) comprises an immunoglobulin Fc region. In certain embodiments, the antibody or antigen-binding fragment of (b) specifically binds to one or more of human Her2/neu, Her1/EGFR, TNF-α, B7H3 antigen, CD20, VEGF, CD52, CD33, CTLA-4, tenascin, alpha-4 (α4) integrin, IL-23, amyloid-β such as Aβ₍₁₋₄₂₎, Huntingtin, CD25, nerve growth factor (NGF), TrkA, or α-synuclein. In certain embodiments, the antibody or antigen-binding fragment of (b) specifically binds to a cell surface receptor. In certain embodiments, the antibody or antigen-binding fragment of (b) specifically binds to a ligand of a cell surface receptor.

In certain embodiments, the antibody or antigen-binding fragment of (b)specifically binds to a cancer-associated antigen. In certain embodiments, the cancer-associated antigen, or cancer antigen, is selected from one or more of human Her2/neu, Her1/EGF receptor (EGFR), Her3, A33 antigen, B7H3, CD5, CD19, CD20, CD22, CD23 (IgE Receptor), C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin.

In certain embodiments, the antibody is selected from trastuzumab, cetuximab, daclizumab, tanezumab, 3F8, 8H9, abagovomab, adecatumumab, afutuzumab, alemtuzumab, alacizumab (pegol), amatuximab, apolizumab, bavituximab, bectumomab, belimumab, bevacizumab, bivatuzumab (mertansine), brentuximab vedotin, cantuzumab (mertansine), cantuzumab (ravtansine), capromab (pendetide), catumaxomab, citatuzumab (bogatox), cixutumumab, clivatuzumab (tetraxetan), conatumumab, dacetuzumab, dalotuzumab, detumomab, drozitumab, ecromeximab, edrecolomab, elotuzumab, enavatuzumab, ensituximab, epratuzumab, ertumaxomab, etaracizumab, farletuzumab, FBTA05, figitumumab, flanvotumab, galiximab, gemtuzumab, ganitumab, gemtuzumab (ozogamicin), girentuximab, glembatumumab (vedotin), ibritumomab tiuxetan, icrucumab, igovomab, indatuximab ravtansine, intetumumab, inotuzumab ozogamicin, ipilimumab (MDX-101), iratumumab, labetuzumab, lexatumumab, lintuzumab, lorvotuzumab (mertansine), lucatumumab, lumiliximab, mapatumumab, matuzumab, milatuzumab, mitumomab, mogamulizumab, moxetumomab (pasudotox), nacolomab (tafenatox), naptumomab (estafenatox), narnatumab, necitumumab, nimotuzumab, nivolumab, Neuradiab^{®} (with or without radioactive iodine), NR-LU-10, ofatumumab, olaratumab, onartuzumab, oportuzumab (monatox), oregovomab, panitumumab, patritumab, pemtumomab, pertuzumab, pritumumab, racotumomab, radretumab, ramucirumab, rilotumumab, rituximab, robatumumab, samalizumab, sibrotuzumab, siltuximab, tabalumab, taplitumomab (paptox), tenatumomab, teprotumumab, TGN1412, ticilimumab, tremelimumab, tigatuzumab, TNX-650, tositumomab, TRBS07, tucotuzumab (celmoleukin), ublituximab, urelumab, veltuzumab, volociximab, votumumab, and zalutumumab, and fragments, variants, and derivatives of the foregoing.

In certain embodiments, the antibody or antigen-binding fragment of (b)specifically binds to antigen associated with at least one nervous system disorder. In certain embodiments, the antibody or antigen-binding fragment of (b) specifically binds to antigen associated with pain. In certain embodiments, the antibody or antigen-binding fragment of (b)specifically binds to antigen associated with autoimmune disorder, optionally an autoimmune disorder of the nervous system or CNS. In certain embodiments, the antigen is selected from one or more of alpha-4 (α4) integrin, CD20, CD52, IL-12, IL-23, the p40 subunit of IL-12 and IL-23, and the axonal regrowth and remyelination inhibitors Nogo-A and LINGO, IL-23, amyloid-β optionally Aβ(1-42), Huntingtin, CD25, nerve growth factor (NGF), neurotrophic tyrosine kinase receptor type 1 (TrkA; the high affinity catalytic receptor for NGF), and α-synuclein.

In certain embodiments, the antibody or antigen-binding fragment of (b) specifically binds to a pro-inflammatory cytokine or chemokine, or a receptor thereof. In certain embodiments, the pro-inflammatory cytokine or chemokine is selected from TNF-α, TNF-β, FasL, CD27L, CD30L, CD40L, Ox40L, 4-1BBL, TRAIL, TWEAK, and Apo3L, IL-1α, IL-1β, IL-2, interferon-y (IFN-y), IFN-α/β, IL-6, IL-8, IL-12, IL-15, IL-17, IL-18, IL-21, LIF, CCL5, GROα, MCP-1, MIP-1α, MIP-1β, macrophage colony stimulating factor (MCSF), granulocyte macrophage colony stimulating factor (GM-CSF), CXCL2, and CCL2.

In certain embodiments, the antibody is adalimumab (Humira^{®}), certolizumab pegol (Cimzia^{®}), golimumab (Cimzia^{®}), infliximab (Remicade^{®}), D2E7, CDP 571, or CDP 870, or an antigen-binding fragment or variant thereof.

In certain embodiments, the receptor is selected from TNF receptor (TNFR), an IL-1 receptor (IL-1R), and an IL-6 receptor (IL-6R).

In certain embodiments, the Fc-fusion polypeptide is selected from etanercept, abatercept, aflibercept, alefacept, belatacept, rilonacept, and romiplastin.

In certain embodiments, the pharmaceutical composition is substantially endotoxin-free.

Also included are methods of treating a subject in need thereof, comprising administering to the subject
(a) a polypeptide that comprises an immunoglobulin Fc region; and
(b) a conjugate, comprising a blood-brain barrier (BBB)-transport moiety linked to (i) a therapeutic antibody or antigen binding fragment thereof or (ii) a therapeutic Fc-fusion polypeptide.

In certain embodiments, (a) and (b) are administered systemically, optionally intravenously. In certain embodiments, (a) and (b) are administered separately, and are optionally defined as described herein. In certain embodiments, (a) is administered prior to (b).

In certain embodiments, (a) is administered about, at least about, or no more than about 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 200, 220, 240, 260, 280, or 300 minutes before (b).

In certain embodiments, (a) is administered about, at least about, or no more than about 1, 2, 3, 4, 5, 6, 7, 8, 9, 10, 11, 12, 16, 24, 30, 36, 42, 48, 60, 72, 84, or 96 hours before (b).

In certain embodiments, (a) and (b) are administered together, optionally as a pharmaceutical composition as described herein.

In certain embodiments, systemic administration of (a) and (b) increases delivery of (a) across the BBB and into tissues of the CNS relative to systemic administration of (a) alone.

Certain embodiments include methods for treating a cancer of the central nervous system (CNS), optionally the brain. Certain embodiments include methods for treating primary cancer of the CNS, optionally the brain. Certain embodiments include methods for treating a metastatic cancer of the CNS, optionally the brain. Certain embodiments include methods for treating a glioma, meningioma, pituitary adenoma, vestibular schwannoma, primary CNS lymphoma, neuroblastoma, or primitive neuroectodermal tumor (medulloblastoma). In some embodiments, the glioma is an astrocytoma, oligodendroglioma, ependymoma, or a choroid plexus papilloma. Certain embodiments include methods for treating glioblastoma multiforme. In some embodiments, the glioblastoma multiforme is a giant cell gliobastoma or a gliosarcoma.

Certain embodiments include methods for treating a degenerative or autoimmune disorder of the central nervous system (CNS). In some embodiments, the degenerative or autoimmune disorder of the CNS is Alzheimer's disease, Huntington's disease, Parkinson's disease, or multiple sclerosis (MS).

Certain embodiments include methods for treating pain. In some embodiments, the pain is acute pain, chronic pain, neuropathic pain, and/or central pain.

Certain embodiments include methods for treating an inflammatory condition. In some embodiments, the inflammatory condition has a central nervous system component. In some embodiments, the inflammatory condition is one or more of meningitis, myelitis, encephalomyelitis, arachnoiditis, sarcoidosis, granuloma, drug-induced inflammation, Alzheimer's disease, stroke, HIV-dementia, encephalitis, parasitic infection, an inflammatory demyelinating disorder, a CD8+ T Cell-mediated autoimmune disease of the CNS, Parkinson's disease, myasthenia gravis, motor neuropathy, Guillain-Barre syndrome, autoimmune neuropathy, Lambert-Eaton myasthenic syndrome, paraneoplastic neurological disease, paraneoplastic cerebellar atrophy, non-paraneoplastic stiff man syndrome, progressive cerebellar atrophy, Rasmussen's encephalitis, amyotrophic lateral sclerosis, Sydeham chorea, Gilles de la Tourette syndrome, autoimmune polyendocrinopathy, dysimmune neuropathy, acquired neuromyotonia, arthrogryposis multiplex, optic neuritis, stroke, traumatic brain injury (TBI), spinal stenosis, acute spinal cord injury, and spinal cord compression.

In particular embodiments, the inflammatory condition is associated with an infection of the central nervous system. In some embodiments, the infection is a bacterial infection caused by one or more of *group B streptococci* (*e.g.,* subtypes III), *Streptococcus pneumoniae* (*e.g.,* serotypes 6, 9, 14, 18 and 23), *Escherichia coli* (*e.g.,* carrying K1 antigen), *Listeria monocytogenes* (*e.g.,* serotype IVb), neisserial infection such as *Neisseria meningitidis* (meningococcus), staphylococcal infection, heamophilus infection such as *Haemophilus influenzae* type B, *Klebsiella, Mycobacterium tuberculosis, Treponema pallidum,* or *Borrelia burgdorferi.*

In some embodiments, the infection is a viral infection caused by one or more of an enterovirus, herpes simplex virus type 1 or 2, human T-lymphotrophic virus, varicella zoster virus, mumps virus, human immunodeficiency virus (HIV), or lymphocytic choriomeningitis virus (LCMV).

In some embodiments, the inflammatory condition is associated with a cancer of the CNS, optionally a malignant meningitis.

Also included are patient care kits, comprising:
(a) a polypeptide that comprises an immunoglobulin Fc region; and
(b) a conjugate, comprising a blood-brain barrier (BBB)-transport moiety linked to (i) a therapeutic antibody or antigen binding fragment thereof or (ii) a therapeutic Fc-fusion polypeptide.

In some embodiments, (a) and (b) are in separate pharmaceutical compositions, and are optionally defined as described herein. In some embodiments, (a) and (b) are in the same composition, optionally as a pharmaceutical composition described herein.

### Detailed Description of the Invention

Embodiments of the present invention are based partly on the theory that central nervous system (CNS)-targeting of antibody or Fc-fusion polypeptide-based conjugates, which comprise a BBB-transport moiety, can be improved by co-administration with a separate polypeptide that comprises an immunoglobulin Fc region. It is believed that the latter will bind, for example, to systemic Fc receptors or ligands in the subject, and thereby alter (e.g., reduce) the interaction between the Fc regions of the conjugate and the Fc receptors/ligands in the subject. Such is expected to enhance the ability of the BBB-transport moiety to facilitate delivery of the conjugate across the BBB, for instance, by minimizing the interactions between the conjugate's Fc region and its ligands and favoring the interaction between the BBB-transport moiety of the conjugate and its BBB-associated receptor(s) or ligand(s).

The practice of the present invention will employ, unless indicated specifically to the contrary, conventional methods of molecular biology and recombinant DNA techniques within the skill of the art, many of which are described below for the purpose of illustration. Such techniques are explained fully in the literature. *See, e.g.,* Sambrook, et al., Molecular Cloning: A Laboratory Manual (3rd Edition, 2000); DNA Cloning: A Practical Approach, vol. I & II (D. Glover, ed.); Oligonucleotide Synthesis (N. Gait, ed., 1984); Oligonucleotide Synthesis: Methods and Applications (P. Herdewijn, ed., 2004); Nucleic Acid Hybridization (B. Hames & S. Higgins, eds., 1985); Nucleic Acid Hybridization: Modern Applications (Buzdin and Lukyanov, eds., 2009); Transcription and Translation (B. Hames & S. Higgins, eds., 1984); Animal Cell Culture (R. Freshney, ed., 1986); Freshney, R.I. (2005) Culture of Animal Cells, a Manual of Basic Technique, 5th Ed. Hoboken NJ, John Wiley & Sons; B. Perbal, A Practical Guide to Molecular Cloning (3rd Edition 2010); Farrell, R., RNA Methodologies: A Laboratory Guide for Isolation and Characterization (3rd Edition 2005).

All publications, patents, and patent applications cited herein are hereby incorporated by reference in their entirety.

### Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (*i.e.,* to at least one) of the grammatical object of the article. By way of example, "an element" means one element or more than one element.

By **"about"** is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

As used herein, the term **"amino acid"** is intended to mean both naturally occurring and non-naturally occurring amino acids as well as amino acid analogs and mimetics. Naturally occurring amino acids include the 20 (L)-amino acids utilized during protein biosynthesis as well as others such as 4-hydroxyproline, hydroxylysine, desmosine, isodesmosine, homocysteine, citrulline and ornithine, for example. Non-naturally occurring amino acids include, for example, (D)-amino acids, norleucine, norvaline, p-fluorophenylalanine, ethionine and the like, which are known to a person skilled in the art. Amino acid analogs include modified forms of naturally and non-naturally occurring amino acids. Such modifications can include, for example, substitution or replacement of chemical groups and moieties on the amino acid or by derivatization of the amino acid. Amino acid mimetics include, for example, organic structures which exhibit functionally similar properties such as charge and charge spacing characteristic of the reference amino acid. For example, an organic structure which mimics Arginine (Arg or R) would have a positive charge moiety located in similar molecular space and having the same degree of mobility as the e-amino group of the side chain of the naturally occurring Arg amino acid. Mimetics also include constrained structures so as to maintain optimal spacing and charge interactions of the amino acid or of the amino acid functional groups. Those skilled in the art know or can determine what structures constitute functionally equivalent amino acid analogs and amino acid mimetics.

Throughout this specification, unless the context requires otherwise, the words **"comprise," "comprises,"** and **"comprising"** will be understood to imply the inclusion of a stated step or element or group of steps or elements but not the exclusion of any other step or element or group of steps or elements. By **"consisting of"** is meant including, and limited to, whatever follows the phrase "consisting of."Thus, the phrase "consisting of" indicates that the listed elements are required or mandatory, and that no other elements may be present. By **"consisting essentially of"** is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

The term **"conjugate"** is intended to refer to the entity formed as a result of covalent or non-covalent attachment or linkage of an agent or other molecule, *e.g*., a biologically active molecule, to a BBB-moiety, as described herein. One example of a conjugate polypeptide is a **"fusion protein"** or **"fusion polypeptide,"** that is, a polypeptide that is created through the joining of two or more coding sequences, which originally coded for separate polypeptides; translation of the joined coding sequences results in a single, fusion polypeptide, typically with functional properties derived from each of the separate polypeptides.

The term **"endotoxin free"** or **"substantially endotoxin free"** relates generally to compositions, solvents, and/or vessels that contain at most trace amounts (*e.g*., amounts having no clinically adverse physiological effects to a subject) of endotoxin, and preferably undetectable amounts of endotoxin. Endotoxins are toxins associated with certain micro-organisms, such as bacteria, typically gram-negative bacteria, although endotoxins may be found in gram-positive bacteria, such as *Listeria monocytogenes.* The most prevalent endotoxins are lipopolysaccharides (LPS) or lipo-oligo-saccharides (LOS) found in the outer membrane of various Gram-negative bacteria, and which represent a central pathogenic feature in the ability of these bacteria to cause disease. Small amounts of endotoxin in humans may produce fever, a lowering of the blood pressure, and activation of inflammation and coagulation, among other adverse physiological effects.

Therefore, in pharmaceutical production, it is often desirable to remove most or all traces of endotoxin from drug products and/or drug containers, because even small amounts may cause adverse effects in humans. A depyrogenation oven may be used for this purpose, as temperatures in excess of 300°C are typically required to break down most endotoxins. For instance, based on primary packaging material such as syringes or vials, the combination of a glass temperature of 250°C and a holding time of 30 minutes is often sufficient to achieve a 3 log reduction in endotoxin levels. Other methods of removing endotoxins are contemplated, including, for example, chromatography and filtration methods, as described herein and known in the art.

Endotoxins can be detected using routine techniques known in the art. For example, the Limulus Amoebocyte Lysate assay, which utilizes blood from the horseshoe crab, is a very sensitive assay for detecting presence of endotoxin. In this test, very low levels of LPS can cause detectable coagulation of the limulus lysate due a powerful enzymatic cascade that amplifies this reaction. Endotoxins can also be quantitated by enzyme-linked immunosorbent assay (ELISA). To be substantially endotoxin free, endotoxin levels may be less than about 0.001, 0.005, 0.01, 0.02, 0.03, 0.04, 0.05, 0.06, 0.08, 0.09, 0.1, 0.5, 1.0, 1.5, 2, 2.5, 3, 4, 5, 6, 7, 8, 9, or 10 EU/mg of active compound. Typically, 1 ng lipopolysaccharide (LPS) corresponds to about 1-10 EU.

As used herein, the terms **"function"** and **"functional"** and the like refer to a biological, enzymatic, or therapeutic function.

**"Homology"** refers to the percentage number of amino acids that are identical or constitute conservative substitutions. Homology may be determined using sequence comparison programs such as GAP (Deveraux et al., Nucleic Acids Research. 12, 387-395, 1984), which is incorporated herein by reference. In this way sequences of a similar or substantially different length to those cited herein could be compared by insertion of gaps into the alignment, such gaps being determined, for example, by the comparison algorithm used by GAP.

By **"isolated"** is meant material that is substantially or essentially free from components that normally accompany it in its native state. For example, an "isolated peptide" or an "isolated polypeptide" and the like, as used herein, includes the *in vitro* isolation and/or purification of a peptide or polypeptide molecule from its natural cellular environment, and from association with other components of the cell; *i.e.,* it is not significantly associated with *in vivo* substances.

The term **"linkage," "linker," "linker moiety,"** or **"L"** is used herein to refer to a linker that can be used to separate a BBB-moiety from an agent of interest, or to separate a first agent from another agent, for instance where two or more agents are linked to form a conjugate. The linker may be physiologically stable or may include a releasable linker such as an enzymatically degradable linker (e.g., proteolytically cleavable linkers). In certain aspects, the linker may be a peptide linker, for instance, as part of a protein. In some aspects, the linker may be a non-peptide linker or non-proteinaceous linker. In some aspects, the linker may be particle, such as a nanoparticle.

A **"lipid nanoparticle"** or **"solid lipid nanoparticle"** refers to one or more spherical nanoparticles with an average diameter of between about 10 to about 1000 nanometers, and which comprise a solid lipid core matrix that can solubilize lipophilic molecules. The lipid core is stabilized by surfactants (e.g., emulsifiers), and can comprise one or more of triglycerides (e.g., tristearin), diglycerides (e.g., glycerol bahenate), monoglycerides (e.g., glycerol monostearate), fatty acids (e.g., stearic acid), steroids (e.g., cholesterol), and waxes (e.g., cetyl palmitate), including combinations thereof. Lipid nanoparticles are described, for example, in Petrilli et al., Curr Pharm Biotechnol. 15:847-55, 2014; and U.S. Patent Nos. 6,217,912; 6,881,421; 7,402,573; 7,404,969; 7,550,441; 7,727,969; 8,003,621; 8,691,750; 8,871,509; 9,017,726; 9,173,853; 9,220,779; 9,227,917; and 9,278,130, which are incorporated by reference in their entireties.

The terms **"modulating"** and **"altering"** include "increasing," "enhancing" or "stimulating," as well as "decreasing" or "reducing," typically in a statistically significant or a physiologically significant amount or degree relative to a control. An **"increased," "stimulated"** or **"enhanced"** amount is typically a "statistically significant" amount, and may include an increase that is 1.1, 1.2, 2, 3, 4, 5, 6, 7, 8, 9, 10, 15, 20, 30 or more times (e.g., 500, 1000 times) (including all integers and decimal points in between and above 1, *e.g.,* 1.5, 1.6, 1.7. 1.8, etc.) the amount produced by no composition or a control composition, sample or test subject. A **"decreased"** or **"reduced"** amount is typically a **"statistically significant"** amount, and may include a 1%, 2%, 3%, 4%, 5%, 6%, 7%, 8%, 9%, 10%, 11%, 12%, 13%, 14%, 15%, 16%, 17%, 18% , 19%, 20%, 25%, 30%, 35%, 40%, 45%, 50%, 55%, 60%, 65%, 70%, 75%, 80%, 85%, 90%, 95%, or 100% decrease in the amount produced by no composition or a control composition, including all integers in between. As one non-limiting example, a control could compare the activity of a conjugate, such as the amount or rate of transport/delivery of the conjugate across the blood brain barrier, the rate and/or levels of distribution of the conjugate to central nervous system tissue, and/or the Cₘₐₓ of the conjugate in plasma, central nervous system tissues, or any other systemic or peripheral non-central nervous system tissues, after administering the conjugate in combination with an Fc region-containing polypeptide relative to administering the conjugate alone. Other examples of comparisons and "statistically significant" amounts are described herein or will be apparent to persons skilled in the art.

In certain embodiments, the **"purity"** of any given agent (*e.g*., a conjugate, Fc region-containing polypeptide) in a composition may be specifically defined. For instance, certain compositions may comprise an agent that is at least 80%, 85%, 90%, 91%, 92%, 93%, 94%, 95%, 96%, 97%, 98%, 99%, or 100% pure, including all decimals in between, as measured, for example and by no means limiting, by high pressure liquid chromatography (HPLC), a well-known form of column chromatography used frequently in biochemistry and analytical chemistry to separate, identify, and quantify compounds.

The terms **"polypeptide"** and **"protein"** are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers. The polypeptides described herein are not limited to a specific length of the product; thus, peptides, oligopeptides, and proteins are included within the definition of polypeptide, and such terms may be used interchangeably herein unless specifically indicated otherwise. The polypeptides described herein may also comprise post-expression modifications, such as glycosylations, acetylations, phosphorylations and the like, as well as other modifications known in the art, both naturally occurring and non-naturally occurring. A polypeptide may be an entire protein, or a subsequence, fragment, variant, or derivative thereof.

A **"physiologically cleavable"** or **"hydrolyzable"** or **"degradable"** bond is a bond that reacts with water (*i.e*., is hydrolyzed) under physiological conditions. The tendency of a bond to hydrolyze in water will depend not only on the general type of linkage connecting two central atoms but also on the substituents attached to these central atoms. Appropriate hydrolytically unstable or weak linkages include, but are not limited to: carboxylate ester, phosphate ester, anhydride, acetal, ketal, acyloxyalkyl ether, imine, orthoester, thio ester, thiol ester, carbonate, and hydrazone, peptides and oligonucleotides.

A **"releasable linker"** includes, but is not limited to, a physiologically cleavable linker and an enzymatically degradable linker. Thus, a "releasable linker" is a linker that may undergo either spontaneous hydrolysis, or cleavage by some other mechanism (*e.g*., enzyme-catalyzed, acid-catalyzed, base-catalyzed, and so forth) under physiological conditions. For example, a "releasable linker" can involve an elimination reaction that has a base abstraction of a proton, (*e.g*., an ionizable hydrogen atom, Hα), as the driving force. For purposes herein, a "releasable linker" is synonymous with a "degradable linker." An **"enzymatically degradable linkage"** includes a linkage, *e.g*., amino acid sequence that is subject to degradation by one or more enzymes, *e.g*., peptidases or proteases. In particular embodiments, a releasable linker has a half life at pH 7.4, 25°C, *e.g.,* a physiological pH, human body temperature (*e.g*., in vivo), of about 30 minutes, about 1 hour, about 2 hour, about 3 hours, about 4 hours, about 5 hours, about 6 hours, about 12 hours, about 18 hours, about 24 hours, about 36 hours, about 48 hours, about 72 hours, or about 96 hours or less.

The term **"reference sequence"** refers generally to a nucleic acid coding sequence, or amino acid sequence, to which another sequence is being compared. All polypeptide and polynucleotide sequences described herein are included as references sequences, including those described by name and those described in the Sequence Listing.

The terms **"sequence identity"** or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (*e.g*., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (*i.e.,* the window size), and multiplying the result by 100 to yield the percentage of sequence identity. Included are nucleotides and polypeptides having at least about 50%, 55%, 60%, 65%, 70%, 75%, 80%, 85%, 90%, 95%, 97%, 98%, 99%, or 100% sequence identity to any of the reference sequences described herein *(see, e.g.,* Sequence Listing), typically where the polypeptide variant maintains at least one biological activity of the reference polypeptide.

Terms used to describe sequence relationships between two or more polynucleotides or polypeptides include "reference sequence," "comparison window," "sequence identity," "percentage of sequence identity," and "substantial identity." A "reference sequence" is at least 12 but frequently 15 to 18 and often at least 25 monomer units, inclusive of nucleotides and amino acid residues, in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (i.e., gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (*i.e*., resulting in the highest percentage homology over the comparison window) generated by any of the various methods selected. Reference also may be made to the BLAST family of programs as for example disclosed by Altschul et al., Nucl. Acids Res. 25:3389, 1997. A detailed discussion of sequence analysis can be found in Unit 19.3 of Ausubel et al., "Current Protocols in Molecular Biology," John Wiley & Sons Inc, 1994-1998, Chapter 15.

By **"statistically significant,"** it is meant that the result was unlikely to have occurred by chance. Statistical significance can be determined by any method known in the art. Commonly used measures of significance include the p-value, which is the frequency or probability with which the observed event would occur, if the null hypothesis were true. If the obtained p-value is smaller than the significance level, then the null hypothesis is rejected. In simple cases, the significance level is defined at a p-value of 0.05 or less.

The term **"solubility"** refers to the property of a protein to dissolve in a liquid solvent and form a homogeneous solution. Solubility is typically expressed as a concentration, either by mass of solute per unit volume of solvent (g of solute per kg of solvent, g per dL (100 mL), mg/ml, etc.), molarity, molality, mole fraction or other similar descriptions of concentration. The maximum equilibrium amount of solute that can dissolve per amount of solvent is the solubility of that solute in that solvent under the specified conditions, including temperature, pressure, pH, and the nature of the solvent. In certain embodiments, solubility is measured at physiological pH, or other pH, for example, at pH 5.0, pH 6.0, pH 7.0, or pH 7.4. In certain embodiments, solubility is measured in water or a physiological buffer such as PBS or NaCl (with or without NaP). In specific embodiments, solubility is measured at relatively lower pH (*e.g*., pH 6.0) and relatively higher salt (*e.g*., 500mM NaCl and 10mM NaP). In certain embodiments, solubility is measured in a biological fluid (solvent) such as blood or serum. In certain embodiments, the temperature can be about room temperature (*e.g*., about 20, 21, 22, 23, 24, 25°C) or about body temperature (∼37°C). In certain embodiments, a conjugate, polypeptide, or polypeptide-based conjugate has a solubility of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, or 30 mg/ml at room temperature or at about 37°C.

A **"subject,"** as used herein, includes any animal that exhibits a symptom, or is at risk for exhibiting a symptom, which can be treated or diagnosed with a conjugate of the invention. Suitable subjects (patients) include laboratory animals (such as mouse, rat, rabbit, or guinea pig), farm animals, and domestic animals or pets (such as a cat or dog). Non-human primates and, preferably, human patients, are included.

**"Substantially"** or **"essentially"** means nearly totally or completely, for instance, 95%, 96%, 97%, 98%, 99% or greater of some given quantity.

**"Substantially free"** refers to the nearly complete or complete absence of a given quantity for instance, less than about 10%, 5%, 4%, 3%, 2%, 1%, 0.5% or less of some given quantity. For example, certain compositions may be "substantially free" of cell proteins, membranes, nucleic acids, endotoxins, or other contaminants.

As used herein, the terms **"therapeutically effective amount", "therapeutic dose," "prophylactically effective amount,"** or **"diagnostically effective amount"** is the amount of an agent (e.g., conjugate, Fc region-containing polypeptide) needed to elicit the desired biological response following administration.

**"Treatment"** or **"treating,"** as used herein, includes any desirable effect on the symptoms or pathology of a disease or condition, and may include even minimal changes or improvements in one or more measurable markers of the disease or condition being treated. "Treatment" or "treating" does not necessarily indicate complete eradication or cure of the disease or condition, or associated symptoms thereof. The subject receiving this treatment is any subject in need thereof. Exemplary markers of clinical improvement will be apparent to persons skilled in the art.

The term **"wild-type"** refers to a gene or gene product that has the characteristics of that gene or gene product when isolated from a naturally-occurring source. A wild type gene or gene product (*e.g*., a polypeptide) is that which is most frequently observed in a population and is thus arbitrarily designed the "normal" or "wild-type" form of the gene.

### Fc Region-Containing Polypeptides

Certain methods and compositions employ or comprise at least one "polypeptide that comprise an immunoglobulin Fc region" or a portion of an Fc region (also referred to as an "Fc region-containing polypeptide"). General examples of Fc region-containing polypeptides include antibodies, for example, whole antibodies, and fragments thereof that comprise an Fc region or a portion thereof. Fc region-containing polypeptides can also comprise heterologous sequences, for example, non-immunoglobulin polypeptide sequences.

In particular embodiments, an "Fc region-containing polypeptide" is an antibody or a fragment thereof which comprises an Fc region or a portion of an Fc region. In some embodiments, the antibody differs from the antibody of the conjugate. For instance, in some instances, the variable region of the antibody or fragment thereof binds to a different antigen than the antibody of the conjugate. In some embodiments, the antibody or fragment thereof does not substantially or specifically bind to a human antigen. In some embodiments, the antibody or fragment thereof binds to a non-human antigen. In certain embodiments, the antibody is a human antibody, or a humanized antibody.

The "Fc region" of a polypeptide, for example, an antibody, is usually derived from the heavy chain of an immunoglobulin (Ig) molecule. A typical Ig molecule is composed of two heavy chains and two light chains. The heavy chains can be divided into at least three functional regions: the Fd region, the Fc region (fragment crystallizable region), and the hinge region, the latter being found only in IgG, IgA, and IgD immunoglobulins. The Fd region comprises the variable (VH) and constant (CH1) domains of the heavy chains, and together with the variable (VL) and constant (CL) domains of the light chains forms the antigen-binding fragment or Fab region.

The Fc region of IgG, IgA, and IgD immunoglobulins comprises the heavy chain constant domains 2 and 3, designated respectively as CH2 and CH3 regions; and the Fc region of IgE and IgM immunoglobulins comprises the heavy chain constant domains 2, 3, and 4, designated respectively as CH2, CH3, and CH4 regions. The Fc region is mainly responsible for the immunoglobulin effector functions, which include, for example, complement fixation and binding to cognate Fc receptors of effector cells.

The hinge region (found in IgG, IgA, and IgD) acts as a flexible spacer that allows the Fab portion to move freely in space relative to the Fc region. In contrast to the constant regions, the hinge regions are structurally diverse, varying in both sequence and length among immunoglobulin classes and subclasses. The hinge region may also contain one or more glycosylation site(s), which include a number of structurally distinct types of sites for carbohydrate attachment. For example, IgA1 contains five glycosylation sites within a 17 amino acid segment of the hinge region, conferring significant resistance of the hinge region polypeptide to intestinal proteases. Residues in the hinge proximal region of the CH2 domain can also influence the specificity of the interaction between an immunoglobulin and its respective Fc receptor(s) (see, e.g., Shin et al., Intern. Rev. Immunol. 10:177-186, 1993).

The term "Fc region" or "Fc fragment" or "Fc" as used herein, thus refers to a polypeptide that comprises one or more of a CH2 region, a CH3 region, and/or a CH4 region from one or more selected immunoglobulin(s), including fragments and variants and combinations thereof. An "Fc region" may also include one or more hinge region(s) of the heavy chain constant region of an immunoglobulin. In some embodiments, the Fc region does not contain one or more of the CH1, CL, VL, and/or VH regions of an immunoglobulin.

The Fc region of a polypeptide can be derived from the CH2 region, CH3 region, CH4 region, and/or hinge region(s) of any one or more immunoglobulin classes, including but not limited to IgA, IgD, IgE, IgG, IgM, including subclasses and combinations thereof. In some embodiments, the Fc region is derived from an IgA immunoglobulin, including subclasses IgA1 and/or IgA2. In certain embodiments, the Fc region is derived from an IgD immunoglobulin. In particular embodiments, the Fc region is derived from an IgE immunoglobulin. In some embodiments, the Fc region is derived from an IgG immunoglobulin, including subclasses IgG1, IgG2, IgG2, IgG3, and/or IgG4. In certain embodiments, the Fc region is derived from an IgM immunoglobulin.

Certain Fc regions demonstrate specific binding for one or more Fc-receptors (FcRs). Examples of classes of Fc receptors include Fcγ receptors (FcγR), Fcα receptors (FcαR), Fcε receptors (FcεR), and the neonatal Fc receptor (FcRn). For instance, certain Fc regions have increased binding to (or affinity for) one or more FcyRs, relative to FcaRs, FcεRs, and/or FcRn. In some embodiments, Fc regions have increased binding to FcaRs, relative to one or more FcyRs, FcεRs, and/or FcRn. In other embodiments, Fc regions have increased binding to FcεRs (e.g., FcaRI), relative to one or more FcyRs, FcaRs, and/or FcRn. In particular embodiments, Fc regions have increased binding to FcRn, relative to one or more FcγRs, FcaRs, and/or FcεRs.

Examples of FcγRs include FcγRI, FcγRIIa, FcγRIIb, FcγRIIc, FcγRIIIa, and FcγRIIIb. FcγRI (CD64) is expressed on macrophages and dendritic cells and plays a role in phagocytosis, respiratory burst, cytokine stimulation, and dendritic cell endocytic transport. Expression of FcγRI is upregulated by both GM-CSF and γ-interferon (γ-IFN) and downregulated by interleukin-4 (IL-4). FcyRlla is expressed on polymorphonuclear leukocytes (PMN), macrophages, dendritic cells, and mast cells. FcγRIIa plays a role in phagocytosis, respiratory burst, and cytokine stimulation. Expression of FcyRlla is upregulated by GM-CSF and γ-IFN, and decreased by IL-4. FcγIIb is expressed on B cells, PMN, macrophages, and mast cells. FcγIIb inhibits immunoreceptor tyrosine-based activation motif (ITAM) mediated responses, and is thus an inhibitory receptor. Expression of FcγRIIc is upregulated by intravenous immunoglobulin (IVIG) and IL-4 and decreased by γ-IFN. FcγRIIc is expressed on NK cells. FcγRIIIa is expressed on natural killer (NK) cells, macrophages, mast cells, and platelets. This receptor participates in phagocytosis, respiratory burst, cytokine stimulation, platelet aggregation and degranulation, and NK-mediated ADCC. Expression of FcγRIII is upregulated by C5a, TGF-β, and γ-IFN and downregulated by IL-4. Fc γ RIIIb is a GPI-linked receptor expressed on PMN.

Certain Fc regions have increased binding to FcγRI, relative to FcγRIIa, FcγRIIb, FcγRIIc, FcγRIIIa, and/or FcγRIIIb. Some embodiments have increased binding to FcγRIIa, relative to FcγRI, FcγRIIb, FcγRIIc, FcγRIIIa, and/or FcγRIIIb. Particular Fc regions have increased binding to FcγRIIb, relative to FcγRI, FcγRIIa, FcγRIIc, FcγRIIIa, and/or FcγRIIIb. Certain Fc regions have increased binding to FcγRIIc, relative to FcγRI, FcγRIIa, FcγRIIb, FcγRIIIa, and/or FcγRIIIb. Some Fc regions have increased binding to FcγRIIIa, relative to FcγRI, FcγRIIa, FcγRIIb, FcγRIIc, and/or FcγRIIIb. Specific Fc regions have increased binding to FcγRIIIb, relative to FcγRI, FcγRIIa, FcγRIIb, FcγRIIc, and/or FcγRIIIa.

FcαRs include FcαRI (CD89). FcαRI is found on the surface of neutrophils, eosinophils, monocytes, certain macrophages (e.g., Kupffer cells), and certain dendritic cells. FcαRI is composed of two extracellular Ig-like domains, is a member of both the immunoglobulin superfamily and the multichain immune recognition receptor (MIRR) family, and signals by associating with two FcRγ signaling chains.

FcεRs include FcεRI and FcεRII. The high-affinity receptor FcεRI is a member of the immunoglobulin superfamily, is expressed on epidermal Langerhans cells, eosinophils, mast cells and basophils, and plays a major role in controlling allergic responses. FcεRI is also expressed on antigen-presenting cells, and regulates the production pro-inflammatory cytokines. The low-affinity receptor FcεRII (CD23) is a C-type lectin that can function as a membrane-bound or soluble receptor. FcεRII regulates B cell growth and differentiation, and blocks IgE-binding of eosinophils, monocytes, and basophils. Certain Fc regions have increased binding to FcεRI, relative to FcεRII. Other Fc regions have increased binding to FcεRII, relative to FcεRI.

Fc regions can be derived from the immunoglobulin molecules of any animal, including vertebrates such as mammals such cows, goats, swine, dogs, mice, rabbits, hamsters, rats, guinea pigs, non-human primates, and humans. In some embodiments, the Fc region-containing polypeptide comprises a human immunoglobulin Fc region, which optionally comprises or does not comprise the variable or binding regions of an immunoglobulin. The amino acid sequences of CH₂, CH₃, CH₄, and hinge regions from exemplary, wild-type human IgA1, IgA2, IgD, IgE, IgG1, IgG2, IgG3, IgG4, and IgM immunoglobulins are provided in **Table A1** below.

| **Table A1. Exemplary Fc Region Sequences** | | |
|---|---|---|
| Name | Sequence | SEQ ID NO: |
| human IgA1 hinge region | VPSTPPTPSPSTPPTPSPS | 225 |
| human IgA1 CH2 region | CCHPRLSLHRPALEDLLLGSEANLTCTLTGLRDASGVTFTWTPSSGKSAVQGPPERDLCGCYSVS SVLPGCAEPWNHGKTFTCTAAYPESKTPLTATLSKS | 226 |
| human IgA1 CH3 region | GNTFRPEVHLLPPPSEELALNELVTLTCLARGFSPKDVLVRWLQGSQELPREKYLTWASRQEPS QGTTTFAVTSILRVAAEDWKKGDTFSCMVGHEALPLAFTQKTIDRLAGKPTHVNVSVVMAEVD GTCY | 227 |
| human IgA2 hinge region | VPPPPP | 228 |
| human IgA2 CH2 | CCHPRLSLHRPALEDLLLGSEANLTCTLTGLRDASGATFTWTPSSGKSAVQGPPERDLCGCYSVS SVLPGCAQPWNHGETFTCTAAHPELKTPLTANITKS | 229 |
| human IgA2 CH3 | GNTFRPEVHLLPPPSEELALNELVTLTCLARGFSPKDVLVRWLQGSQELPREKYLTWASRQEPS QGTTTFAVTSILRVAAEDWKKGDTFSCMVGHEALPLAFTQKTIDRLAGKPTHVNVSVVMAEVD GTCY | 230 |
| human IgD hinge region | ESPKAQASSVPTAQPQAEGSLAKATTAPATTRNTGRGGEEKKKEKEKEEQEERETKTP | 231 |
| human IgD CH2 | ECPSHTQPLGVYLLTPAVQDLWLRDKATFTCFVVGSDLKDAHLTWEVAGKVPTGGVEEGLLER HSNGSQSQHSRLTLPRSLWNAGTSVTCTLNHPSLPPQRLMALREP | 232 |
| human IgD CH3 region | AAQAPVKLSLNLLASSDPPEAASWLLCEVSGFSPPNILLMWLEDQREVNTSGFAPARPPPQPRS TTFWAWSVLRVPAPPSPQPATYTCVVSHEDSRTLLNASRSLEVSYVTDHGPMK | 233 |
| human IgE CH2 region | VCSRDFTPPTVKILQSSCDGGGHFPPTIQLLCLVSGYTPGTINITWLEDGQVMDVDLSTASTTQE GELASTQSELTLSQKHWLSDRTYTCQVTYQGHTFEDSTKKCA | 234 |
| human IgE CH3 region | DSNPRGVSAYLSRPSPFDLFIRKSPTITCLVVDLAPSKGTVNLTWSRASGKPVNHSTRKEEKQRN GTLTVTSTLPVGTRDWIEGETYQCRVTHPHLPRALMRSTTKTS | 235 |
| human IgE CH4 region | GPRAAPEVYAFATPEWPGSRDKRTLACLIQNFMPEDISVQWLHNEVQLPDARHSTTQPRKTK GSGFFVFSRLEVTRAEWEQKDEFICRAVHEAASPSQTVQRAVSVNPGK | 236 |
| human IgG1 hinge region | EPKSCDKTHTCPPCP | 237 |
| modified human IgG1 hinge region | SDKTHTCPPCP | 238 |
| human IgG1 CH2 region | APELLGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAK | 239 |
| human IgG1 CH3 region | GQPREPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK | 240 |
| human IgG1 heavy chain sequence | MSDKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPPVLDSDGSFFLYSKL TVDKSRWQQGNVFSCSVMHEALHNHYTQKSLSLSPGK | 241 |
| human IgG2 hinge region | ERKCCVECPPCP | 242 |
| human IgG2 CH2 region | APPVAGPSVFLFPPKPKDTLMISRTPEVTCVVVDVSHEDPEVQFNWYVDGVEVHNAKTKPREE QFNSTFRVVSVLTVVHQDWLNGKEYKCKVSNKGLPAPIEKTISKTK | 243 |
| human IgG2 CH3 region | GQPREPQVYTLPPSREEMTKNQVSLTCLVKGFYPSDIAVEWESNGQPENNYKTTPPMLDSDGS FFLYSKLTVDKSRWQQGNVFSCSVMHEALHNHYTQKSLSLSPGK | 244 |
| human IgG3 hinge region | ELKTPLGDTTHTCPRCPEPKSCDTPPPCPRCPEPKSCDTPPPCPRCPEPKSCDTPPPCPRCP | 245 |
| human IgG3 CH2 region | APELLGGPSVFLFPPKPKDTLMISRTPEVTCVVVDVSHEDPEVQFKWYVDGVEVHNAKTKPREE QYNSTFRVVSVLTVLHQDWLNGKEYKCKVSNKALPAPIEKTISKTK | 246 |
| human IgG3 CH3 region | GQPREPQVYTLPPSREEMTKNQVSLTCLVKGFYPSDIAVEWESSGQPENNYNTTPPMLDSDGS FFLYSKLTVDKSRWQQGNIFSCSVMHEALHNRFTQKSLSLSPGK | 247 |
| human IgG4 hinge region | ESKYGPPCPSCP | 248 |
| human IgG4 CH2 region | APEFLGGPSVFLFPPKPKDTLMISRTPEVTCVVVDVSQEDPEVQFNWYVDGVEVHNAKTKPRE EQFNSTYRVVSVLTVLHQDWLNGKEYKCKVSNKGLPSSIEKTISKAK | 249 |
| human IgG4 CH3 region | GQPREPQVYTLPPSQEEMTKNQVSLTCLVKGFYPSDIAVEWESNGQPENNYKTTPPVLDSDGS FFLYSRLTVDKSRWQEGNVFSCSVMHEALHNHYTQKSLSLSLGK | 250 |
| human IgM CH2 region | VIAELPPKVSVFVPPRDGFFGNPRKSKLICQATGFSPRQIQVSWLREGKQVGSGVTTDQVQAEA KESGPTTYKVTSTLTIKESDWLGQSMFTCRVDHRGLTFQQNASSMCVP | 251 |
| human IgM CH3 region | DQDTAIRVFAIPPSFASIFLTKSTKLTCLVTDLTTYDSVTISWTRQNGEAVKTHTNISESHPNATFS AVGEASICEDDWNSGERFTCTVTHTDLPSPLKQTISRPK | 252 |
| human IgM CH4 region | GVALHRPDVYLLPPAREQLNLRESATITCLVTGFSPADVFVQWMQRGQPLSPEKYVTSAPMPE PQAPGRYFAHSILTVSEEEWNTGETYTCVVAHEALPNRVTERTVDKSTGKPTLYNVSLVMSDTA GTCY | 253 |

A Fc region-containing polypeptide can thus comprise, consist of, or consist essentially of one or more of the human Fc region amino acid sequences of **Table A1,** including variants, fragments, homologs, orthologs, paralogs, and combinations thereof. Certain illustrative embodiments comprise an Fc region-containing polypeptide that ranges in size from about 20-50, 20-100, 20-150, 20-200, 20-250, 20-300, 20-400, 50-100, 50-150, 50-200, 50-250, 50-300, 50-400, 100-150, 100-200, 100-250, 100-300, 100-350, 100-400, 200-250, 200-300, 200-350, or 200-400, 200-500, 200-600, 200-700, 200-800, 200-900, or 200-1000, 200-1500, 200-2000, 200-2500, or 200-3000 amino acids in length or more, and optionally comprises, consists of, or consists essentially of any one or more of the sequences in Table A1. Certain embodiments comprise an Fc region of about or up to about 50, 60, 70, 80, 90, 100, 110, 120, 130, 140, 150, 160, 170, 180, 190, 200, 210, 220, 230, 240, 250, 300, 350, 400, 500, 600, 700, 800, 900, 1000, 1500, 2000, 2500, 3000 amino acids in length or more, which optionally comprises, consists of, or consists essentially of any one or more of the sequences in Table A1.

Certain Fc region-containing polypeptides comprise, consist of, or consist essentially of human IgA1 sequences set forth in Table A1, in any order reading from N-terminus to C-terminus, including combinations thereof, and variants and fragments thereof.

Some Fc region-containing polypeptides comprise, consist of, or consist essentially of human IgA2 sequences set forth in Table A1, in any order reading from N-terminus to C-terminus, including combinations thereof, and variants and fragments thereof.

Certain Fc region-containing polypeptides comprise, consist of, or consist essentially of human IgD sequences set forth in Table A1, in any order reading from N-terminus to C-terminus, including combinations thereof, and variants and fragments of these sequences and combinations.

Certain Fc region-containing polypeptides comprise, consist of, or consist essentially of human IgE sequences set forth in Table A1, in any order reading from N-terminus to C-terminus, including combinations thereof, and variants and fragments of these sequences and combinations.

Certain Fc regions comprise, consist of, or consist essentially of human IgG1 sequences set forth in Table A1, in any order reading from N-terminus to C-terminus, including combinations thereof, and variants and fragments of these sequences and combinations.

Certain Fc region-containing polypeptides comprise, consist of, or consist essentially of human IgG2 sequences set forth in Table A1, in any order reading from N-terminus to C-terminus, including combinations thereof, and variants and fragments of these sequences and combinations.

Certain Fc region-containing polypeptides comprise, consist of, or consist essentially of human IgG3 sequences set forth in Table A1, in any order reading from N-terminus to C-terminus, including combinations thereof, and variants and fragments of these sequences and combinations.

Certain Fc region-containing polypeptides comprise, consist of, or consist essentially of human IgG4 sequences set forth in Table A1, in any order reading from N-terminus to C-terminus, including combinations thereof, and variants and fragments of these sequences and combinations.

Certain Fc region-containing polypeptides comprise, consist of, or consist essentially of human IgM sequences set forth in Table A1, in any order reading from N-terminus to C-terminus, including combinations thereof, and variants and fragments of these sequences and combinations.

As noted above, certain embodiments employ variants, fragments, hybrids, and/or otherwise modified forms an Fc region described herein and known in the art. Polypeptide variants and fragments are described herein.

### CNS-Targeted Conjugates

The conjugates described herein comprise (i) "blood-brain barrier (BBB)-transport moiety," that is, a component that increases delivery of the conjugated agent across the BBB relative to the agent alone, and (ii) an agent which is an antibody or antigen-binding fragment thereof, or a therapeutic Fc-fusion polypeptide. In some embodiments, the BBB-moiety and the agent are covalently attached via (iii) at least one linker. The BBB-transport moiety can covalently, non-covalently, or operatively coupled to the agent of interest, such as a therapeutic antibody, therapeutic Fc-fusion polypeptide), to form a conjugate or CNS-targeted conjugate. In some embodiments, the BBB-transport moiety is a polypeptide or peptide sequence. Exemplary BBB-transport peptide moieties or sequences are described below, and include p97 (melanotransferrin) polypeptides, aprotinin-based peptide sequences, and others. See, for example, Gabathuler, Neurobiology of Disease. 37:48-57, 2010; and Gabathuler, Therapeutic Delivery. 1:571-586, 2010.

### 1. BBB-Moieties

Certain conjugates or CNS-targeted conjugates comprise at least one BBB-moiety. Examples of BBB-moieties include p97 (melanotransferrin) polypeptides, Receptor Associated Proteins (RAP), aprotinin peptides or analogs thereof, protein transduction domains (PTD), human low-density lipoprotein receptor (hLDLR) binding peptides or analogs thereof, antibodies or natural ligands that binds to a BBB-associated receptor, and glutathione (GSH).

p97 Polypeptides. In certain embodiments, the BBB-transport moiety is a p97 (melanotransferrin) polypeptide or sequence, for example, a human p97 polypeptide. In some embodiments, a p97 polypeptide sequence used in a composition and/or conjugate of the invention comprises, consists essentially of, or consists of the human p97 melanotransferrin sequence selected from Table B1 or Table B2. Also included are active variants and fragments thereof.

In some embodiments, a p97 polypeptide sequence comprises a sequence having at least 70%, 75%, 80%, 85%, 90%, 95%, 96%, 97%, 98%, or 99% identity or homology, along its length, to the human p97 sequence selected from Table B1 or Table B2, or a fragment thereof.

In particular embodiments, a p97 polypeptide sequence comprises a fragment of a human p97 sequence selected from Table B1 or Table B2. In certain embodiments, a p97 polypeptide fragment is about, at least about, or up to about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700. 700, 710, 720, 730 or more amino acids in length, including all integers and ranges in between, and which may comprise all or a portion of the sequence of a reference p97 sequence selected from Table B1 or Table B2, such as SEQ ID NO:1.

In certain embodiments, a p97 polypeptide fragment is about 5-700, 5-600, 5-500, 5-400, 5-300, 5-200, 5-100, 5-50, 5-40, 5-30, 5-25, 5-20, 5-15, 5-10, 10-700, 10-600, 10-500, 10-400, 10-300, 10-200, 10-100, 10-50, 10-40, 10-30, 10-25, 10-20, 10-15, 20-700, 20-600, 20-500, 20-400, 20-300, 20-200, 20-100, 20-50, 20-40, 20-30, 20-25, 30-700, 30-600, 30-500, 30-400, 30-300, 30-200, 30-100, 30-50, 30-40, 40-700, 40-600, 40-500, 40-400, 40-300, 40-200, 40-100, 40-50, 50-700, 50-600, 50-500, 50-400, 50-300, 50-200, 50-100, 60-700, 60-600, 60-500, 60-400, 60-300, 60-200, 60-100, 60-70, 70-700, 70-600, 70-500, 70-400, 70-300, 70-200, 70-100, 70-80, 80-700, 80-600, 80-500, 80-400, 80-300, 80-200, 80-100, 80-90, 90-700, 90-600, 90-500, 90-400, 90-300, 90-200, 90-100, 100-700, 100-600, 100-500, 100-400, 100-300, 100-250, 100-200, 100-150, 200-700, 200-600, 200-500, 200-400, 200-300, or 200-250 amino acids in length, and comprises all or a portion of a reference p97 sequence selected from Table B1 or Table B2, such as SEQ ID NO:1.

In certain embodiments, p97 polypeptide sequences of interest include p97 amino acid sequences, subsequences, and/or variants of p97 that are effective for transporting an agent of interest across the blood brain barrier and into the central nervous system (CNS). In particular embodiments, the variant or fragment comprises the N-lobe of human p97 (residues 20-361 of SEQ ID NO:1), or an active fragment or variant thereof. In specific aspects, the variant or fragment comprises an intact and functional Fe³⁺-binding site.

In some embodiments, a p97 polypeptide is a soluble form of a p97 polypeptide (see Yang et al., Prot Exp Purif. 34:28-48, 2004), or an active fragment or variant thereof. In some aspects, the soluble p97 polypeptide has a deletion of the all or a portion of the hydrophobic domain (residues 710-738 of SEQ ID NO:1), alone or in combination with a deletion of all or a portion of the signal peptide (residues 1-19 of SEQ ID NO:1). In specific aspects, the soluble p97 polypeptide comprises or consists of residues 20-709, 20-710, 20-711 or 20-712 of SEQ ID NO:1, including variants and fragments thereof.

In specific embodiments, the p97 polypeptide comprises, consists, or consists essentially of DSSHAFTLDELR (SEQ ID NO:14), or an active fragment or variant thereof.

In certain embodiments, for instance, those that employ liposomes, the p97 polypeptide sequence is a lipid soluble form of a p97 polypeptide. For instance, certain of these and related embodiments include a p97 polypeptide that comprises all or a portion of the hydrophobic domain, optionally with or without the signal peptide.

In some embodiments, the p97 polypeptide, for example, DSSHAFTLDELR (SEQ ID NO:14), has one or more terminal (e.g., N-terminal, C-terminal) cysteines and/or tyrosines, which can be added for conjugation and iodination, respectively.

Specific examples of p97 polypeptides and fragments are provided in **Table B1** below.

| **Table B1** | |
|---|---|
| Exemplary p97-Based BBB-transport Peptides | SEQ ID NO: |
| MRGPSGALWLLLALRTVLGGMEVRWCATSDPEQHKCGNMSEAFREAGIQPSLLCVRGTSADHCVQLIAAQE ADAITLDGGAIYEAGKEHGLKPVVGEVYDQEVGTSYYAVAVVRRSSHVTIDTLKGVKSCHTGINRTVGWNVPV GYLVESGRLSVMGCDVLKAVSDYFGGSCVPGAGETSYSESLCRLCRGDSSGEGVCDKSPLERYYDYSGAFRCLA EGAGDVAFVKHSTVLENTDGKTLPSWGQALLSQDFELLCRDGSRADVTEWRQCHLARVPAHAVVVRADTD GGLIFRLLNEGQRLFSHEGSSFQMFSSEAYGQKDLLFKDSTSELVPIATQTYEAWLGHEYLHAMKGLLCDPNRL PPYLRWCVLSTPEIQKCGDMAVAFRRQRLKPEIQCVSAKSPQHCMERIQAEQVDAVTLSGEDIYTAGKTYGLV PAAGEHYAPEDSSNSYYVVAVVRRDSSHAFTLDELRGKRSCHAGFGSPAGWDVPVGALIQRGFIRPKDCDVLT AVSEFFNASCVPVNNPKNYPSSLCALCVGDEQGRNKCVGNSQERYYGYRGAFRCLVENAGDVAFVRHTTVFD NTNGHNSEPWAAELRSEDYELLCPNGARAEVSQFAACNLAQIPPHAVMVRPDTNIFTVYGLLDKAQDLFGD DHNKNGFKMFDSSNYHGQDLLFKDATVRAVPVGEKTTYRGWLGLDYVAALEGMSSQQCSGAAAPAPGAPL LPLLLPALAARLLPPAL (full-length human p97) | 1 |
| GMEVRWCATSDPEQHKCGNMSEAFREAGIQPSLLCVRGTSADHCVQLIAAQEADAITLDGGAIYEAGKEHGL KPVVGEVYDQEVGTSYYAVAVVRRSSHVTIDTLKGVKSCHTGINRTVGWNVPVGYLVESGRLSVMGCDVLKA VSDYFGGSCVPGAGETSYSESLCRLCRGDSSGEGVCDKSPLERYYDYSGAFRCLAEGAGDVAFVKHSTVLENTD GKTLPSWGQALLSQDFELLCRDGSRADVTEWRQCHLARVPAHAVVVRADTDGGLIFRLLNEGQRLFSHEGSS FQMFSSEAYGQKDLLFKDSTSELVPIATQTYEAWLGHEYLHAMKGLLCDPNRLPPYLRWCVLSTPEIQKCGD MAVAFRRQRLKPEIQCVSAKSPQHCMERIQAEQVDAVTLSGEDIYTAGKTYGLVPAAGEHYAPEDSSNSYYV VAVVRRDSSHAFTLDELRGKRSCHAGFGSPAGWDVPVGALIQRGFIRPKDCDVLTAVSEFFNASCVPVNNPK NYPSSLCALCVGDEQGRNKCVGNSQERYYGYRGAFRCLVENAGDVAFVRHTTVFDNTNGHNSEPWAAELRS EDYELLCPNGARAEVSQFAACNLAQIPPHAVMVRPDTNIFTVYGLLDKAQDLFGDDHNKNGFKMFDSSNYH GQDLLFKDATVRAVPVGEKTTYRGWLGLDYVAALEGMSSQQCSG (residues 20-711 of human p97) | 2 |
| WCATSDPEQHK | 3 |
| RSSHVTIDTLK | 4 |
| SSHVTIDTLKGVK | 5 |
| LCRGDSSGEGVCDK | 6 |
| GDSSGEGVCDKSPLER | 7 |
| YYDYSGAFR | 8 |
| ADVTEWR | 9 |
| VPAHAVVVR | 10 |
| ADTDGGLIFR | 11 |
| CGDMAVAFR | 12 |
| LKPEIQCVSAK | 13 |
| DSSHAFTLDELR | 14 |
| SEDYELLCPNGAR | 15 |
| AQDLFGDDHNKNGFK | 16 |
| FSSEAYGQKDLLFKDSTSELVPIATQTYEAWLGHEYLHAM | 17 |
| ERIQAEQVDAVTLSGEDIYTAGKTYGLVPAAGEHYAPEDSSNSYYVVAVVRRDSSHAFTLDELRGKRSCHAGF GSPAGWDVPVGALIQRGFIRPKDCDVLTAVSEFFNASCVPVNNPKNYPSSLCALCVGDEQGRNKCVGNSQER YYGYRGAFRCLVENAGDVAFVRHTTVFDNTNGHNSEPWAAELRSEDYELLCPNGARAEVSQFAACNLAQIPP HAVM | 18 |
| VRPDTNIFTVYGLLDKAQDLFGDDHNKNGFKM | 19 |

In certain embodiments, variants of the DSSHAFTLDELR (SEQ ID NO:14) p97 polypeptide can be based on the sequence of p97 sequences from other organisms, as shown in **Table B2** below. Variant amino acids relative to the human sequence are underlined.

| **Table B2** | | | | | |
|---|---|---|---|---|---|
| **Common Name** | **Species** | **Protein Name** | **% Identity** | **Sequence** | **SEQ ID NO:** |
| Human | Homo sapiens | Melanotransferrin | 100% | DSSHAFTLDELR | 14 |
| Black-capped squirrel monkey | Saimiri boliviensis boliviensis | Melanotransferrin | 100% | DSSHAFTLDELR | 14 |
| Bonobo | Pan paniscus | Melanotransferrin | 100% | DSSHAFTLDELR | 14 |
| Chimpanzee | Pan troglodytes | Melanotransferrin | 100% | DSSHAFTLDELR | 14 |
| Crab-eating macaque | Macaca fascicularis | hypothetical protein | 100% | DSSHAFTLDELR | 14 |
| Northern white-cheeked gibbon | Nomascus leucogenys | Melanotransferrin | 100% | DSSHAFTLDELR | 14 |
| Olive baboon | Papio anubis | Melanotransferrin | 100% | DSSHAFTLDELR | 14 |
| Rhesus macaque | Macaca mulatta | hypothetical protein | 100% | DSSHAFTLDELR | 14 |
| Rhesus macaque | Macaca mulatta | hypothetical protein | 100% | DSSHAFTLDELR | 14 |
| Western lowland gorilla | Gorilla gorilla gorilla | Melanotransferrin | 100% | DSSHAFTLDELR | 14 |
| White-tufted-ear marmoset | Callithrix jacchus | Melanotransferrin | 100% | DSSHAFTLDELR | 14 |
| Lesser Egyptian jerboa | Jaculus jaculus | Melanotransferrin | 92% | DSSDAFTLDELR | 254 |
| Northern greater galago | Otolemur garnettii | Melanotransferrin | 92% | DSSHSFTLDELR | 255 |
| Sumatran orangutan | Pongo abelii | Melanotransferrin | 92% | DSSDAFTLDELR | 254 |
| Thirteen-lined ground squirrel | Ictidomys tridecemlineatus | Melanotransferrin | 92% | DSSYAFTLDELR | 256 |
| white rhinoceros | Ceratotherium simum simum | Melanotransferrin | 92% | NSSHAFTLDELR | 257 |
| alpaca | Vicugna pacos | Melanotransferrin | 83% | NSSYAFTLDELR | 258 |
| American pika | Ochotona princeps | Melanotransferrin | 83% | DSSYAFPLDELR | 259 |
| black flying fox | Pteropus alecto | Melanotransferrin | 83% | NSSYAFTLDELR | 258 |
| bottlenosed dolphin | Tursiops truncatus | Melanotransferrin | 83% | NSSYAFTLDELR | 258 |
| Chinese tree shrew | Tupaia chinensis | Melanotransferrin | 83% | DSTHAFTVDELR | 260 |
| Chiru | Pantholops hodgsonii | Melanotransferrin | 83% | NSSYAFTLDELR | 258 |
| Domestic cat | Felis catus | Melanotransferrin | 83% | NSSYAFTLDELR | 258 |
| Domestic cattle | Bos taurus | Melanotransferrin | 83% | NSSYAFTLDELR | 258 |
| Domestic ferret | Mustela putorius furo | Melanotransferrin | 83% | NSSYAFTLDELR | 258 |
| Giant panda | Ailuropoda Melanoleuca | Melanotransferrin | 83% | NSSYAFTLDELR | 258 |
| Goat | Capra hircus | Melanotransferrin | 83% | NSSYAFTLDELR | 258 |
| House mouse | Mus musculus | Melanotransferrin | 83% | DSSYSFTLDELR | 261 |
| Killer whale | Orcinus orca | Melanotransferrin | 83% | NSSNAFTLDELR | 262 |
| Long-tailed chinchilla | Chinchilla lanigera | Melanotransferrin | 83% | DSSSAFTLNELR | 262 |
| Nine-banded armadillo | Dasypus novemcinctus | Melanotransferrin | 83% | DSSYAFTLDELW | 263 |
| Norway rat | Rattus norvegicus | Melanotransferrin | 83% | DSSYSFTLDELR | 261 |
| Pacific walrus | Odobenus rosmarus divergens | Melanotransferrin | 83% | NSSSAFTLDELR | 264 |
| Prairie vole | Microtus ochrogaster | Melanotransferrin | 83% | DSSYSFTLDELR | 261 |
| Sheep | Ovis aries | Melanotransferrin | 83% | NSSYAFTLDELR | 258 |
| Weddell seal | Leptonychotes weddellii | Melanotransferrin | 83% | NSSYAFTLDELR | 258 |
| Wild Bactrian camel | Camelus ferus | Melanotransferrin | 83% | NSSYAFTLDELR | 258 |
| Wild boar | Sus scrofa | Melanotransferrin | 83% | NSSYAFTLDELR | 258 |
| Yak | Bos mutus | Melanotransferrin | 83% | NSSYAFTLDELR | 258 |
| (Fungus) | Cyphellophora europaea | hypothetical protein | 75% | ATSHAITLDELR | 265 |
| African savanna elephant | Loxodonta africana | Melanotransferrin | 75% | NSSYAFTMDELR | 266 |
| Chinese hamster | Cricetulus griseus | Melanotransferrin | 75% | DRSYSFTLDELR | 267 |
| Common rabbit | Oryctolagus cuniculus | Melanotransferrin | 75% | DSAYAFTVDELR | 268 |
| Degu | Octodon degus | Melanotransferrin | 75% | DSSSAFNLNELR | 269 |
| Domestic Dog | Canis lupus familiaris | Melanotransferrin | 75% | NSSDAFSLDELR | 270 |
| Domestic guinea pig | Cavia porcellus | Melanotransferrin | 75% | DSSSAFSLNELR | 271 |
| European shrew | Sorex araneus | Melanotransferrin | 75% | NSSDAFSLDELR | 270 |
| Florida manatee | Trichechus manatus latirostris | Melanotransferrin | 75% | NSSYAFTMDELR | 266 |
| Golden hamster | Mesocricetus auratus | Melanotransferrin | 75% | DRSYSFTLDELR | 267 |
| Gray short-tailed opossum | Monodelphis domestica | Melanotransferrin | 75% | NSSYSFTLDELR | 272 |
| Horse | Equus caballus | Melanotransferrin | 75% | NSSYAFTVDELR | 273 |
| Small Madagascar hedgehog | Echinops telfairi | Melanotransferrin | 75% | NSSYAFTVDELR | 273 |
| Star-nosed mole | Condylura cristata | Melanotransferrin | 75% | NSSYAFSLDELR | 274 |
| Human | Homo sapien | Transferrin | 33% | SASD_LTWDNLK | 275 |
| Human | Homo sapien | Lactoferrin | 17% | _SDTSLTWNSVK | 276 |

Hence, in certain embodiments, the p97 peptide comprises, consists, or consists essentially of a sequence in **Table B2.** In specific aspects, the p97 peptide retains the short alpha-helix (LDEL) at the C-terminus of the DSSHAFTLDELR (SEQ ID NO:14) peptide.

Additional variants and fragments of reference p97 polypeptides and other reference polypeptides are described in greater detail herein.

Receptor Associated Protein (RAP). In some embodiments, the BBB-transport moiety comprises or consists of a RAP sequence, or an active variant or fragment or analog thereof. The amino acid sequence of mature RAP (w/o signal sequence) is shown in **Table B3** below.

| **Table B3** | |
|---|---|
| Mature RAP Amino Acid Sequence | SEQ ID NO: |
| YSREKNQPKPSPKRESGEEFRMEKLNQLWEKAQRLHLPPVRLAELHADLKIQERDELAWKKLKLDGLDEDGEK EARLIRNLNVILAKYGLDGKKDARQVTSNSLSGTQEDGLDDPRLEKLWHKAKTSGKFSGEELDKLWREFLHHK EKVHEYNVLLETLSRTEEIHENVISPSDLSDIKGSVLHSRHTELKEKLRSINQGLDRLRRVSHQGYSTEAEFEEPRV IDLWDLAQSANLTDKELEAFREELKHFEAKIEKHNHYQKQLEIAHEKLRHAESVGDGERVSRSREKHALLEGRT KELGYTVKKHLQDLSGRISRARHNEL | 20 |

In certain embodiments, a RAP fragment is about, at least about, or up to about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, or 320 or more amino acids in length, including all integers and ranges in between, and which may comprise all or a portion of the RAP sequence set forth in SEQ ID NO:20.

Further examples of RAPs, including RAP variants, are described, for instance, in U.S. Application Nos. 2009/0269346; 2009/0281024; 2010/0028370; 2010/0183581; 2010/0210517; 2011/0142763; and U.S. Patent Nos. 7,560,431; 7,569,544; 7,700,554; 7,829,537; 7,977,317; and 8,440,629, each of which is incorporated by reference in its entirety. Additional variants and fragments of reference RAP sequences and other reference polypeptides are described in greater detail below.

Aprotinin and Related Sequences. In some embodiments, the BBB-transport moiety comprises or consists of an aprotinin polypeptide or peptide, an aprotinin-related polypeptide or peptide, or an active variant or fragment or analog thereof. Particular examples include aprotinin fragments of about 15-58 contiguous amino acids of the aprotinin sequence set forth in SEQ ID NO:21; or fragments of about, at least about, or up to about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 37, 38, 39, 40, 41, 42, 43, 44, 45, 46, 47, 48, 49, 50, 51, 52, 53, 54, 55, 56, 57, 58 contiguous amino acids of SEQ ID NO:21.

The sequences of exemplary aprotinin peptide/analogs are provided in **Table B4** below.

| **Table B4** | |
|---|---|
| Exemplary BBB-transport Peptides | SEQ ID NO: |
| RPDFCLEPPYTGPCKARIIRYFYNAKAGLCQTFVYGGCRAKRNNFKSAEDCMRTCGGA (Aprotinin) | 21 |
| TFVYGGCRAKRNNFKSAED (Aprotinin fragment) | 22 |
| TFFYGGCRGKRNNFKTEEY (Angiopep-1) | 23 |
| TFFYGGSRGKRNNFKTEEY (Angiopep-2) | 24 |
| TFVYGGCRAKRNNFKSAED | 25 |
| TFQYGGCMGNGNNFVTEKE | 26 |
| PFFYGGCGGNRNNFDTEEY | 27 |
| SFYYGGCLGNKNNYLREEE | 28 |
| TFFYGGCRAKRNNFKRAKY | 29 |
| TFFYGGCRGKRNNFKRAKY | 30 |
| TFFYGGCRAKKNNYKRAKY | 31 |
| TFFYGGCRGKKNNFKRAKY | 32 |
| TFQYGGCRAKRNNFKRAKY | 33 |
| TFQYGGCRGKKNNFKRAKY | 34 |
| TFFYGGCLGKRNNFKRAKY | 35 |
| TFFYGGSLGKRNNFKRAKY | 36 |
| PFFYGGCGGKKNNFKRAKY | 37 |
| TFFYGGCRGKGNNYKRAKY | 38 |
| PFFYGGCRGKRNNFLRAKY | 39 |
| TFFYGGCRGKRNNFKREKY | 40 |
| PFFYGGCRAKKNNFKRAKE | 41 |
| TFFYGGCRGKRNNFKRAKD | 42 |
| TFFYGGCRAKRNNFDRAKY | 43 |
| TFFYGGCRGKKNNFKRAEY | 44 |
| PFFYGGCGANRNNFKRAKY | 45 |
| TFFYGGCGGKKNNFKTAKY | 46 |
| TFFYGGCRGNRNNFLRAKY | 47 |
| TFFYGGCRGNRNNFKTAKY | 48 |
| TFFYGGSRGNRNNFKTAKY | 49 |
| TFFYGGCLGNGNNFKRAKY | 50 |
| TFFYGGCLGNRNNFLRAKY | 51 |
| TFFYGGCLGNRNNFKTAKY | 52 |
| TFFYGGCRGNGNNFKSAKY | 53 |
| TFFYGGCRGKKNNFDREKY | 54 |
| TFFYGGCRGKRNNFLREKE | 55 |
| TFFYGGCRGKGNNFDRAKY | 56 |
| TFFYGGSRGKGNNFDRAKY | 57 |
| TFFYGGCRGNGNNFVTAKY | 58 |
| PFFYGGCGGKGNNYVTAKY | 59 |
| TFFYGGCLGKGNNFLTAKY | 60 |
| SFFYGGCLGNKNNFLTAKY | 61 |
| TFFYGGCGGNKNNFVREKY | 62 |
| LGNKNNYVREKY | 63 |
| TFFYGGCGGNGNNFLTAKY | 64 |
| TFFYGGCRGNRNNFLTAEY | 65 |
| TFFYGGCRGNGNNFKSAEY | 66 |
| PFFYGGCLGNKNNFKTAEY | 67 |
| TFFYGGCRGNRNNFKTEEY | 68 |
| TFFYGGCRGKRNNFKTEED | 69 |
| PFFYGGCGGNGNNFVREKY | 70 |
| SFFYGGCMGNGNNFVREKY | 71 |
| PFFYGGCGGNGNNFLREKY | 72 |
| TFFYGGCLGNGNNFVREKY | 73 |
| SFFYGGCLGNGNNYLREKY | 74 |
| TFFYGGSLGNGNNFVREKY | 75 |
| TFFYGGCRGNGNNFVTAEY | 76 |
| TFFYGGCLGKGNNFVSAEY | 77 |
| TFFYGGCLGNRNNFDRAEY | 78 |
| TFFYGGCLGNRNNFLREEY | 79 |
| TFFYGGCLGNKNNYLREEY | 80 |
| PFFYGGCGGNRNNYLREEY | 81 |
| PFFYGGSGGNRNNYLREEY | 82 |
| MRPDFCLEPPYTGPCVARI | 83 |
| ARIIRYFYNAKAGLCQTFVYG | 84 |
| YGGCRAKRNNYKSAEDCMRTCG | 85 |
| PDFCLEPPYTGPCVARIIRYFY | 86 |
| TFFYGGCRGKRNNFKTEEY | 87 |
| KFFYGGCRGKRNNFKTEEY | 88 |
| TFYYGGCRGKRNNYKTEEY | 89 |
| TFFYGGSRGKRNNFKTEEY | 90 |
| CTFFYGCCRGKRNNFKTEEY | 91 |
| TFFYGGCRGKRNNFKTEEYC | 92 |
| TFFYGGKRGKRNNFKTEEY | 93 |
| TFFYGGCRGKRNNFKTKRY | 94 |
| TFFYGGKRGKRNNFKTAEY | 95 |
| TFFYGGKRGKRNNFKRAGY | 96 |
| TFFYGGKRGKRNNFKREKY | 97 |
| TFFYGGKRGKRNNFKTAKY | 98 |
| TFFYGGCLGNRNNFKTEEY | 99 |
| TFFYGCGRGKRNNFKTEEY | 100 |
| TFFYGGRCGKRNNFKTEEY | 101 |
| TFFYGGCLGNGNNFDTEEEQ | 102 |
| TFQYGGCRGKRNNFKTEEY | 103 |
| YNKEFGTFNTKGCERGYRF | 104 |
| RFKYGGCLGNMNNFETLEE | 105 |
| RFKYGGCLGNKNNFLRLKY | 106 |
| RFKYGGCLGNKNNYLRLKY | 107 |
| KTKRKRKKQRVKIAYEEIFKNY | 108 |
| KTKRKRKKQRVKIAY | 109 |
| RGGRLSYSRRFSTSTGR | 110 |
| RRLSYSRRRF | 111 |
| RQIKIWFQNRRMKWKK | 112 |
| TFFYGGSRGKRNNFKTEEY | 113 |
| MRPDFCLEPPYTGPCVARIIRYFYNAKAGLCQTFVYGGCRAKRNNFKSAEDCMRTCGGA | 114 |
| TFFYGGCRGKRNNFKTKEY | 115 |
| TFFYGGCRAKRNNFKRAKY | 116 |
| NAKAGLCQTFVYGGCLAKRNNFESAEDCMRTCGGA | 117 |
| YGGCRAKRNNFKSAEDCMRTCGGA | 118 |
| GLCQTFVYGGCRAKRNNFKSAE | 119 |
| LCQTFVYGGCEAKRNNFKSA | 120 |
| TFFYGGSRGKRNNFKTEEY | 121 |
| RFFYGGSRGKRNNFKTEEY | 122 |
| RFFYGGSRGKRNNFKTEEY | 123 |
| RFFYGGSRGKRNNFRTEEY | 124 |
| TFFYGGSRGKRNNFRTEEY | 125 |
| TFFYGGSRGRRNNFRTEEY | 126 |
| CTFFYGGSRGKRNNFKTEEY | 127 |
| TFFYGGSRGKRNNFKTEEYC | 128 |
| CTFFYGGSRGRRNNFRTEEY | 129 |
| TFFYGGSRGRRNNFRTEEYC | 130 |

Certain embodiments employ aprotinin-peptide coated liposomes or nanoparticles. Additional examples of aprotinin-based BBB targeting peptides are described, for example, in U.S. Application Nos. 2006/0189515; 2008/0299039; 2010/0297120; 2011/0171128; and 2012/0277158; U.S. Patent Nos. 7,902,156 and 7,557,182; and PCT Publication Nos. WO 2006/086870 and WO 2004/060403, each of which is incorporated by reference in its entirety. Additional variants and fragments of reference aprotinin and aprotinin-related polypeptides and other reference polypeptides are described in greater detail below.

Protein Transduction Domains (PTDs). In certain embodiments the BBB-transport moiety comprises or consists of an absorptive mediated protein transduction domain (PTD). PTDs are typically amino acid sequences located on transcription factors which allow transport of larger molecules across cell membranes. Particular examples of PTDs include TAT (e.g., HIV-1 TAT), the homeodomain of Antennapedia, SynB peptides (e.g., SynB1, SynB3, SynB5), penetratin, polyarginine, and others. Residues 37-48 (core domain) of TAT bind to LRP domains II, II, and IV and residues 49-57 promote absorptive endocytosis across cell membranes. The amino acid sequences of TAT (LAI/Bru strain), penetratin, and SynB (PG-1) peptides are shown in **Table B5** below.

| **Table B5** | |
|---|---|
| Exemplary PTD Sequences | SEQ ID NO: |
| MEPVDPRLEPWKHPGSQPKTACTTCYCKKCCFHCQVCFTTKALGISYGRKKRRQRRRPPQGSQTHQVSLSKQ PTSQPRGDPTGPKE (full-length HIV-1 TAT; isolate BRU/LAI) | 131 |
| FTTKALGISYGRKKRRQRRR (residues 37-57 of TAT) | 132 |
| RQIKIWFQNRRMKWKK (Penetratin) | 133 |
| RGGRLCYCRRRFCVCVGR (PG-1) | 134 |
| RGGRLSYSRRRFSTSTGRA (SynB1) | 135 |
| RRLSYSRRRF (SynB3) | 136 |
| RGGRLAYLRRRWAVLGR (SynB5) | 137 |

Additional variants and fragments of reference PTD sequences and other reference polypeptides are described in greater detail below.

hLDLR-Binding Peptides. In certain embodiments, the BBB-transport moiety is a peptide that binds to human low-density lipoprotein receptor (hLDLR). LDLR is a transmembrane protein of 839 amino acids comprising three regions: the extracellular region (1-768), the transmembrane region (768-790) and the cytoplasmic region (790-839). The extracellular region is divided into two subregions: that of LDL binding (1-322) and that outside the LDL binding region (322-768) (see WO2007/014992). Certain hLDLR-binding peptides are about, at least about, or up to about 5, 6, 7, 8, 9, 10, 11, 12, 13, 14, 15, 16, 17, 18, 19, 20, 21, 22, 23, 24, 25, 26, 27, 28, 29, 30 amino acids in length. Such peptides may contain the sequence of one or more exemplary hLDLR-binding peptides shown in **Table B6** below.

| **Table B6** | |
|---|---|
| Exemplary hLDLR-Binding Peptides | SEQ ID NO: |
| HLDCMPRGCFRN | 138 |
| ACQVKSMPRC | 139 |
| ACTTPMPRLC | 140 |
| ACKAPQMPRC | 141 |
| ACLNPSMPRC | 142 |
| ACLVSSMPRC | 143 |
| ACLQPMPRLC | 144 |
| ACPVSSMPRC | 145 |
| ACQSPMPRLC | 146 |
| ACLTPMPRLC | 147 |
| DSGLCM PRLRGCDPR | 148 |
| ACMPRLRGCA | 149 |
| ASGLCMPRLRGCDPR | 150 |
| DAGLCMPRLRGCDPR | 151 |
| DSALCMPRLRGCDPR | 152 |
| DSGACMPRLRGCDPR | 153 |
| DSGLCMPRARGCDPR | 154 |
| DSGLCM PRLAGCDPR | 155 |
| DSGLCMPRLRACDPR | 156 |
| DSGLCM PRLRGCAPR | 157 |
| DSGLCM PRLRGCDAR | 158 |
| DSGLCMPRLRGCDPA | 159 |
| SGLCMPRLRGCDPR | 160 |
| GLCMPRLRGCDPR | 161 |
| CMPRLRGC | 162 |
| CMPRARGC | 163 |
| CMPRLAGC | 164 |
| CMPRLRAC | 165 |
| CMPRLKGC | 166 |
| (D)-CMPRLRGC | 167 |
| CMPR-(D)-LRGC | 168 |
| CMPRL-(D)-RGC | 169 |
| CMPRLRG-(D)-C | 170 |
| (D)-CMPRLRG-(D)- | 171 |
| (D)-CMPRLRSarC | 172 |
| (D)-CMPRKRGC | 173 |
| (D)-CMPRLRC | 174 |
| (D)-CMPRLCR | 175 |
| (D)-CMPRCLRG | 176 |
| CMPRGC | 177 |
| PenMPRLRGC | 178 |
| (D)-PenMPRLRGC | 179 |
| (D)-CMPRLRGPen | 180 |
| PenMPRLRGPen | 181 |
| (D)-PenMPRLRGPen | 182 |
| (D)-PenMPRLRG-(D)-Pen | 183 |
| MpaMPRLRGC | 184 |
| MTVMPTGLWNPLIPS | 185 |
| SASWFAVPIPPLRLE | 186 |
| MTPMSTPRMLPVYVA | 187 |
| MTATHLSTLFQPLTY | 188 |
| MSPIPPAASTWANTL | 189 |
| MTANPLQNAPGPLSL | 190 |
| MQTAPPPPLTRVQWS | 191 |
| GTPRMHIPLNVDHLP | 192 |
| LTLPPISGLSSYPLP | 193 |
| TPSAHAMALQSLSVG | 194 |
| LTLPPISGLSSYPLP | 195 |
| MGTLNAPTAYPQDSL | 196 |
| LTNPPAYLPQNTDPH | 197 |
| MGLPLPYIQTILHTP | 198 |
| SAALIAMSSFKSITA | 199 |
| SGFAFARSVPTESRR | 200 |
| MTSPYMSLPPSTDD | 201 |
| LTNPPAYLPQNTDPH | 202 |
| A1-M-A2-R-L-AR-A3-A4 | 203 |
| wherein A1 and A4 independently represent cysteine (Cys) or an analogue or isostere thereof, A2 represents proline (Pro) or an analogue or isostere thereof, and A3 represents glycine (Gly) or an analogue or isostere thereof | |
| A1-M-A2-R-L-R-G-C (I') wherein A1 represents Cys, (D)-cys, Pen, or (D)-Pen; and A2 represents Pro, Pip or Thz | 204 |
| (D)-cys-Met-Pip-Arg-Leu-Arg-Gly-Cys | 205 |
| (D)-pen-Met-Pip-Arg-Leu-Arg-Gly-Cys | 206 |
| Pen-Met-Pip-Arg-Leu-Arg-Gly-Cys | 207 |
| (D)-cys-Met-Thz-Arg-Leu-Arg-Gly-Cys | 208 |
| (D)-pen-Met-Thz-Arg-Leu-Arg-Gly-Cys | 209 |
| Pen-Met-Thz-Arg-Leu-Arg-Gly-Cys | 210 |
| (D)-cys-Met-Thz-Arg-Leu-Arg-Sar-Cys | 211 |
| (D)-Cys-Met-Thz-Arg-Leu-Arg-Gly- Pen | 212 |
| (D)-Cys-Met-Thz-Arg-Leu-Arg-Sar-Pen | 213 |
| (D)-Cys-M et-Pip-Arg-Le u-Arg-Sa r-Cys | 214 |
| (D)-Cys-M et-Pip-Arg-Leu-Arg-Gly-Pen | 215 |
| (D)-Cys-Met-Pip-Arg-Leu-Arg-Sar-Pen | 216 |
| (D)-Cys-Met-Pro-hArg-Leu-Arg-Gly-Cys | 217 |
| (D)-Cys-M et-Pro-Agb-Leu-Arg-Gly-Cys | 218 |
| (D)-Cys-M et-Pro-Agp-Leu-Arg-Gly-Cys | 219 |
| (D)-Cys-Met-Pro-Cit-Leu-Arg-Gly-Cys | 220 |
| (D)-Cys-Met-Pro-Arg-Leu-Cit-Gly-Cy | 221 |
| (D)-Cys-Met-Pro-Arg-Leu-Arg(NO2)-Gly-Cys | 222 |
| (D)-Cys-Met-Pro-Arg-Leu-Arg-Gly-Cys | 223 |
| Cys-Met-Pro-Arg-Leu-Arg-Gly-Cys | 224 |

The hLDLR-binding peptides can be linear or cyclic peptides, composed of natural and/or non-natural amino acids. Additional examples of hLDLR-binding peptides are described, for example, in U.S. Application Nos. 2011/0230416 and 2013/0108548, each of which is incorporated by reference in its entirety.

BBB-Transport Antibodies/Ligands: In some embodiments, the BBB-transport moiety comprises or consists of an immunoglobulin molecule, such as a monoclonal antibody, or a portion thereof (e.g., antigen binding fragment, ScFv, Fab, sdAb) that binds or specifically binds to a BBB-associated receptor, i.e., a receptor that mediates transport across the BBB. In other embodiments, the BBB-transport moiety is a natural ligand (or a variant of fragment thereof) of a BBB-associated receptor.

Typically, the BBB-associated receptor is a specific transporter protein that facilitates transfer of nutrients , hormones, insulin, and other substances across the BBB, by receptor-mediated transcytosis. Examples of BBB-associated receptors include the insulin receptor, the transferrin receptor, the leptin receptor, lipoprotein receptors such as the lipoprotein receptor-related protein (LRP-1) receptor, insulin-like growth factor (IGF) receptors such as IGF1R and IGF2R, the low-density lipoprotein receptor, the diptheria toxin receptor, and TMEM 30A (Flippase).

Accordingly, certain BBB-transport moieties include an immunoglobulin molecule, such as a monoclonal antibody, or a portion thereof (e.g., antigen binding fragment, ScFv, Fab, sdAb) that binds or specifically binds to the insulin receptor, the transferrin receptor, the leptin receptor, lipoprotein receptors such as the lipoprotein receptor-related protein (LRP-1) receptor, insulin-like growth factor (IGF) receptors such as IGF1R and IGF2R, the low-density lipoprotein receptor, the diptheria toxin receptor, or TMEM 30A (Flippase).

Specific examples of BBB-transport antibodies are described, for example, in U.S. Application Nos. 2008/0152645; 2008/0170994; 2008/0171055; 2009/0053219; 2009/0156498; 2011/0110935; and 2013/0142794; and U.S. Patent Nos. 7,741,446; 8,053,569; 8,124,095; 8,142,781; 8,486,399; and 8,497,246; and PCT Publication Nos. WO 2013/081706; WO 2011/088409; WO 2011/044542; WO 2010/108048; WO 2009/070597; WO 2009/018122; WO 2008/022349; and WO 2007/044323, each of which is incorporated by reference in its entirety.

Examples of ligands of BBB-associated receptor ligands include insulin, transferrin and transferrin fragments, lactoferrin and lactoferrin fragments, apolipoprotein A (Apo A), apolipoprotein B (Apo B), apolipoprotein E (Apo E), melanotransferrin (*supra*)*,* RAP and RAP fragments (*supra*), diptheria toxin (including non-toxic mutants thereof such as CRM45 and CRM197), and others described herein and known in the art. Certain BBB-transport moieties thus comprise or consist of one or more of the foregoing BBB-associated receptor ligands.

Glutathione (GSH). In certain embodiments, the BBB-transport moiety comprises or consists of glutathione (GSH). GSH is a tripeptide with a gamma peptide linkage between the amine group of cysteine (which is attached by normal peptide linkage to a glycine) and the carboxyl group of the glutamate side-chain.

In some embodiments, the GSH moiety is operatively linked (e.g., non-covalently linked) to the antibody or Fc-fusion polypeptide, for instance, as part a carrier system such as a liposome. The carrier may comprise nanoparticles, polymeric nanoparticles, solid liquid nanoparticles, polymeric micelles, liposomes, microemulsions, and/or liquid-based nanoparticles. The liposomes may comprise at least one of lecithin such as soy lecithin and hydrogenated lecithin such as hydrogenated soy lecithin. In some aspects, the liposomes may comprise cholesterol, water-soluble vitamin E, and/or octadecyl amine, for instance, to increase serum resistance or charge amounts. In particular embodiments, the molar composition ratio of the liposome may be 0.5-100% of lecithin or hydrogenated lecithin, 0.005-75% of cholesterol or water-soluble vitamin E, 0.001-25% of octadecyl amine.

Specific examples include a carrier which comprises an antibody or Fc-fusion polypeptide (as described herein) and glutathione, where the glutathione is covalently bound to polyethylene glycol, optionally where the polyethylene glycol is covalently bound to vitamin E or a phospholipid, and the vitamin E or phospholipid is intercalated into the carrier such that the glutathione is on an outside surface of the carrier (e.g., GSH-PEG-coated liposomes). Exemplary GSH-based conjugates and carrier systems are described, for example, in U.S. Application Nos. 2007/0141133; 2008/0095836; and 2009/0123531; and U.S. Patent Nos. 7,446,096; 7,700,564; 7,704,956; and 8,067,380, each of which is incorporated by reference in its entirety.

### 2A. Antibodies

In certain embodiments, the conjugate comprises an antibody or antigen-binding fragment thereof as the agent. The antibody used in the conjugates or compositions of the present invention can be of essentially any type. Particular examples include therapeutic and diagnostic antibodies. As is well known in the art, an antibody is an immunoglobulin molecule capable of specific binding to a target, such as a carbohydrate, polynucleotide, lipid, polypeptide, *etc.,* through at least one epitope recognition site, located in the variable region of the immunoglobulin molecule. In particular embodiments, the antibody or antigen-binding fragment of (b) comprises an immunoglobulin Fc region, and optionally relies on that Fc region for at least part of its therapeutic activity.

As used herein, the term "antibody" encompasses not only intact polyclonal or monoclonal antibodies, but also fragments thereof (such as dAb, Fab, Fab', F(ab')₂, Fv), single chain (ScFv), synthetic variants thereof, naturally occurring variants, fusion proteins comprising an antibody portion with an antigen-binding fragment of the required specificity, humanized antibodies, chimeric antibodies, and any other modified configuration of the immunoglobulin molecule that comprises an antigen-binding site or fragment (epitope recognition site) of the required specificity.

The term "antigen-binding fragment" as used herein refers to a polypeptide fragment that contains at least one CDR of an immunoglobulin heavy and/or light chains that binds to the antigen of interest. In this regard, an antigen-binding fragment of the herein described antibodies may comprise 1, 2, 3, 4, 5, or all 6 CDRs of a VH and VL sequence from antibodies that bind to a therapeutic or diagnostic target.

The term "antigen" refers to a molecule or a portion of a molecule capable of being bound by a selective binding agent, such as an antibody, and additionally capable of being used in an animal to produce antibodies capable of binding to an epitope of that antigen. An antigen may have one or more epitopes.

The term "epitope" includes any determinant, preferably a polypeptide determinant, capable of specific binding to an immunoglobulin or T-cell receptor. An epitope is a region of an antigen that is bound by an antibody. In certain embodiments, epitope determinants include chemically active surface groupings of molecules such as amino acids, sugar side chains, phosphoryl or sulfonyl, and may in certain embodiments have specific three-dimensional structural characteristics, and/or specific charge characteristics. Epitopes can be contiguous or non-contiguous in relation to the primary structure of the antigen.

A molecule such as an antibody is said to exhibit "specific binding" or "preferential binding" if it reacts or associates more frequently, more rapidly, with greater duration and/or with greater affinity with a particular cell or substance than it does with alternative cells or substances. An antibody "specifically binds" or "preferentially binds" to a target if it binds with greater affinity, avidity, more readily, and/or with greater duration than it binds to other substances. For example, an antibody that specifically or preferentially binds to a specific epitope is an antibody that binds that specific epitope with greater affinity, avidity, more readily, and/or with greater duration than it binds to other epitopes. It is also understood by reading this definition that, for example, an antibody (or moiety or epitope) that specifically or preferentially binds to a first target may or may not specifically or preferentially bind to a second target. As such, "specific binding" or "preferential binding" does not necessarily require (although it can include) exclusive binding. Generally, but not necessarily, reference to binding means preferential binding.

Immunological binding generally refers to the non-covalent interactions of the type which occur between an immunoglobulin molecule and an antigen for which the immunoglobulin is specific, for example by way of illustration and not limitation, as a result of electrostatic, ionic, hydrophilic and/or hydrophobic attractions or repulsion, steric forces, hydrogen bonding, van der Waals forces, and other interactions. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation. The ratio of K_{off} /Kₒₙ enables cancellation of all parameters not related to affinity, and is thus equal to the dissociation constant K_{d}.

Immunological binding properties of selected antibodies and polypeptides can be quantified using methods well known in the art (see Davies et al., Annual Rev. Biochem. 59:439-473, 1990). In some embodiments, an antibody or other polypeptide is said to specifically bind an antigen or epitope thereof when the equilibrium dissociation constant is about ≤10⁻⁷ or 10⁻⁸ M. In some embodiments, the equilibrium dissociation constant of an antibody may be about ≤10⁻⁹ M or ≤10⁻¹⁰ M. In certain illustrative embodiments, an antibody or other polypeptide has an affinity (K_{d}) for an antigen or target described herein (to which it specifically binds) of at least about 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, or 50 nM.

In some embodiments, antibody or antigen-binding fragment specifically binds to a cell surface receptor. In certain embodiments, the antibody or antigen-binding fragment specifically binds to a ligand of a cell surface receptor.

In some embodiments, the antibody or antigen-binding fragment thereof specifically binds to a cancer-associated antigen. In some instances, the antigen is associated with a cancer of the central nervous system (CNS), i.e., a neurological cancer. In some instances, the antigen is associated with a metastatic cancer of the CNS, i.e., a metastatic brain cancer. Exemplary cancer antigens include cell surface proteins such as cell surface receptors. Also included as cancer-associated antigens are ligands that bind to such cell surface proteins or receptors. In specific embodiments, the antibody or antigen-binding fragment specifically binds to a intracellular cancer antigen. In some embodiments, the cancer that associates with the cancer antigen is one or more of breast cancer, metastatic brain cancer, prostate cancer, gastrointestinal cancer, lung cancer, ovarian cancer, testicular cancer, head and neck cancer, stomach cancer, bladder cancer, pancreatic cancer, liver cancer, kidney cancer, squamous cell carcinoma, CNS or brain cancer, melanoma, non-melanoma cancer, thyroid cancer, endometrial cancer, epithelial tumor, bone cancer, or a hematopoietic cancer.

Exemplary cancer-associated antigens include, without limitation, Her2/neu, B7H3, CD20, Her1/EGF receptor(s), VEGF receptor(s), PDGF receptor(s), CD30, CD52, CD33, CTLA-4, and tenascin. An additional example of a cancer-associated antigen is the B7H3 antigen (*see* Chen et al., Curr. Cancer Drug Targets. 8:404-413, 2008). In specific embodiments, the antibody is the 8H9 monoclonal antibody (*see, e.g.,* Modak et al., Cancer Res. 61:4048-54, 2001), or an antigen-binding fragment thereof. In particular embodiments, the cancer-associated antigen, or cancer antigen, includes one or more of human Her2/neu, Her1/EGF receptor (EGFR), Her3, A33 antigen, CD5, CD19, CD20, CD22, CD23 (IgE Receptor), C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), integrin αᵥβ₃, integrin α₅β₁, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and/or mesothelin.

In specific embodiments, the antibody or antigen-binding fragment thereof or other polypeptide specifically binds to the human Her2/neu protein. Essentially any anti-Her2/neu antibody, antigen-binding fragment or other Her2/neu-specific binding agent may be used in producing the conjugates of the present invention. Illustrative anti-Her2/neu antibodies are described, for example, in US Patent Nos. 5,677,171; 5,720,937; 5,720,954; 5,725,856; 5,770,195; 5,772,997; 6,165,464; 6,387,371; and 6,399,063, the contents of which are incorporated herein by reference in their entireties.

In specific embodiments, the anti-Her2/neu antibody used in a conjugate is trastuzumab (Herceptin^{®}), or a fragment, variant or derivative thereof, as described herein. Herceptin^{®} is a Her2/neu-specific monoclonal antibody approved for the treatment of human breast cancer. In certain embodiments, a Her2/neu-binding antigen-binding fragment comprises one or more of the CDRs of a Her2/neu antibody. In this regard, it has been shown in some cases that the transfer of only the VHCDR3 of an antibody can be performed while still retaining desired specific binding (Barbas et al., PNAS. 92: 2529-2533, 1995). See also, McLane et al., PNAS USA. 92:5214-5218, 1995; and Barbas et al., J. Am. Chem. Soc. 116:2161-2162, 1994.

In some embodiments, the antibody or antigen-binding fragment thereof or other polypeptide specifically binds to the human Her1/EGFR (epidermal growth factor receptor). Essentially any anti-Her1/EGFR antibody, antigen-binding fragment or other Her1-EGFR-specific binding agent may be used in producing the conjugates of the present invention. Illustrative anti-Herl/EGFR antibodies are described, for example, in U.S. Patent Nos. 5,844,093; 7,132,511; 7,247,301; 7,595,378; 7,723,484; 7,939,072; and 7,960,516, the contents of which are incorporated by reference in their entireties.

In specific embodiments, the anti-Herl/EGFR antibody used in a conjugate of the invention is cetuximab (Erbitux^{®}), or a fragment or derivative thereof, as described herein. In certain embodiments, an anti-Herl/EGFR binding fragment comprises one or more of the CDRs of a Her1/EGFR antibody such as cetuximab. Cetuximab is approved for the treatment of head and neck cancer, and colorectal cancer. Cetuximab is composed of the Fv (variable; antigen-binding) regions of the 225 murine EGFR monoclonal antibody specific for the N-terminal portion of human EGFR with human IgG1 heavy and kappa light chain constant (framework) regions.

In certain embodiments, the antibody is a therapeutic antibody selected from trastuzumab, cetuximab, daclizumab, tanezumab, 3F8, 8H9, abagovomab, adecatumumab, afutuzumab, alemtuzumab, alacizumab (pegol), amatuximab, apolizumab, bavituximab, bectumomab, belimumab, bevacizumab, bivatuzumab (mertansine), brentuximab vedotin, cantuzumab (mertansine), cantuzumab (ravtansine), capromab (pendetide), catumaxomab, citatuzumab (bogatox), cixutumumab, clivatuzumab (tetraxetan), conatumumab, dacetuzumab, dalotuzumab, detumomab, drozitumab, ecromeximab, edrecolomab, elotuzumab, enavatuzumab, ensituximab, epratuzumab, ertumaxomab, etaracizumab, farletuzumab, FBTA05, figitumumab, flanvotumab, galiximab, gemtuzumab, ganitumab, gemtuzumab (ozogamicin), girentuximab, glembatumumab (vedotin), ibritumomab tiuxetan, icrucumab, igovomab, indatuximab ravtansine, intetumumab, inotuzumab ozogamicin, ipilimumab (MDX-101), iratumumab, labetuzumab, lexatumumab, lintuzumab, lorvotuzumab (mertansine), lucatumumab, lumiliximab, mapatumumab, matuzumab, milatuzumab, mitumomab, mogamulizumab, moxetumomab (pasudotox), nacolomab (tafenatox), naptumomab (estafenatox), narnatumab, necitumumab, nimotuzumab, nivolumab, Neuradiab^{®} (with or without radioactive iodine), NR-LU-10, ofatumumab, olaratumab, onartuzumab, oportuzumab (monatox), oregovomab, panitumumab, patritumab, pemtumomab, pertuzumab, pritumumab, racotumomab, radretumab, ramucirumab, rilotumumab, rituximab, robatumumab, samalizumab, sibrotuzumab, siltuximab, tabalumab, taplitumomab (paptox), tenatumomab, teprotumumab, TGN1412, ticilimumab, tremelimumab, tigatuzumab, TNX-650, tositumomab, TRBS07, tucotuzumab (celmoleukin), ublituximab, urelumab, veltuzumab, volociximab, votumumab, and zalutumumab. Also included are fragments, variants, and derivatives of the foregoing.

In certain embodiments, the antibody specifically binds to a host protein or antigen that is associated with a neuropathology (e.g., neuroinflammatory condition), including any one or more neuroinflammatory and/or auto-immune-associated antigens. In particular embodiments, the antibody specifically binds to a host protein or antigen that is associated with a neuroinflammatory condition. In some aspects, the host protein or antigen is a cell surface receptor or other cell surface protein. In some embodiments, the antigen is a ligand of a cell surface receptor or other cell surface protein. In certain embodiments, the antigen is an intracellular protein. In particular embodiments, the host protein or antigen is a human protein.

In some embodiments, the antibody specifically binds to an antigen associated with (e.g., treatment of) at least one nervous system disorder, including disorders of the peripheral and/or central nervous system (CNS) disorder. In certain embodiments, the antibody or other polypeptide specifically binds to an antigen associated with (e.g., treatment of) pain, including acute pain, chronic pain, and neuropathic pain. In some embodiments, the antibody specifically binds an antigen associated with (e.g., treatment of) an autoimmune disorder, including autoimmune disorders of the nervous system or CNS.

Examples of nervous system-, pain-, and/or autoimmune-associated antigens include, without limitation, alpha-4 (α4) integrin, tumor necrosis factor (TNF), IL-12, IL-23, the p40 subunit of IL-12 and IL-23, CD20, CD52, amyloid-β (e.g., Aβ₍₁₋₄₂₎), Huntingtin, CD25 (i.e., the alpha chain of the IL-2 receptor), nerve growth factor (NGF), neurotrophic tyrosine kinase receptor type 1 (TrkA; the high affinity catalytic receptor for NGF), and α-synuclein. In some embodiments, the antibody specifically binds at least one of the interleukin-2 (IL-2) receptor, α4 integrin, CD20, CD52, IL-12, IL-23, the p40 subunit of IL-12 and IL-23, or the axonal regrowth and remyelination inhibitors Nogo-A and LINGO. These targets have been considered useful in the treatment of a variety of nervous system, pain, and/or autoimmune disorders, such as multiple sclerosis (α4 integrin, IL-23, CD25, CD20, CD52, IL-12, IL-23, the p40 subunit of IL-12 and IL-23, Nogo-A, LINGO-1), Alzheimer's Disease (Aβ, TNF), Huntington's Disease (Huntingtin), Parkinson's Disease (a-synuclein), and pain (NGF and TrkA).

*IL-2 Receptor.* In some aspects, the antibody specifically binds to the IL-2 receptor (IL-2R). The IL-2R is a heterotrimeric protein expressed on the surface of certain immune cells, such as lymphocytes, which binds and responds to the cytokine IL-2. The IL-2R has 3 non-covalently-associated subunits: α (CD25), β (CD122) and γ (CD132). The α and β subunits bind to IL-2, and the β and γ-subunits together facilitate signal transduction after said binding. The β and γ subunits of IL-2R are members of the type I cytokine receptor family.

Hence, in certain aspects, the antibody specifically binds to the alpha-subunit of the IL-2 receptor (CD25), and optionally reduces the interaction between IL2 and CD25. In some aspects, the antibody specifically binds to the beta-subunit of the IL-2 receptor (CD122), and optionally reduces the interaction between IL-2 and CD122, and/or reduces the signal transduction resulting from binding of IL-2 to IL-2R. In particular aspects, the antibody specifically binds to the gamma-subunit of the IL-2 receptor (CD132), and optionally reduces the signal transduction resulting from binding of IL-2 to IL-2R. In specific aspects, the antibody is daclizumab, or a variant or fragment thereof that specifically binds to CD25, as described herein.

*Alpha-4 integrin.* In certain aspects, the antibody specifically binds to α4 integrin. Alpha-4 integrin (or CD49d) is one component of the integrin dimer Very Late Antigen-4 (VLA-4, or Integrin alpha4beta1), the other component being beta-1 integrin (CD29, or integrin beta-1). Unlike other integrin alpha chains, α4 integrin neither contains an I-domain, nor undergoes disulfide-linked cleavage. α4 integrin also associates with beta 7 chain. In some aspects, the antibody is natalizumab, or a variant or fragment thereof that specifically binds to α4 integrin, as described herein.

*CD20.* In some aspects, the antibody specifically binds to CD20. CD20 is an activated-glycosylated phosphoprotein expressed on the surface of all B-cells beginning at the pro-B phase (CD45R+, CD117+) and progressively increasing in concentration until maturity. Hence, in some embodiments, the antibody specifically binds to a CD20+ B-cell in a subject or *in vitro.* In specific embodiments, the antibody specifically binds to CD20, and has one or more of the following effects on a CD20+ B-cell: (a) mediates antibody-dependent cellular cytotoxicity (ADCC) and/or complement-dependent cytotoxicity (CDC) against the B-cell, (b) increases expression of MHC II, LFA-1, and/or LFA-3 (lymphocyte function-associated antigen), (c) increases shedding of CD23, (d) downregulates the B cell receptor, (e) induces apoptosis of the B-cell, or (e) any combination of the foregoing. In specific embodiments, the antibody is rituximab, tositumomab, ocrelizumab, or ofatumumab, TRU-015, or veltuzumab, or a variant or fragment thereof that specifically binds to CD20, as described herein.

*CD52.* In some aspects, the antibody specifically binds to CD52. CD52 is a glycosylphosphatidylinositol (GPI)-anchored antigen expressed on mature lymphocytes, monocytes, and certain dendritic cell subsets. In specific embodiments, the antibody is alemtuzumab, or a variant or fragment thereof that specifically binds to CD52, as described herein.

*IL-12.* In some embodiments, the antibody specifically binds to interleukin-12 (IL-12). IL-12 is an interleukin produced by dendritic cells, macrophages and human B-lymphoblastoid cells (NC-37) in response to antigenic stimulation. It is a heterodimeric cytokine encoded by two separate genes, IL-12A (p35) and IL-12B (p40).

IL-12 is involved in the differentiation of naive T cells into Th0 cells, which will further develop into either Th1 cells or Th2 cells. It is known as a T cell-stimulating factor, which can stimulate the growth and function of T cells. It stimulates the production of interferon-gamma (IFN-y) and tumor necrosis factor-alpha (TNF-α) from T and natural killer (NK) cells, and reduces IL-4 mediated suppression of IFN-y. IL-12 also mediates enhancement of the cytotoxic activity of NK cells and CD8+ cytotoxic T lymphocytes. IL-12 is associated with autoimmunity, which is believed to result from its role in the induction of Th1 immune responses. Hence, certain embodiments include antibodies that specifically bind to IL-12A (p35), IL-12B (p40), or both, and which optionally reduce or antagonize one or of the above-described biological activities of IL-12, including its role in the induction of Th1 immune responses, as described herein.

*IL-23.* In some embodiments, the antibody specifically binds to interleukin-23 (IL-23). IL-23 is a heterodimeric cytokine composed of two subunits, p40 and p19 (the IL-23 alpha subunit). In conjunction with IL-6 and TGF-β1, IL-23 stimulates naive CD4+ T cells to differentiate into Th17 cells, which are distinct from the classical Th1 and Th2 cells. Th17 cells produce IL-17, a pro-inflammatory cytokine that enhances T cell priming and stimulates the production of pro-inflammatory molecules such as IL-1, IL-6, TNF-alpha, NOS-2, and chemokines resulting in inflammation. Knockout mice deficient in either p40 or p19, or in either subunit of the IL-23 receptor (IL-23R and IL12R-β1) develop less severe symptoms of multiple sclerosis and inflammatory bowel disease. Certain embodiments thus include antibodies that specifically bind to p40, p19, or both, and which optionally reduce or antagonize one or of the above-described biological activities of IL-23, including its role in the production of Th17 cells, as described herein

*p40 subunit of IL-12 and IL-23.* In some embodiments, the antibody specifically binds to the p40 subunit of IL-12 (*i.e.,* IL-12 beta subunit) and IL-23. p40 (i.e., natural killer cell stimulatory factor 2, cytotoxic lymphocyte maturation factor 2) is a shared subunit of both IL-12 and IL-23. Overexpression of this gene has been observed in the central nervous system of patients with multiple sclerosis (MS), suggesting a role of this cytokine in the pathogenesis of the disease. Certain embodiments therefore relate to antibodies that specifically bind to p40, and which optionally reduce or antagonize one or more of the above-described activities of IL-12 and IL-23. In specific embodiments, the anti-p40 antibody is ustekinumab (CNTO 1275), or a variant or fragment thereof that specifically binds to the p40 subunit of IL-12 and IL-23, as described herein.

*Nogo-A.* Nogo-A is a splice isoform of Reticulon-4 (or Neurite outgrowth inhibitor; Nogo) and an axonal regrowth and remyelination inhibitor. Certain embodiments include antibodies that specifically bind to Nogo-A, and which optionally reduce or antagonize its activity as an axonal regrowth and remyelination inhibitor, that is, they increase axonal regrowth and remyelination in a subject, as described herein.

*LINGO-1.* LINGO-1 is a CNS-specific protein and a functional component of the NgR1/p75/LINGO-1 and NgR1/TAJ(TROY)/LINGO-1 signaling complexes that mediate inhibition of axonal outgrowth. These receptor complexes mediate the axonal growth inhibitory effects of Nogo, myelin-associated glycoprotein (MAG), and oligodendrocyte-myelin glycoprotein (OMgp) via RhoA activation. Certain embodiments include antibodies or antigen-binding fragments thereof that specifically bind to LINGO-1, and which optionally reduce or antagonize its activity as an axonal regrowth and remyelination inhibitor, that is, they increase axonal regrowth and remyelination in a subject.

In certain embodiments, the antibody specifically binds to a host protein or antigen that is associated with one or more types of pain, or a pain-associated antigen. In some aspects, the host protein or antigen is a cell surface receptor or other cell surface protein. In some embodiments, the antigen is a ligand of a cell surface receptor or other cell surface protein. In certain embodiments, the antigen is an intracellular protein. In particular embodiments, the host protein or antigen is a human protein. Exemplary pain-associated antigens include nerve growth factor (NGF) and neurotrophic tyrosine kinase receptor type 1 (TrkA).

*Nerve Growth Factor.* NGF is a secreted protein contributes to the growth, maintenance, and survival of certain neurons, and is critical to the survival of sympathetic and sensory neurons. NGF forms a cysteine knot structure made up of beta strands twisted around each other and linked by disulfide bonds. Most structures are dimeric. NGF interacts with at least two classes of receptors: the p75 LNGFR (low affinity nerve growth factor receptor) neurotrophin receptor (p75(NTR)) and TrkA, a transmembrane tyrosine kinase. Both receptors are associated with neurodegenerative disorders.

NGF has also been shown to be a major mediator of pain, including inflammatory and neuropathic pain. For instance, preclinical animal models of inflammatory and neuropathic pain showed increased NGF levels, while the sequestration of NGF alleviated the associated hyperalgesia (see Watson et al., BioDrugs. 22:349-59, 2008). Certain embodiments thus include antibodies that specifically bind to NGF, and which optionally reduce its binding to TrkA, p75(NTR), or both, as described herein. The identification of NGF epitopes that interact with TrkA can be facilitated by referring to the crystal structure of NGF in complex with the ligand-binding domain of TrkA (see Wiesmann et al., Nature. 401:184-188, 1999). In some embodiments, the antibody is tanezumab, or a variant or fragment thereof that specifically binds to NGF, and optionally reduces the binding between NGF and TrkA, as described herein.

*TrkA.* High affinity nerve growth factor receptor (neurotrophic tyrosine kinase receptor type 1; TRK1-transforming tyrosine kinase protein; TrkA) is a membrane-bound receptor kinase that upon binding to NGF, phosphorylates itself and members of the MAPK pathway. TrkA activation by NGF has been associated with pain, including inflammatory and neuropathic pain. Indeed, neutralizing antibodies directed against the TrkA receptor may display potent analgesic effects in inflammatory and chronic pain (*see* Ugolini et al., PNAS USA. 104:2985-2990, 2007).

TrkA is composed of an extracellular NGF-binding domain and an intracellular tyrosine kinase domain. Certain embodiments thus include antibodies that specifically bind to the extracellular NGF-binding domain of TrkA, and which optionally reduce the interaction between TrkA and NGF, as described herein. The identification of TrkA epitopes that interact with NGF can be facilitated by referring to the crystal structure of NGF in complex with the ligand-binding domain of TrkA (*see* Wiesmann et *al., supra*) Other embodiments include antibodies or antigen-binding fragments thereof that specifically bind to the intracellular tyrosine kinase domain, and optionally inhibit the kinase activity of TrkA, for example, its autophosphorylation and/or its phosphorylation of members of the MAPK pathway. In specific embodiments, the antibody is MNAC13, or a variant or fragment thereof that specifically binds to TrkA, as described herein.

In some embodiments, the antibody specifically binds to a pro-inflammatory molecule, for example, a pro-inflammatory cytokine or chemokine. In these and related embodiments, the conjugate can be used to treat a variety of inflammatory conditions, as described herein. Examples of pro-inflammatory molecules include tumor necrosis factors (TNF) such as TNF-α and TNF-β, TNF superfamily molecules such as FasL, CD27L, CD30L, CD40L, Ox40L, 4-1BBL, TRAIL, TWEAK, and Apo3L, interleukin-1 (IL-1) including IL-1α and IL-1β, IL-2, interferon-y (IFN-y), IFN-α/β, IL-6, IL-8, IL-12, IL-15, IL-17, IL-18, IL-21, LIF, CCL5, GROα, MCP-1, MIP-1α, MIP-1β, macrophage colony stimulating factor (MCSF), granulocyte macrophage colony stimulating factor (GM-CSF), CXCL2, CCL2, among others. In some embodiments, the antibody specifically binds to a receptor of one or more of the foregoing pro-inflammatory molecules, such as a TNF receptor (TNFR), an IL-1 receptor (IL-1R), or an IL-6 receptor (IL-6R), among others.

In specific embodiments, as noted above, the antibody specifically binds to TNF-α or TNF-β. In particular embodiments, the anti-TNF antibody is adalimumab (Humira^{®}), certolizumab pegol (Cimzia^{®}), golimumab (Cimzia^{®}), or infliximab (Remicade^{®}), D2E7, CDP 571, or CDP 870, as described herein. Conjugates comprising an anti-TNF antibody can be used, for instance, in the treatment of various inflammatory conditions, as described herein. Such conjugates can also be used in the treatment of various neurological conditions or disorders such as Alzheimer's disease, stroke, traumatic brain injury (TBI), spinal stenosis, acute spinal cord injury, and spinal cord compression (see U.S. Patent Nos. 6,015,557; 6,177,077; 6,419,934; 6,419,944; 6,537,549; 6,982,089; and 7,214,658).

In specific embodiments, the antibody specifically binds to IL-1α or IL-1β. In particular embodiments, the anti-IL-1 antibody is canakinumab or gevokizumab, or a variant or fragment thereof that specifically binds to IL-1β, as described herein. Among other inflammatory conditions described herein, conjugates comprising an anti-IL-1 antibody can be used to treat cryopyrin-associated periodic syndromes (CAPS), including familial cold autoinflammatory syndrome, Muckle-Wells syndrome, and neonatal-onset multisystem inflammatory disease.

Antibodies may be prepared by any of a variety of techniques known to those of ordinary skill in the art. *See, e.g.,* Harlow and Lane, Antibodies: A Laboratory Manual, Cold Spring Harbor Laboratory, 1988. Monoclonal antibodies specific for a polypeptide of interest may be prepared, for example, using the technique of Kohler and Milstein, Eur. J. Immunol. 6:511-519, 1976, and improvements thereto. Also included are methods that utilize transgenic animals such as mice to express human antibodies. See, e.g., Neuberger et al., Nature Biotechnology 14:826, 1996; Lonberg et al., Handbook of Experimental Pharmacology 113:49-101, 1994; and Lonberg et al., Internal Review of Immunology 13:65-93, 1995. Particular examples include the VELOCIMMUNE^{®} platform by REGENEREX^{®} *(see, e.g.,* U.S. Patent No. 6,596,541).

Antibodies can also be generated or identified by the use of phage display or yeast display libraries *(see, e.g.,* U.S. Patent No. 7,244,592; Chao et al., Nature Protocols. 1:755-768, 2006). Non-limiting examples of available libraries include cloned or synthetic libraries, such as the Human Combinatorial Antibody Library (HuCAL), in which the structural diversity of the human antibody repertoire is represented by seven heavy chain and seven light chain variable region genes. The combination of these genes gives rise to 49 frameworks in the master library. By superimposing highly variable genetic cassettes (CDRs = complementarity determining regions) on these frameworks, the vast human antibody repertoire can be reproduced. Also included are human libraries designed with human-donor-sourced fragments encoding a light-chain variable region, a heavy-chain CDR-3, synthetic DNA encoding diversity in heavy-chain CDR-1, and synthetic DNA encoding diversity in heavy-chain CDR-2. Other libraries suitable for use will be apparent to persons skilled in the art.

In certain embodiments, antibodies and antigen-binding fragments thereof as described herein include a heavy chain and a light chain CDR set, respectively interposed between a heavy chain and a light chain framework region (FR) set which provide support to the CDRs and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (*e.g.,* a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRs. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures-regardless of the precise CDR amino acid sequence. Further, certain FR residues are known to participate in non-covalent interdomain contacts which stabilize the interaction of the antibody heavy and light chains.

The structures and locations of immunoglobulin variable domains may be determined by reference to Kabat, E. A. et al., Sequences of Proteins of Immunological Interest. 4th Edition. US Department of Health and Human Services. 1987, and updates thereof.

In certain embodiments, the antibody is a monoclonal antibody. A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific, being directed against a single epitope. The term "monoclonal antibody" encompasses not only intact monoclonal antibodies and full-length monoclonal antibodies, but also fragments thereof (such as Fab, Fab', F(ab')₂, Fv), single chain (ScFv), variants thereof, fusion proteins comprising an antigen-binding portion, humanized monoclonal antibodies, chimeric monoclonal antibodies, and any other modified configuration of the immunoglobulin molecule that comprises an antigen-binding fragment (epitope recognition site) of the required specificity and the ability to bind to an epitope. It is not intended to be limited as regards the source of the antibody or the manner in which it is made (*e.g*., by hybridoma, phage selection, recombinant expression, transgenic animals). The term includes whole immunoglobulins as well as the fragments *etc.* described above under the definition of "antibody."

In certain embodiments, the antibodies or antigen-binding fragments thereof are humanized. These embodiments refer to a chimeric molecule, generally prepared using recombinant techniques, having an antigen-binding site derived from an immunoglobulin from a non-human species and the remaining immunoglobulin structure of the molecule based upon the structure and/or sequence of a human immunoglobulin. The antigen-binding site may comprise either complete variable domains fused onto constant domains or only the CDRs grafted onto appropriate framework regions in the variable domains. Epitope binding sites may be wild type or modified by one or more amino acid substitutions. This eliminates the constant region as an immunogen in human individuals, but the possibility of an immune response to the foreign variable region remains (LoBuglio et al., PNAS USA 86:4220-4224, 1989; Queen et al., PNAS USA. 86:10029-10033, 1988; Riechmann et al., Nature. 332:323-327, 1988). Illustrative methods for humanization of antibodies include the methods described in U.S. Patent No. 7,462,697.

Another approach focuses not only on providing human-derived constant regions, but modifying the variable regions as well so as to reshape them as closely as possible to human form. It is known that the variable regions of both heavy and light chains contain three complementarity-determining regions (CDRs) which vary in response to the epitopes in question and determine binding capability, flanked by four framework regions (FRs) which are relatively conserved in a given species and which putatively provide a scaffolding for the CDRs. When nonhuman antibodies are prepared with respect to a particular epitope, the variable regions can be "reshaped" or "humanized" by grafting CDRs derived from nonhuman antibody on the FRs present in the human antibody to be modified. Application of this approach to various antibodies has been reported by Sato et al., Cancer Res. 53:851-856, 1993; Riechmann et al., Nature 332:323-327, 1988; Verhoeyen et al., Science 239:1534-1536, 1988; Kettleborough et al., Protein Engineering. 4:773-3783, 1991; Maeda et al., Human Antibodies Hybridoma 2:124-134, 1991; Gorman et al., PNAS USA. 88:4181-4185, 1991; Tempest et al., Bio/Technology 9:266-271, 1991; Co et al., PNAS USA. 88:2869-2873, 1991; Carter et al., PNAS USA. 89:4285-4289, 1992; and Co et al., J Immunol. 148:1149-1154, 1992. In some embodiments, humanized antibodies preserve all CDR sequences (for example, a humanized mouse antibody which contains all six CDRs from the mouse antibodies). In other embodiments, humanized antibodies have one or more CDRs (one, two, three, four, five, six) which are altered with respect to the original antibody, which are also termed one or more CDRs "derived from" one or more CDRs from the original antibody.

### 2B. Fc-Fusion Polypeptides

In certain embodiments, the conjugate comprises an Fc-fusion polypeptide as the agent. Typically, the Fc-fusion polypeptide is a therapeutic Fc-fusion polypeptide, optionally an existing and/or commercially-approved Fc-fusion polypeptide. Particular examples of Fc-fusion polypeptides include abatercept, aflibercept, alefacept, belatacept, etanercept, rilonacept, and romiplastin.

Abatercept blocks the interactions between CD80 or CD86 on antigen-presenting cells (APCs) and CD28 on T-cells, thereby inhibiting T-cell activation. It is currently approved in the United States for the treatment of rheumatoid arthritis. Abatercept is composed of the extracellular domain (ECD) of human cytotoxic T lymphocyte associated molecule-4 (CTLA-4) fused to human IgG1 Fc. Hence, certain conjugates comprise a BBB-transport moiety linked to abatercept, or an active fragment or variant thereof.

Aflibercept binds to all forms of VEGF-A, as well as placental growth factor, thereby inhibiting angiogenesis. It is currently approved in the United States for the treatment of wet macular degeneration and metastatic colorectal cancer. Aflibercept is composed of the ECDs of VEGF receptors 1 and 2 fused to human IgG1 Fc. Certain conjugates thus comprise a BBB-transport moiety linked to aflibercept, or an active fragment or variant thereof.

Alefacept binds CD2, blocks the interactions between lymphocyte function-associated antigen (LFA) on APCs and CD2 on T-cells, thereby inhibiting T-cell activation. It is approved in the United States for the treatment of plaque psoriasis. Alefacept is composed of the first ECD of lymphocyte function-associated antigen 3 (LFA-3) fused to human IgG1 Fc. Certain conjugates therefore comprise a BBB-transport moiety linked to alefacept, or an active fragment or variant thereof.

Belatacept blocks the interactions between CD80 or CD86 on APCs and CD28 on T-cells, thereby inhibiting T-cell activation. It is approved in the United States for prophylaxis of organ rejection in adults receiving a kidney transplant. Belatacept is composed of the ECD of CTLA-4 fused to human IgG1 Fc, and differs from abatacept by two amino acid substitutions (L104E, A29Y) in the CTLA-4 region. Certain conjugates comprise a BBB-transport moiety linked to belatacept, or an active fragment or variant thereof.

Etanercept binds membrane-bound and soluble forms of TNF, thereby reducing concentrations of inflammatory cytokines. It is approved in the United States for the treatment of rheumatoid arthritis. Etanercept is composed of the 75 kDa soluble ECD of tumor necrosis factor (TNF) receptor II fused to human IgG1 Fc. Certain conjugates comprise a BBB-transport moiety linked to etanercept, or an active fragment or variant thereof.

Rilonacept binds IL-1, thereby preventing its interaction with endogenous cell-surface receptors. It is approved in the United States for the treatment of plaque psoriasis. Rilonacept is composed of two chains, each comprising the C-terminus of the IL-1R accessory protein ligand binding region fused to the N-terminus of the IL-1RI ECD, fused to human IgG1 Fc. Certain conjugates thus comprise a BBB-transport moiety linked to rilonacept, or an active fragment or variant thereof.

Romiplastin binds to and agonizes the TPO receptor. It is approved in the United States for the treatment of thrombocytopenia. Romiplastin is composed of a peptide thrombopoietin (TPO) mimetic fused to the C-terminus of aglycosylated human IgG1 Fc. It is recombinantly produced in *E. Coli* and its Fc functionality is minimized due to lack of glycosylation. Certain conjugates thus comprise a BBB-transport moiety linked to romiplastin, or an active fragment or variant thereof.

### 3. Linkers

As noted above, certain conjugates may employ one or more optional linker groups. The term "linkage," "linker," "linker moiety," or "L" is used herein to refer to a linker that can be used to separate a BBB-moiety from an agent, or to separate a first agent from another agent or label (fluorescence label), for instance where two or more agents are linked to form a conjugate. The linker may be physiologically stable or may include a releasable linker such as a labile linker or an enzymatically degradable linker (e.g., proteolytically cleavable linkers). In certain aspects, the linker may be a peptide linker. In some aspects, the linker may be a non-peptide linker or non-proteinaceous linker. In some aspects, the linker may be particle, such as a nanoparticle.

The linker may be charge neutral or may bear a positive or negative charge. A reversible or labile linker contains a reversible or labile bond. A linker may optionally include a spacer that increases the distance between the two joined atoms. A spacer may further add flexibility and/or length to the linker. Spacers may include, but are not be limited to, alkyl groups, alkenyl groups, alkynyl groups, aryl groups, aralkyl groups, aralkenyl groups, aralkynyl groups; each of which can contain one or more heteroatoms, heterocycles, amino acids, nucleotides, and saccharides.

A labile bond is a covalent bond other than a covalent bond to a hydrogen atom that is capable of being selectively broken or cleaved under conditions that will not break or cleave other covalent bonds in the same molecule. More specifically, a labile bond is a covalent bond that is less stable (thermodynamically) or more rapidly broken (kinetically) under appropriate conditions than other non-labile covalent bonds in the same molecule. Cleavage of a labile bond within a molecule may result in the formation of two molecules. For those skilled in the art, cleavage or lability of a bond is generally discussed in terms of half-life (t_{1/2}) of bond cleavage (the time required for half of the bonds to cleave). Thus, labile bonds encompass bonds that can be selectively cleaved more rapidly than other bonds a molecule.

Appropriate conditions are determined by the type of labile bond and are well known in organic chemistry. A labile bond can be sensitive to pH, oxidative or reductive conditions or agents, temperature, salt concentration, the presence of an enzyme (such as esterases, including nucleases, and proteases), or the presence of an added agent. For example, increased or decreased pH is the appropriate conditions for a pH-labile bond.

In some embodiments, the linker is an organic moiety constructed to contain an alkyl, aryl and/or amino acid backbone, and containing an amide, ether, ester, hydrazone, disulphide linkage or any combination thereof. Linkages containing amino acid, ether and amide bound components are stable under conditions of physiological pH, normally 7.4 in serum. As above, also included are linkages that contain esters or hydrazones and are stable at serum pH, but which hydrolyze to release the siRNA molecule when exposed to lysosomal pH. Disulphide linkages are also included, at least in part because they are sensitive to reductive cleavage. In addition, amino acid linkers may be designed to be sensitive to cleavage by specific enzymes in the desired target organ or, for example, in the lysosome. Exemplary linkers are described in Blattler et al. (19S5) Biochem. 24:1517-1524; King et al (1986) Biochem. 25:5774-5779; Srinivasachar and Nevill (1989) Biochem. 28:2501-2509, and elsewhere.

In some embodiments, the linker is about 1 to about 30 atoms in length, or about 1, 2, 3, 4, 5, 6, 7, 8, 9, 0, 11, 12, 13, 14, 15, 16, 17, 18, 19, 20, 21, 22, 23, 24, 25, 26, 27, 28, 29, or 30 atoms in length, including all ranges in between. In certain embodiments, the linker is about 1 to 30 atoms in length with carbon chain atoms which may be substituted by heteroatoms independently selected from the group consisting of O, N. or S. In some embodiments, from 1- 4 or from 5 to 15 of the C atoms are substituted with a heteroatom independently selected from O, N, S.

In certain embodiments, the linker comprises or consists of a structure selected from the following: -O-, -NH-, -S-, -C(O)-, C(O)-NH, NH-C(O)-NH, O-C(O)-NH, -C(S)-, - CH₂-, -CH₂-CH₂-, -CH₂-CH₂-CH₂-, -CH₂-CH₂-CH₂-CH₂-, -O-CH₂-, -CH₂-O-, - O-CH₂-CH₂-, -CH₂-O-CH₂-, -CH₂-CH₂-O-, -O-CH₂-CH₂-CH₂-, -CH₂-O-CH₂-CH₂-, -CH₂-CH₂-O-CH₂-, -CH₂-CH₂-CH₂-O-, -O-CH₂-CH₂-CH₂-CH₂-, -CH₂-O-CH₂-CH₂-CH₂-, -CH₂-CH₂-O-CH₂-CH₂-, -CH₂-CH₂-CH₂-O-CH₂-, -CH₂-CH₂-CH₂-CH₂-O-, -C(O)-NH-CH₂-, -C(O)-NH-CH₂-CH₂-, -CH₂-C(O)-NH-CH₂-, -CH₂-CH₂-C(O)-NH-, -C(O)-NH-CH₂-CH₂-CH₂-, -CH₂-C(O)-NH-CH₂-CH₂-, -CH₂-CH₂-C(O)-NH-CH₂-, -CH₂-CH₂-CH₂-C(O)-NH-, -C(O)-NH-CH₂-CH₂-CH₂-CH₂-, -CH₂-C(O)-NH-CH₂-CH₂-CH₂-, -CH₂-CH₂-C(O)-NH-CH₂-CH₂-, -CH₂-CH₂-CH₂-C(O)-NH-CH₂-, -CH₂-CH₂-CH₂-C(O)-NH-CH₂-CH₂-, -CH₂-CH₂-CH₂-CH₂-C(O)-NH-, -NH-C(O)-CH₂-, -CH₂-NH-C(O)-CH₂-, -CH₂-CH₂-NH-C(O)-CH₂-, -NH-C(O)-CH₂-CH₂-, - CH₂-NH-C(O)-CH₂-CH₂, -CH₂-CH₂-NH-C(O)-CH₂-CH₂, -C(O)-NH-CH₂-, -C(O)-NH-CH₂-CH₂-, -O-C(O)-NH-CH₂-, -O-C(O)-NH-CH₂-CH₂-, -NH-CH₂-, -NH-CH₂-CH₂-, -CH₂-NH-CH₂-, -CH₂-CH₂-NH-CH₂-, -C(O)-CH₂-, -C(O)-CH₂-CH₂-, -CH₂-C(O)-CH₂-, -CH₂-CH₂-C(O)-CH₂-, -CH₂-CH₂-C(O)-CH₂-CH₂-, -CH₂-CH₂-C(O)-, - CH₂-CH₂-CH₂-C(O)-NH-CH₂-CH₂-NH-, -CH₂-CH₂-CH₂-C(O)-NH-CH₂-CH₂-NH-C(O)-, -CH₂-CH₂-CH₂-C(O)-NH-CH₂-CH₂-NH-C(O)-CH₂-, bivalent cycloalkyl group, - N(R⁶)-, R⁶ is H or an organic radical selected from the group consisting of alkyl, substituted alkyl, alkenyl, substituted alkenyl, alkynyl, substituted alkynyl, aryl and substituted aryl.

In some embodiments, the linker comprises a releasable linker. In some embodiments, the releasable linker is selected from the group consisting of: carboxylate ester, phosphate ester, anhydride, acetal, ketal, acyloxyalkyl ether, imine, orthoester, thio ester, thiol ester, carbonate, and hydrazone. In certain embodiments, the linker contains a moiety subject to hydrolysis upon delivery to the lysosomal environment (e.g., susceptible to hydrolysis at the lysosomal pH or upon contact to a lysosomal enzyme).

In some embodiments, the linker comprises a stable linker. In some embodiments, the stable linkage is selected from the group consisting of: succinimide, propionic acid, carboxymethylate linkages, ethers, carbamates, amides, amines, carbamides, imides, aliphatic C-C bonds, and thio ethers.

In some embodiments, the linker comprises or consists of polymer such as a polyethylene glycol or polypropylene glycol. The terms "PEG," "polyethylene glycol" and "poly(ethylene glycol)" as used herein, are interchangeable and meant to encompass any water-soluble poly(ethylene oxide) derivative. PEG is a well-known polymer with good solubility in many aqueous and organic solvents, which exhibits low toxicity, lack of immunogenicity, and is clear, colorless, odorless, and stable. Similar products may be obtained with other water-soluble polymers, as described herein, including without limitation; polyvinyl alcohol, other poly(alkylene oxides) such as poly(propylene glycol) and the like, poly(oxyethylated polyols) such as poly(oxyethylated glycerol) and the like, carboxymethylcellulose, dextran, polyvinyl alcohol, polyvinyl purrolidone, poly-1,3-dioxolane, poly-1,3,6-trioxane, ethylene/maleic anhydride, and polyaminoacids. One skilled in the art will be able to select the desired polymer based on the desired dosage, circulation time, resistance to proteolysis, and other considerations.

Typically, PEGs for use in accordance with the conjugates described herein comprise the following structure "-(OCH2CH2)n-" where (n) is about 1 to 4000, about 20 to 1400, or about 20-800. In particular embodiments, PEG also includes "-O-(CH2CH2O)n-CH2CH2-" and "-(OCH2CH2)n-O-" depending upon whether or not the terminal oxygens have been displaced. The term "PEG" includes structures having various terminal or "end capping" groups. The term "PEG" also includes a polymer that contains a majority, that is to say, greater than 50%, of -OCH2CH2- repeating subunits. With respect to specific forms, the PEG can take any number of a variety of molecular weights, as well as structures or geometries such as "branched," "linear," "forked," "multifunctional" PEG molecules.

Representative polymeric reagents and methods for conjugating such polymers to an active moiety are described in Harris, J.M. and Zalipsky, S., Eds, Poly(ethylene glycol), Chemistry and Biological Applications, ACS, Washington, 1997; Veronese, F., and J.M. Harris, Eds., Peptide and Protein PEGylation, Advanced Drug Delivery Reviews, 54(4); 453-609 (2002); Zalipsky, S., et al., "Use of Functionalized Poly Ethylene Glycols) for Modification of Polypeptides" in Polyethylene Glycol Chemistry: Biotechnical and Biomedical Applications, J.M. Harris, ed., Plenus Press, New York (1992); Zalipsky (1995) Advanced Drug Reviews 16:157-182; and in Roberts et al., Adv. Drug Delivery Reviews, 54, 459-476 (2002).

A wide variety of PEG derivatives are both commercially available and suitable for use in the preparation of the PEG-conjugates of the invention. For example, NOF Corp.'s SUNBRIGHT^{®} Series provides numerous PEG derivatives, including methoxypolyethylene glycols and activated PEG derivatives such as succinimidyl ester, methoxy-PEG amines, maleimides, and carboxylic acids, for coupling by various methods to polypeptides and polynucleotides and Nektar Therapeutics' Advanced PEGylation also offers diverse PEG-coupling technologies to improve the safety and efficacy of therapeutics. Additional PEGs for use in forming conjugates include those available from Polypure (Norway), from QuantaBioDesign LTD (Ohio) JenKem Technology, Nanocs Corporation, and Sunbio, Inc (South Korea). Further PEG reagents suitable for use in forming a conjugate, and methods of conjugation are described, for example, in Pasut et al., Expert Opin. Ther. Patents. 14(6) 859-893, 2004.

The preparation of linear or branched PEG polymers and derivatives or conjugates thereof is described, for example, in U.S. Pat. Nos. 4,904,584; 5,428,128; 5,621,039; 5,622,986; 5,643,575; 5,728,560; 5,730,990; 5,738,846; 5,811,076; 5,824,701; 5,840,900; 5,880,131; 5,900,402; 5,902,588; 5,919,455; 5,951,974; 5,965,119; 5,965,566; 5,969,040; 5,981,709; 6,011,042; 6,042,822; 6,113,906; 6,127,355; 6,132,713; 6,177,087; 6,180,095; 6,448,369; 6,495,659; 6.602,498; 6,858,736; 6,828,401; 7,026,440; 7,608,678; 7,655,747; 7,786,221; 7,872,072; and 7,910,661, each of which is incorporated herein by reference in its entirety.

In some embodiments, the linker group is hydrophilic, for instance, to enhance the solubility of the conjugate in body fluids. In some embodiments, the BBB-moiety or moieties and the agent(s) are joined by a linker comprising amino acids or peptides, lipids, or sugar residues. In some embodiments, the BBB-moiety or moieties and the agent(s) are joined at groups introduced synthetically or by posttranslational modifications.

### Exemplary Methods for Conjugation

Conjugation or coupling of a BBB-moiety to an agent of interest can be carried out using standard chemical, biochemical and/or molecular techniques. Indeed, it will be apparent how to make a conjugate in light of the present disclosure using available art-recognized methodologies. Of course, it will generally be preferred when coupling the primary components of a conjugate that the techniques employed and the resulting linking chemistries do not substantially disturb the desired functionality or activity of the individual components of the conjugate.

The particular coupling chemistry employed will depend upon the structure of the biologically active agent (e.g., small molecule, polypeptide), the potential presence of multiple functional groups within the biologically active agent, the need for protection/deprotection steps, chemical stability of the agent, and the like, and will be readily determined by one skilled in the art. Illustrative coupling chemistry useful for preparing the conjugates of the invention can be found, for example, in Wong (1991), "Chemistry of Protein Conjugation and Crosslinking", CRC Press, Boca Raton, Fla.; and Brinkley "A Brief Survey of Methods for Preparing Protein Conjugates with Dyes, Haptens, and Crosslinking Reagents," in Bioconjug. Chem., 3:2013, 1992. Preferably, the binding ability and/or activity of the conjugate is not substantially reduced as a result of the conjugation technique employed, for example, relative to the unconjugated agent or the unconjugated BBB-moiety.

In certain embodiments, a BBB-moiety may be coupled to an agent of interest either directly or indirectly. A direct reaction between a BBB-moiety and an agent of interest is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to indirectly couple a BBB-moiety and an agent of interest via a linker group, including non-peptide linkers and peptide linkers. A linker group can also function as a spacer to distance an agent of interest from the BBB-moiety in order to avoid interference with binding capabilities, targeting capabilities or other functionalities. A linker group can also serve to increase the chemical reactivity of a substituent on an agent, and thus increase the coupling efficiency. An increase in chemical reactivity may also facilitate the use of agents, or functional groups on agents, which otherwise would not be possible. The selection of releasable or stable linkers can also be employed to alter the pharmacokinetics of a conjugate and attached agent of interest. Illustrative linking groups include, for example, disulfide groups, thioether groups, acid labile groups, photolabile groups, peptidase labile groups and esterase labile groups. In other illustrative embodiments, the conjugates include linking groups such as those disclosed in U.S. Pat. No. 5,208,020 or EP Patent 0 425 235 B1, and Chari et al., Cancer Research. 52: 127-131, 1992. Additional exemplary linkers are described below.

In some embodiments, it may be desirable to couple more than one BBB-moiety to an agent, or vice versa. For example, in certain embodiments, multiple BBB-moieties are coupled to one agent, or alternatively, one or more BBB-moieties are conjugated to multiple agents. The BBB-moieties can be the same or different. Regardless of the particular embodiment, conjugates containing multiple BBB-moieties may be prepared in a variety of ways. For example, more than one BBB-moiety may be coupled directly to an agent, or linkers that provide multiple sites for attachment can be used. Any of a variety of known heterobifunctional crosslinking strategies can be employed for making conjugates of the invention. It will be understood that many of these embodiments can be achieved by controlling the stoichiometries of the materials used during the conjugation/crosslinking procedure.

In certain exemplary embodiments, a reaction between an agent comprising a succinimidyl ester functional group and a BBB-moiety comprising an amino group forms an amide linkage; a reaction between an agent comprising a oxycarbonylimidizaole functional group and a BBB-moiety comprising an amino group forms an carbamate linkage; a reaction between an agent comprising a p-nitrophenyl carbonate functional group and a BBB-moiety comprising an amino group forms an carbamate linkage; a reaction between an agent comprising a trichlorophenyl carbonate functional group and a BBB-moiety comprising an amino group forms an carbamate linkage; a reaction between an agent comprising a thio ester functional group and a BBB-moiety comprising an n-terminal amino group forms an amide linkage; a reaction between an agent comprising a proprionaldehyde functional group and a BBB-moiety comprising an amino group forms a secondary amine linkage.

In some exemplary embodiments, a reaction between an agent comprising a butyraldehyde functional group and a BBB-moiety comprising an amino group forms a secondary amine linkage; a reaction between an agent comprising an acetal functional group and a BBB-moiety comprising an amino group forms a secondary amine linkage; a reaction between an agent comprising a piperidone functional group and a BBB-moiety comprising an amino group forms a secondary amine linkage; a reaction between an agent comprising a methylketone functional group and a BBB-moiety comprising an amino group forms a secondary amine linkage; a reaction between an agent comprising a tresylate functional group and a BBB-moiety comprising an amino group forms a secondary amine linkage; a reaction between an agent comprising a maleimide functional group and a BBB-moiety comprising an amino group forms a secondary amine linkage; a reaction between an agent comprising a aldehyde functional group and a BBB-moiety comprising an amino group forms a secondary amine linkage; and a reaction between an agent comprising a hydrazine functional group and a BBB-moiety comprising an carboxylic acid group forms a secondary amine linkage.

In particular exemplary embodiments, a reaction between an agent comprising a maleimide functional group and a BBB-moiety comprising a thiol group forms a thio ether linkage; a reaction between an agent comprising a vinyl sulfone functional group and a BBB-moiety comprising a thiol group forms a thio ether linkage; a reaction between an agent comprising a thiol functional group and a BBB-moiety comprising a thiol group forms a di-sulfide linkage; a reaction between an agent comprising a orthopyridyl disulfide functional group and a BBB-moiety comprising a thiol group forms a di-sulfide linkage; and a reaction between an agent comprising an iodoacetamide functional group and a BBB-moiety comprising a thiol group forms a thio ether linkage.

In a specific embodiment, an amine-to-sulfhydryl crosslinker is used for preparing a conjugate. In one preferred embodiment, for example, the crosslinker is succinimidyl-4-(N-maleimidomethyl)cyclohexane-1-carboxylate (SMCC) (Thermo Scientific), which is a sulfhydryl crosslinker containing NHS-ester and maleimide reactive groups at opposite ends of a medium-length cyclohexane-stabilized spacer arm (8.3 angstroms). SMCC is a non-cleavable and membrane permeable crosslinker that can be used to create sulfhydryl-reactive, maleimide-activated agents (e.g., polypeptides, antibodies) for subsequent reaction with BBB-moieties. NHS esters react with primary amines at pH 7-9 to form stable amide bonds. Maleimides react with sulfhydryl groups at pH 6.5-7.5 to form stable thioether bonds. Thus, the amine reactive NHS ester of SMCC crosslinks rapidly with primary amines of an agent and the resulting sulfhydryl-reactive maleimide group is then available to react with cysteine residues of the BBB-moiety to yield specific conjugates of interest.

In certain specific embodiments, the BBB-moiety is modified to contain exposed sulfhydryl groups to facilitate crosslinking, e.g., to facilitate crosslinking to a maleimide-activated agent. In a more specific embodiment, the BBB-moiety is modified with a reagent which modifies primary amines to add protected thiol sulfhydryl groups. In an even more specific embodiment, the reagent N-succinimidyl-S-acetylthioacetate (SATA) (Thermo Scientific) is used to produce thiolated BBB-moieties.

In other specific embodiments, a maleimide-activated agent is reacted under suitable conditions with a thiolated BBB-moiety to produce a conjugate. It will be understood that by manipulating the ratios of SMCC, SATA, agent, and BBB-moiety in these reactions it is possible to produce conjugates having differing stoichiometries, molecular weights and properties.

In still other illustrative embodiments, conjugates are made using bifunctional protein coupling agents such as N-succinimidyl-3-(2-pyridyldithio)propionate (SPDP), succinimidyl-4-(N-maleimidomethyl)cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl)hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particular coupling agents include N-succinimidyl-3-(2-pyridyldithio)propionate (SPDP) (Carlsson et al., Biochem. J. 173:723-737 [1978]) and N-succinimidyl-4-(2-pyridylthio)pentanoate (SPP) to provide for a disulfide linkage.

The specific crosslinking strategies discussed herein are but a few of many examples of suitable conjugation strategies that may be employed in producing conjugates of the invention. It will be evident to those skilled in the art that a variety of other bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, e.g., U.S. Patent No. 4,671,958, to Rodwell et al.

Conjugates can also be prepared by a various "click chemistry" techniques, including reactions that are modular, wide in scope, give very high yields, generate mainly inoffensive byproducts that can be removed by non-chromatographic methods, and can be stereospecific but not necessarily enantioselective (see Kolb et al., Angew Chem Int Ed Engl. 40:2004-2021, 2001). Particular examples include conjugation techniques that employ the Huisgen 1,3-dipolar cycloaddition of azides and alkynes, also referred to as "azide-alkyne cycloaddition" reactions (see Hein et al., Pharm Res. 25:2216-2230, 2008). Non-limiting examples of azide-alkyne cycloaddition reactions include copper-catalyzed azide-alkyne cycloaddition (CuAAC) reactions and ruthenium-catalyzed azide-alkyne cycloaddition (RuAAC) reactions.

CuAAC works over a broad temperature range, is insensitive to aqueous conditions and a pH range over 4 to 12, and tolerates a broad range of functional groups (see Himo et al, J Am Chem Soc. 127:210-216, 2005). The active Cu(I) catalyst can be generated, for example, from Cu(I) salts or Cu(II) salts using sodium ascorbate as the reducing agent. This reaction forms 1,4-substituted products, making it region-specific (see Hein et al., supra).

RuAAC utilizes pentamethylcyclopentadienyl ruthenium chloride [Cp*RuCl] complexes that are able to catalyze the cycloaddition of azides to terminal alkynes, regioselectively leading to 1,5-disubstituted 1,2,3-triazoles (see Rasmussen et al., Org. Lett. 9:5337-5339, 2007). Further, and in contrast to CuAAC, RuAAC can also be used with internal alkynes to provide fully substituted 1,2,3-triazoles.

Certain embodiments thus include BBB-moieties or polypeptide agents that comprise at least one unnatural amino acid with an azide side-chain or an alkyne side-chain, including internal and terminal unnatural amino acids (e.g., N-terminal, C-terminal). Certain of these polypeptides can be formed by in vivo or in vitro (e.g., cell-free systems) incorporation of unnatural amino acids that contain azide side-chains or alkyne side-chains. Exemplary in vivo techniques include cell culture techniques, for instance, using modified E. coli (see Travis and Schultz, The Journal of Biological Chemistry. 285:11039-44, 2010; and Deiters and Schultz, Bioorganic & Medicinal Chemistry Letters. 15:1521-1524, 2005), and exemplary in vitro techniques include cell-free systems (see Bundy, Bioconjug Chem. 21:255-63, 2010).

In the case where the conjugate is a fusion polypeptide, the fusion polypeptide may generally be prepared using standard techniques. Preferably, however, a fusion polypeptide is expressed as a recombinant polypeptide in an expression system, described herein and known in the art. Fusion polypeptides of the invention can contain one or multiple copies of a polypeptide sequence and may contain one or multiple copies of a polypeptide-based agent of interest (e.g., antibody or antigen-binding fragment thereof), present in any desired arrangement.

For fusion proteins, DNA sequences encoding the BBB-moiety, the polypeptide agent (e.g., antibody), and optionally peptide linker components may be assembled separately, and then ligated into an appropriate expression vector. The 3' end of the DNA sequence encoding one polypeptide component is ligated, with or without a peptide linker, to the 5' end of a DNA sequence encoding the other polypeptide component(s) so that the reading frames of the sequences are in phase. The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the most C-terminal polypeptide. This permits translation into a single fusion polypeptide that retains the biological activity of both component polypeptides.

Similar techniques, mainly the arrangement of regulatory elements such as promoters, stop codons, and transcription termination signals, can be applied to the recombinant production of non-fusion proteins, for instance, BBB-moiety polypeptides and polypeptide agents (e.g., antibody agents) for the production of non-fusion conjugates.

Polynucleotides and fusion polynucleotides of the invention can contain one or multiple copies of a nucleic acid encoding a BBB-moiety polypeptide sequence, and/or may contain one or multiple copies of a nucleic acid encoding a polypeptide agent.

In some embodiments, a nucleic acids encoding a BBB-moiety polypeptide, polypeptide agent, and/or fusion thereof are introduced directly into a host cell, and the cell incubated under conditions sufficient to induce expression of the encoded polypeptide(s). The polypeptide sequences of this disclosure may be prepared using standard techniques well known to those of skill in the art in combination with the polypeptide and nucleic acid sequences provided herein.

Therefore, according to certain related embodiments, there is provided a recombinant host cell which comprises a polynucleotide or a fusion polynucleotide that encodes a polypeptide described herein. Expression of a BBB-moiety polypeptide, polypeptide agent, or fusion thereof in the host cell may conveniently be achieved by culturing under appropriate conditions recombinant host cells containing the polynucleotide. Following production by expression, the polypeptide(s) may be isolated and/or purified using any suitable technique, and then used as desired.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary (CHO) cells, HeLa cells, baby hamster kidney cells, HEK-293 cells, NSO mouse melanoma cells and many others. A common, preferred bacterial host is E. coli. The expression of polypeptides in prokaryotic cells such as E. coli is well established in the art. For a review, see for example Pluckthun, A. Bio/Technology. 9:545-551 (1991). Expression in eukaryotic cells in culture is also available to those skilled in the art as an option for recombinant production of polypeptides (see Ref, Curr. Opinion Biotech. 4:573-576, 1993; and Trill et al., Curr. Opinion Biotech. 6:553-560, 1995.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992, or subsequent updates thereto.

The term "host cell" is used to refer to a cell into which has been introduced, or which is capable of having introduced into it, a nucleic acid sequence encoding one or more of the polypeptides described herein, and which further expresses or is capable of expressing a selected gene of interest, such as a gene encoding any herein described polypeptide. The term includes the progeny of the parent cell, whether or not the progeny are identical in morphology or in genetic make-up to the original parent, so long as the selected gene is present. Host cells may be chosen for certain characteristics, for instance, the expression of a formylglycine generating enzyme (FGE) to convert a cysteine or serine residue within a sulfatase motif into a formylglycine (FGly) residue, or the expression of aminoacyl tRNA synthetase(s) that can incorporate unnatural amino acids into the polypeptide, including unnatural amino acids with an azide side-chain, alkyne side-chain, or other desired side-chain, to facilitate conjugation.

Accordingly there is also contemplated a method comprising introducing such nucleic acid(s) into a host cell. The introduction of nucleic acids may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. The introduction may be followed by causing or allowing expression from the nucleic acid, e.g., by culturing host cells under conditions for expression of the gene. In one embodiment, the nucleic acid is integrated into the genome (e.g. chromosome) of the host cell. Integration may be promoted by inclusion of sequences which promote recombination with the genome, in accordance-with standard techniques.

The present invention also provides, in certain embodiments, a method which comprises using a nucleic acid construct described herein in an expression system in order to express a particular polypeptide, such as a BBB-moiety polypeptide, polypeptide agent, or fusion protein thereof, as described herein.

### Polypeptide Variants and Fragments

Certain embodiments include variants and/or fragments of the reference polypeptides described herein, whether described by name or by reference to a sequence identifier. Included are variants and fragments of the polypeptides that comprise an immunoglobulin Fc region (Fc region-containing polypeptides), BBB-moieties, antibodies and antigen binding fragments thereof, and therapeutic Fc fusion polypeptides. The wild-type or most prevalent or commercially-employed sequences of these polypeptides are known in the art, and can be used as a comparison for the variants and fragments described herein.

A polypeptide "variant," as the term is used herein, is a polypeptide that typically differs from a polypeptide specifically disclosed herein by one or more substitutions, deletions, additions and/or insertions. Variant polypeptides are biologically active, that is, they continue to possess the enzymatic or binding activity of a reference polypeptide. Such variants may result from, for example, genetic polymorphism and/or from human manipulation.

In many instances, a biologically active variant will contain one or more conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. As described above, modifications may be made in the structure of the polynucleotides and polypeptides of the present invention and still obtain a functional molecule that encodes a variant or derivative polypeptide with desirable characteristics. When it is desired to alter the amino acid sequence of a polypeptide to create an equivalent, or even an improved, variant or portion of a polypeptide of the invention, one skilled in the art will typically change one or more of the codons of the encoding DNA sequence according to Table A below.

| **Table A** | | | | | | | | |
|---|---|---|---|---|---|---|---|---|
| Amino Acids | | | Codons | | | | | |
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in the peptide sequences of the disclosed compositions, or corresponding DNA sequences which encode said peptides without appreciable loss of their utility.

In making such changes, the hydropathic index of amino acids may be considered. The importance of the hydropathic amino acid index in conferring interactive biologic function on a protein is generally understood in the art (Kyte & Doolittle, 1982, incorporated herein by reference). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte & Doolittle, 1982). These values are: isoleucine (+4.5); valine (+4.2); leucine (+3.8); phenylalanine (+2.8); cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). It is known in the art that certain amino acids may be substituted by other amino acids having a similar hydropathic index or score and still result in a protein with similar biological activity, i.e., still obtain a biological functionally equivalent protein. In making such changes, the substitution of amino acids whose hydropathic indices are within ±2 is preferred, those within ±1 are particularly preferred, and those within ±0.5 are even more particularly preferred.

It is also understood in the art that the substitution of like amino acids can be made effectively on the basis of hydrophilicity. U.S. Patent 4,554,101 (specifically incorporated herein by reference in its entirety), states that the greatest local average hydrophilicity of a protein, as governed by the hydrophilicity of its adjacent amino acids, correlates with a biological property of the protein. As detailed in U. S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

A variant may also, or alternatively, contain non-conservative changes. In a preferred embodiment, variant polypeptides differ from a native sequence by substitution, deletion or addition of fewer than about 10, 9, 8, 7, 6, 5, 4, 3, 2 amino acids, or even 1 amino acid. Variants may also (or alternatively) be modified by, for example, the deletion or addition of amino acids that have minimal influence on the immunogenicity, secondary structure, enzymatic activity, and/or hydropathic nature of the polypeptide.

In certain embodiments, a polypeptide sequence is about, at least about, or up to about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700. 700, 710, 720, 730, 740, 750, 760, 770, 780, 790, 800. 800, 810, 820, 830, 840, 850, 860, 870, 880, 890, 900, 900, 910, 920, 930, 940, 950, 960, 970, 980, 990, 1000 or more contiguous amino acids in length, including all integers in between, and which may comprise all or a portion of a reference sequence (*see, e.g.,* Sequence Listing).

In other specific embodiments, a polypeptide sequence consists of about or no more than about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800. 800, 810, 820, 830, 840, 850, 860, 870, 880, 890, 900, 900, 910, 920, 930, 940, 950, 960, 970, 980, 990, 1000 or more contiguous amino acids, including all integers in between, and which may comprise all or a portion of a reference sequence (*see, e.g.,* Sequence Listing).

In still other specific embodiments, a polypeptide sequence is about 10-1000, 10-900, 10-800, 10-700, 10-600, 10-500, 10-400, 10-300, 10-200, 10-100, 10-50, 10-40, 10-30, 10-20, 20-1000, 20-900, 20-800, 20-700, 20-600, 20-500, 20-400, 20-300, 20-200, 20-100, 20-50, 20-40, 20-30, 50-1000, 50-900, 50-800, 50-700, 50-600, 50-500, 50-400, 50-300, 50-200, 50-100, 100-1000, 100-900, 100-800, 100-700, 100-600, 100-500, 100-400, 100-300, 100-200, 200-1000, 200-900, 200-800, 200-700, 200-600, 200-500, 200-400, or 200-300 contiguous amino acids, including all ranges in between, and comprises all or a portion of a reference sequence. In certain embodiments, the C-terminal or N-terminal region of any reference polypeptide may be truncated by about 1, 2, 3, 4, 5, 6, 7, 8, 9, 10, 15, 20, 25, 30, 35, 40, 45, 50, 60, 70, 80, 90, 100, 110, 120, 130, 140, 150, 160, 170, 180, 190, 200, 250, 300, 350, 400, 450, 500, 550, 600, 650, 700, 750, or 800 or more amino acids, or by about 10-50, 20-50, 50-100, 100-150, 150-200, 200-250, 250-300, 300-350, 350-400, 400-450, 450-500, 500-550, 550-600, 600-650, 650-700, 700-750, 750-800 or more amino acids, including all integers and ranges in between (e.g., 101, 102, 103, 104, 105), so long as the truncated polypeptide retains the binding properties and/or activity of the reference polypeptide. Typically, the biologically-active fragment has no less than about 1%, about 5%, about 10%, about 25%, or about 50% of an activity of the biologically-active reference polypeptide from which it is derived.

In general, variants will display at least about 30%, 40%, 50%, 55%, 60%, 65%, 70%, 75%, 80%, 85%, 90%, 91%, 92%, 93%, 94%, 95%, 96%, 97%, 98%, 99% similarity or sequence identity or sequence homology to a reference polypeptide sequence. Moreover, sequences differing from the native or parent sequences by the addition (e.g., C-terminal addition, N-terminal addition, both), deletion, truncation, insertion, or substitution of about 1, 2, 3, 4, 5, 6, 7, 8, 9, 10, 11, 12, 13, 14, 15, 16, 17, 18, 19, 20, 30, 40, 50, 60, 70, 80, 90, 100 or more amino acids but which retain the properties or activities of a parent or reference polypeptide sequence are contemplated.

In some embodiments, variant polypeptides differ from reference sequence by at least one but by less than 50, 40, 30, 20, 15, 10, 8, 6, 5, 4, 3 or 2 amino acid residue(s). In other embodiments, variant polypeptides differ from a reference sequence by at least 1% but less than 20%, 15%, 10% or 5% of the residues. (If this comparison requires alignment, the sequences should be aligned for maximum similarity. "Looped" out sequences from deletions or insertions, or mismatches, are considered differences.)

Calculations of sequence similarity or sequence identity between sequences (the terms are used interchangeably herein) are performed as follows. To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In certain embodiments, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position.

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch, (J. Mol. Biol. 48: 444-453, 1970) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller (Cabios. 4:11-17, 1989) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al., (1990, J. Mol. Biol, 215: 403-10). BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (Nucleic Acids Res. 25: 3389-3402, 1997). When utilizing BLAST and Gapped BLAST programs, the default parameters of the respective programs (e.g., XBLAST and NBLAST) can be used.

In one embodiment, as noted above, polynucleotides and/or polypeptides can be evaluated using a BLAST alignment tool. A local alignment consists simply of a pair of sequence segments, one from each of the sequences being compared. A modification of Smith-Waterman or Sellers algorithms will find all segment pairs whose scores cannot be improved by extension or trimming, called high-scoring segment pairs (HSPs). The results of the BLAST alignments include statistical measures to indicate the likelihood that the BLAST score can be expected from chance alone.

The raw score, S, is calculated from the number of gaps and substitutions associated with each aligned sequence wherein higher similarity scores indicate a more significant alignment. Substitution scores are given by a look-up table (*see* PAM, BLOSUM).

Gap scores are typically calculated as the sum of G, the gap opening penalty and L, the gap extension penalty. For a gap of length n, the gap cost would be G+Ln. The choice of gap costs, G and L is empirical, but it is customary to choose a high value for G (10-15), *e.g.,* 11, and a low value for L (1-2) *e.g.,* 1.

The bit score, S', is derived from the raw alignment score S in which the statistical properties of the scoring system used have been taken into account. Bit scores are normalized with respect to the scoring system, therefore they can be used to compare alignment scores from different searches. The terms "bit score" and "similarity score" are used interchangeably. The bit score gives an indication of how good the alignment is; the higher the score, the better the alignment.

The E-Value, or expected value, describes the likelihood that a sequence with a similar score will occur in the database by chance. It is a prediction of the number of different alignments with scores equivalent to or better than S that are expected to occur in a database search by chance. The smaller the E-Value, the more significant the alignment. For example, an alignment having an E value of e⁻¹¹⁷ means that a sequence with a similar score is very unlikely to occur simply by chance. Additionally, the expected score for aligning a random pair of amino acids is required to be negative, otherwise long alignments would tend to have high score independently of whether the segments aligned were related. Additionally, the BLAST algorithm uses an appropriate substitution matrix, nucleotide or amino acid and for gapped alignments uses gap creation and extension penalties. For example, BLAST alignment and comparison of polypeptide sequences are typically done using the BLOSUM62 matrix, a gap existence penalty of 11 and a gap extension penalty of 1.

In one embodiment, sequence similarity scores are reported from BLAST analyses done using the BLOSUM62 matrix, a gap existence penalty of 11 and a gap extension penalty of 1.

In a particular embodiment, sequence identity/similarity scores provided herein refer to the value obtained using GAP Version 10 (GCG, Accelrys, San Diego, Calif.) using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix (Henikoff and Henikoff, PNAS USA. 89:10915-10919, 1992). GAP uses the algorithm of Needleman and Wunsch (J Mol Biol. 48:443-453, 1970) to find the alignment of two complete sequences that maximizes the number of matches and minimizes the number of gaps.

In one particular embodiment, the variant polypeptide comprises an amino acid sequence that can be optimally aligned with a reference polypeptide sequence (*see, e.g.,* the Tables or Sequence Listing) to generate a BLAST bit scores or sequence similarity scores of at least about 50, 60, 70, 80, 90, 100, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or more, including all integers and ranges in between, wherein the BLAST alignment used the BLOSUM62 matrix, a gap existence penalty of 11, and a gap extension penalty of 1.

As noted above, a reference polypeptide may be altered in various ways including amino acid substitutions, deletions, truncations, additions, and insertions. Methods for such manipulations are generally known in the art. For example, amino acid sequence variants of a reference polypeptide can be prepared by mutations in the DNA. Methods for mutagenesis and nucleotide sequence alterations are well known in the art. See, for example, Kunkel (PNAS USA. 82: 488-492, 1985); Kunkel et al., (Methods in Enzymol. 154: 367-382, 1987), U.S. Pat. No. 4,873,192, Watson, J. D. et al., ("Molecular Biology of the Gene," Fourth Edition, Benjamin/Cummings, Menlo Park, Calif., 1987) and the references cited therein. Guidance as to appropriate amino acid substitutions that do not affect biological activity of the protein of interest may be found in the model of Dayhoff et al., (1978) Atlas of Protein Sequence and Structure (Natl. Biomed. Res. Found., Washington, D.C.).

Methods for screening gene products of combinatorial libraries made by such modifications, and for screening cDNA libraries for gene products having a selected property are known in the art. Such methods are adaptable for rapid screening of the gene libraries generated by combinatorial mutagenesis of reference polypeptides. As one example, recursive ensemble mutagenesis (REM), a technique which enhances the frequency of functional mutants in the libraries, can be used in combination with the screening assays to identify polypeptide variants (Arkin and Yourvan, PNAS USA 89: 7811-7815, 1992; Delgrave et al., Protein Engineering. 6: 327-331, 1993).

### Methods of Use and Pharmaceutical Compositions

Certain embodiments include methods of treating a subject in need thereof, comprising administering to the subject (a) a polypeptide that comprises an immunoglobulin Fc region, and (b) a conjugate described herein. Also included are methods of delivering a conjugate to the nervous system (e.g., central nervous system tissues) of a subject, for example, methods of enhancing delivery of a conjugate agent across the BBB of a subject. In certain embodiments, the administration is systemic administration, for example, intravenous administration. In particular embodiments, the conjugate comprises a blood-brain barrier (BBB)-transport moiety linked to (i) a therapeutic antibody or antigen binding fragment thereof or (ii) a therapeutic Fc-fusion polypeptide.

In certain embodiments, (a) and (b) are administered separately, for example, as separate pharmaceutical compositions, as described herein. In some embodiments, (a) and (b) are administered at the same time or about the same time. In some embodiments, (a) is administered about, at least about, or no more than about 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 200, 220, 240, 260, 280, or 300 minutes before (b). In some embodiments, (a) is administered about, at least about, or no more than about 1, 2, 3, 4, 5, 6, 7, 8, 9, 10, 11, 12, 16, 24, 30, 36, 42, 48, 60, 72, 84, or 96 hours before (b).

In particular embodiments, (a) and (b) are administered together, for example, as a pharmaceutical composition that comprises (a) and (b), as described herein.

In specific embodiments, systemic administration of (a) and (b) increase the delivery of (a) across the BBB and into tissues of the CNS relative to systemic administration of (a) alone.

In certain embodiments, the methods or compositions increase the rate and/or levels (amount) of delivery of the conjugate to CNS tissues, relative to, for example, delivery by administering the conjugate alone. In particular embodiments, such is increased by about or at least about 10, 20, 30, 40, 50, 60, 70, 80, 90, 100, 200, 300, 400, 500, 600, 700, 800, 900, 1000% or more, relative to administration of the conjugate alone.

In some instances, a subject has a disease, disorder, or condition that is associated with the central nervous system (CNS) or that has a CNS component. In certain instances, the increased delivery of the conjugate across the blood brain barrier to CNS tissues relative to peripheral tissues can improve treatment, for instance, by increasing the tissue concentration of the conjugate in the CNS, and/or by reducing side-effects associated with exposure of the conjugate to peripheral tissues/organs.

In some embodiments, the method and compositions may be used to treat various cancers, including cancers of the central nervous system (CNS), or neurological cancers. In some instances, the neurological cancer is a metastatic brain cancer. Examples of cancers that can metastasize to the brain include, without limitation, breast cancers, lung cancers, genitourinary tract cancers, gastrointestinal tract cancers (e.g., colorectal cancers, pancreatic carcinomas), osteosarcomas, melanomas, head and neck cancers, prostate cancers (e.g., prostatic adenocarcinomas), and lymphomas. Certain embodiments thus include methods for treating, inhibiting or preventing metastasis of a cancer (e.g., administering an amount that, following administration, inhibits, prevents or delays metastasis of a cancer in a statistically significant manner, i.e., relative to an appropriate control as will be known to those skilled in the art). In particular embodiments, the subject has a cancer that has not yet metastasized to the central nervous system, including one or more of the above-described cancers, among others known in the art.

Also included are methods for treating a cancer of the central nervous system (CNS), optionally the brain, where the subject in need thereof has such a cancer or is at risk for developing such a condition. In some embodiments, the cancer is a primary cancer of the CNS, such as a primary cancer of the brain. For instance, the methods can be for treating a glioma, meningioma, pituitary adenoma, vestibular schwannoma, primary CNS lymphoma, or primitive neuroectodermal tumor (medulloblastoma). In some embodiments, the glioma is an astrocytoma, oligodendroglioma, ependymoma, or a choroid plexus papilloma. In certain embodiments, the primary CNS or brain cancer is glioblastoma multiforme, such as a giant cell gliobastoma or a gliosarcoma.

In particular embodiments, the cancer is a metastatic cancer of the CNS, for instance, a cancer that has metastasized to the brain. Examples of such cancers include, without limitation, breast cancers, lung cancers, genitourinary tract cancers, gastrointestinal tract cancers (e.g., colorectal cancers, pancreatic carcinomas), osteosarcomas, melanomas, head and neck cancers, prostate cancers (e.g., prostatic adenocarcinomas), and lymphomas. Certain embodiments thus include methods for treating, inhibiting or preventing metastasis of a cancer by administering to a patient a therapeutically effective amount of a herein disclosed conjugate (e.g., in an amount that, following administration, inhibits, prevents or delays metastasis of a cancer in a statistically significant manner, i.e., relative to an appropriate control as will be known to those skilled in the art). In particular embodiments, the subject has a cancer that has not yet metastasized to the central nervous system, including one or more of the herein-described cancers, among others known in the art.

In particular embodiments, the cancer (cell) expresses or overexpresses one or more of Her2/neu, B7H3, CD20, Her1/EGF receptor(s), VEGF receptor(s), PDGF receptor(s), CD30, CD52, CD33, CTLA-4, or tenascin.

Also included is the treatment of other cancers, including breast cancer, prostate cancer, gastrointestinal cancer, lung cancer, ovarian cancer, testicular cancer, head and neck cancer, stomach cancer, bladder cancer, pancreatic cancer, liver cancer, kidney cancer, squamous cell carcinoma, melanoma, non-melanoma cancer, thyroid cancer, endometrial cancer, epithelial tumor, bone cancer, or a hematopoietic cancer. Hence, in certain embodiments, the cancer cell being treated by a conjugate overexpresses or is associated with a cancer antigen, such as human Her2/neu, Her1/EGF receptor (EGFR), Her3, A33 antigen, B7H3, CD5, CD19, CD20, CD22, CD23 (IgE Receptor), C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and/or mesothelin.

In specific embodiments, the subject has a Her2/neu-expressing cancer, such as a breast cancer, ovarian cancer, stomach cancer, aggressive uterine cancer, or metastatic cancer, such as a metastatic CNS cancer, and the BBB-moiety is conjugated to trastuzumab. In other specific embodiments, a 8H9 monoclonal antibody conjugate is used to treat a neurological cancer such as a metastatic brain cancer.

The method and compositions for treating cancers including cancers of the CNS can be combined with other therapeutic modalities. For example, the methods and compositions can be employed or administered to a subject before, during, or after other therapeutic interventions, including symptomatic care, radiotherapy, surgery, transplantation, immunotherapy, hormone therapy, photodynamic therapy, antibiotic therapy, or any combination thereof. Symptomatic care includes administration of corticosteroids, to reduce cerebral edema, headaches, cognitive dysfunction, and emesis, and administration of anti-convulsants, to reduce seizures. Radiotherapy includes whole-brain irradiation, fractionated radiotherapy, and radiosurgery, such as stereotactic radiosurgery, which can be further combined with traditional surgery.

Certain embodiments relate to methods of treating inflammation or an inflammatory condition in a subject in need thereof, including inflammatory conditions of the CNS and/or those having a CNS component. "Inflammation" refers generally to the biological response of tissues to harmful stimuli, such as pathogens, damaged cells (e.g., wounds), and irritants. The term "inflammatory response" refers to the specific mechanisms by which inflammation is achieved and regulated, including, merely by way of illustration, immune cell activation or migration, cytokine production, vasodilation, including kinin release, fibrinolysis, and coagulation, among others described herein and known in the art. Ideally, inflammation is a protective attempt by the body to both remove the injurious stimuli and initiate the healing process for the affected tissue or tissues. In the absence of inflammation, wounds and infections would never heal, creating a situation in which progressive destruction of the tissue would threaten survival. On the other hand, excessive or chronic inflammation may associate with a variety of diseases, such as hay fever, atherosclerosis, and rheumatoid arthritis, among others described herein and known in the art.

The method and compositions may modulate acute inflammation, chronic inflammation, or both. Depending on the needs of the subject, certain embodiments relate to reducing acute inflammation or inflammatory responses, and certain embodiments relate to reducing chronic inflammation or chronic inflammatory responses.

Clinical signs of chronic inflammation are dependent upon duration of the illness, inflammatory lesions, cause and anatomical area affected. (see, e.g., Kumar et al., Robbins Basic Pathology-8th Ed., 2009 Elsevier, London; Miller, LM, Pathology Lecture Notes, Atlantic Veterinary College, Charlottetown, PEI, Canada). Chronic inflammation is associated with a variety of pathological conditions or diseases, including, for example, allergies, Alzheimer's disease, anemia, aortic valve stenosis, arthritis such as rheumatoid arthritis and osteoarthritis, cancer, congestive heart failure, fibromyalgia, fibrosis, heart attack, kidney failure, lupus, pancreatitis, stroke, surgical complications, inflammatory lung disease, inflammatory bowel disease, atherosclerosis, and psoriasis, among others described herein and known in the art. Hence, conjugates may be used to treat or manage chronic inflammation, modulate any of one or more of the individual chronic inflammatory responses, or treat any one or more diseases or conditions associated with chronic inflammation.

In certain embodiments, the method and compositions reduce local inflammation, systemic inflammation, or both. In certain embodiments, the method and compositions may reduce or maintain (i.e., prevent further increases) local inflammation or local inflammatory responses. In certain embodiments, conjugates may reduce or maintain (i.e., prevent further increases) systemic inflammation or systemic inflammatory responses.

In certain embodiments, the modulation of inflammation or inflammatory responses can be associated with one or more tissues or organs. Non-limiting examples of such tissues or organs include skin (e.g., dermis, epidermis, subcutaneous layer), hair follicles, nervous system (e.g., brain, spinal cord, peripheral nerves, meninges including the dura mater, arachnoid mater, and pia mater), auditory system or balance organs (e.g., inner ear, middle ear, outer ear), respiratory system (e.g., nose, trachea, lungs), gastroesophogeal tissues, the gastrointestinal system (e.g., mouth, esophagus, stomach, small intestines, large intestines, rectum), vascular system (e.g., heart, blood vessels and arteries), liver, gallbladder, lymphatic/immune system (e.g., lymph nodes, lymphoid follicles, spleen, thymus, bone marrow), uro-genital system (e.g., kidneys, ureter, bladder, urethra, cervix, Fallopian tubes, ovaries, uterus, vulva, prostate, bulbourethral glands, epidiymis, prostate, seminal vesicles, testicles), musculoskeletal system (e.g., skeletal muscles, smooth muscles, bone, cartilage, tendons, ligaments), adipose tissue, mammaries, and the endocrine system (e.g., hypothalamus, pituitary, thyroid, pancreas, adrenal glands). Accordingly, the method and compositions may be used to modulate inflammation associated with any of these tissues or organs, such as to treat conditions or diseases that are associated with the inflammation of these tissues or organs.

In particular embodiments, the inflammatory condition has a nervous system or central nervous system component, including inflammation of the brain, spinal cord, and/or the meninges. In particular embodiments, the inflammatory condition of the CNS in meningitis (e.g., bacteria, viral), encephalitis (e.g., caused by infection or autoimmune inflammation such as Acute Disseminated Enchephalomyelitis), sarcoidosis, non-metastatic diseases associated with neoplasia. Particular examples of nervous system or CNS associated inflammatory conditions include, without limitation, meningitis (i.e., inflammation of the protective membranes covering the brain and spinal cord), myelitis, encaphaloymyelitis (e.g., myalgic encephalomyelitis, acute disseminated encephalomyelitis, encephalomyelitis disseminata or multiple sclerosis, autoimmune encephalomyelitis), arachnoiditis (i.e., inflammation of the arachnoid, one of the membranes that surround and protect the nerves of the central nervous system), granuloma, drug-induced inflammation or meningitis, neurodegenerative diseases such as Alzheimer's disease, stroke, HIV-dementia, encephalitis such viral encephalitis and bacterial encephalitis, parasitic infections, inflammatory demyelinating disorders, and auto-immune disorders such as CD8+ T Cell-mediated autoimmune diseases of the CNS. Additional examples include Parkinson's disease, myasthenia gravis, motor neuropathy, Guillain-Barre syndrome, autoimmune neuropathy, Lambert-Eaton myasthenic syndrome, paraneoplastic neurological disease, paraneoplastic cerebellar atrophy, non-paraneoplastic stiff man syndrome, progressive cerebellar atrophy, Rasmussen's encephalitis, amyotrophic lateral sclerosis, Sydeham chorea, Gilles de la Tourette syndrome, autoimmune polyendocrinopathy, dysimmune neuropathy, acquired neuromyotonia, arthrogryposis multiplex, optic neuritis, stiff-man syndrome, stroke, traumatic brain injury (TBI), spinal stenosis, acute spinal cord injury, and spinal cord compression.

As noted above, also included is the treatment of inflammation associated with infections of the nervous system or CNS. Specific examples of bacterial infections associated with inflammation of the nervous system include, without limitation, streptococcal infection such as group B streptococci (e.g., subtypes III) and Streptococcus pneumoniae (e.g., serotypes 6, 9, 14, 18 and 23), Escherichia coli (e.g., carrying K1 antigen), Listeria monocytogenes (e.g., serotype IVb), neisserial infection such as Neisseria meningitidis (meningococcus), staphylococcal infection, heamophilus infection such as Haemophilus influenzae type B, Klebsiella, and Mycobacterium tuberculosis. Also included are infections by staphylococci and pseudomonas and other Gram-negative bacilli, mainly with respect to trauma to the skull, which gives bacteria in the nasal cavity the potential to enter the meningeal space, or in persons with cerebral shunt or related device (e.g., extraventricular drain, Ommaya reservoir). Specific examples of viral infections associated with inflammation of the nervous system include, without limitation, enteroviruses, herpes simplex virus type 1 and 2, human T-lymphotrophic virus, varicella zoster virus (chickenpox and shingles), mumps virus, human immunodeficiency virus (HIV), and lymphocytic choriomeningitis virus (LCMV). Meningitis may also result from infection by spirochetes such as Treponema pallidum (syphilis) and Borrelia burgdorferi (Lyme disease), parasites such as malaria (e.g., cerebral malaria), fungi such as Cryptococcus neoformans, and ameoba such as Naegleria fowleri.

Meningitis or other forms of nervous system inflammation may also associate with the spread of cancer to the meninges (malignant meningitis), certain drugs such as non-steroidal anti-inflammatory drugs, antibiotics and intravenous immunoglobulins, sarcoidosis (or neurosarcoidosis), connective tissue disorders such as systemic lupus erythematosus, and certain forms of vasculitis (inflammatory conditions of the blood vessel wall) such as Behçet's disease. Epidermoid cysts and dermoid cysts may cause meningitis by releasing irritant matter into the subarachnoid space. Accordingly, conjugates may be used to treat or manage any one or more of these conditions.

Some embodiments include methods of treating a degenerative or autoimmune disorder of the central nervous system (CNS). In some instances, the degenerative or autoimmune disorder of the CNS is Alzheimer's disease, Huntington's disease, Parkinson's disease, or multiple sclerosis (MS).

In certain instances, the subject is experiencing one or more types of pain, and the conjugate is administered to treat or reduce the pain. General examples of pain include acute pain and chronic pain. In some instances, the pain has at least one CNS component. Specific examples of pain include nociceptive pain, neuropathic pain, breakthrough pain, incident pain, phantom pain, inflammatory pain including arthritic pain, or any combination thereof.

In particular instances, the pain is nociceptive pain, optionally visceral, deep somatic, or superficial somatic pain. Nociceptive pain is usually caused by stimulation of peripheral nerve fibers that respond to stimuli approaching or exceeding harmful intensity (nociceptors), and may be classified according to the mode of noxious stimulation; for example, "thermal" (e.g., heat or cold), "mechanical" (e.g., crushing, tearing, cutting) and "chemical." Visceral structures are highly sensitive to stretch, ischemia and inflammation, but relatively insensitive to other stimuli such as burning and cutting. Visceral pain is most often diffuse, difficult to locate, and is sometimes referred to as having a distant, or superficial, structure. Visceral pain can be accompanied by nausea and vomiting, and is sometimes described as sickening, deep, squeezing, and dull. Deep somatic pain is usually initiated by the stimulation of nociceptors in ligaments, tendons, bones, blood vessels, fasciae and muscles, and is often characterized as a dull, aching, or poorly localized pain. Examples include sprains and broken bones. Superficial pain is mainly initiated by activation of nociceptors in the skin or other superficial tissue, and is sharp, well-defined and clearly located. Examples of injuries that produce superficial somatic pain include wounds and burns.

Neuropathic pain results from damage or disease affecting the somatosensory system. It may be associated with abnormal sensations called dysesthesia, and pain produced by normally non-painful stimuli (allodynia). Neuropathic pain may have continuous and/or episodic (paroxysmal) components, the latter being compared to an electric shock. Common characteristics of neuropathic pain include burning or coldness, "pins and needles" sensations, numbness, and itching. Neuropathic pain may result from disorders of the peripheral nervous system or the central nervous system (e.g., brain, spinal cord). Neuropathic pain may be characterized as peripheral neuropathic pain, central neuropathic pain, or mixed (peripheral and central) neuropathic pain.

Central neuropathic pain is found in spinal cord injury, multiple sclerosis, and strokes. Additional causes of neuropathic pain include diabetic neuropathy, herpes zoster infection, HIV-related neuropathies, nutritional deficiencies, toxins, remote manifestations of malignancies, immune mediated disorders, and physical trauma to a nerve trunk. Neuropathic pain also associates with cancer, mainly as a direct result of a cancer or tumor on peripheral or central nerves (e.g., compression by a tumor), or as a side effect of chemotherapy, radiation injury, or surgery.

In some instances, the pain is breakthrough pain. Breakthrough pain is pain that comes on suddenly for short periods of time and is not alleviated by the subject's normal pain management regimen. It is common in cancer patients who often have a background level of pain controlled by medications, but whose pain periodically "breaks through" the medication. Hence, in certain instances, the subject is taking pain medication, and is optionally a subject with cancer pain, e.g., neuropathic cancer pain.

In certain instances, the pain is incident pain, a type of pain that arises as a result of an activity. Examples include moving an arthritic or injured joint, and stretching a wound.

In specific instances, the pain is osteoarthritis, low back pain (or lumbago), including acute, sub-acute, and chronic low back pain (CLBP), bone cancer pain, or interstitial cystitis.

Osteoarthritis (OA), also referred to as degenerative arthritis or degenerative joint disease or osteoarthrosis, is a group of mechanical abnormalities involving degradation of joints, including articular cartilage and subchondral bone. Symptoms of OA may include joint pain, tenderness, stiffness, locking, and sometimes an effusion. OA may be initiated by variety of causes, including hereditary, developmental, metabolic, and mechanical causes, most of which lead to the loss of cartilage. When bone surfaces become less well protected by cartilage, bone may be exposed and damaged. As a result of decreased movement secondary to pain, regional muscles may atrophy, and ligaments may become increasingly lax. Particular examples include osteoarthritis of the knee, and osteoarthritis of the hip.

Interstitial cystitis, or bladder pain syndrome, is a chronic, oftentimes severely debilitating disease of the urinary bladder. Of unknown cause, it is characterized, for instance, by pain associated with the bladder, pain associated with urination (dysuria), urinary frequency (e.g., as often as every 10 minutes), urgency, and/or pressure in the bladder and/or pelvis.

Certain methods and compositions include further combination therapies for treating pain. For instance, a subject with pain may be further administered one or more pain medications, including analgesics and anesthetics. Exemplary analgesics include, without limitation, paracetamol/acetaminophen; non-steroidal anti-inflammatory drugs (NSAIDS) such as salicylates (e.g., aspirin), propionic acid derivatives (e.g., ibuprofen, naproxen), acetic acid derivatives (e.g., indomethacin), enolic acid derivatives, fenamic acid derivatives, and selective COX-2 inhibitors; opiates/opioids and morphinomimetics such as morphine, buprenorphine, codeine, oxycodone, oxymorphone, hydrocodone, dihydromorphine, dihydrocodeine, levorphanol, methadone, dextropropoxyphene, pentazocine, dextromoramide, meperidine (or pethidin), tramadol, noscapine, nalbuphine, pentacozine, papverine, papaveretum, alfentanil, fentanyl, remifentanil, sufentanil, and etorphine; and other agents, such as flupirtine, carbamazepine, gabapentin, and pregabalin, including any combination of the foregoing.

Methods for identifying subjects with one or more of the diseases or conditions described herein are known in the art.

For *in vivo* use, for instance, for the treatment of human disease or testing, the (a) polypeptides that comprise an immunoglobulin Fc region (i.e., an Fc region-containing polypeptide) and/or (b) conjugates described herein are generally incorporated into one or more pharmaceutical or therapeutic compositions prior to administration. In certain embodiments, a pharmaceutical or therapeutic composition comprises one or more of (a) and/or (b), as described herein, optionally in combination with at least one pharmaceutically-acceptable or physiologically-acceptable carrier or excipient.

To prepare a pharmaceutical composition, an effective or desired amount of one or more Fc region-containing polypeptides and/or conjugates is mixed with any pharmaceutical carrier(s) or excipient known to those skilled in the art to be suitable for the particular mode of administration. A pharmaceutical carrier may be liquid, semi-liquid or solid. Solutions or suspensions used for parenteral, intradermal, subcutaneous or topical application may include, for example, a sterile diluent (such as water), saline solution (*e.g*., phosphate buffered saline; PBS), fixed oil, polyethylene glycol, glycerin, propylene glycol or other synthetic solvent; antimicrobial agents (such as benzyl alcohol and methyl parabens); antioxidants (such as ascorbic acid and sodium bisulfite) and chelating agents (such as ethylenediaminetetraacetic acid (EDTA)); buffers (such as acetates, citrates and phosphates). If administered intravenously (e.g., by IV infusion), suitable carriers include physiological saline or phosphate buffered saline (PBS), and solutions containing thickening and solubilizing agents, such as glucose, polyethylene glycol, polypropylene glycol and mixtures thereof.

Administration of Fc region-containing polypeptides and/or conjugates described herein, in pure form or in an appropriate pharmaceutical composition, can be carried out via any of the accepted modes of administration of agents for serving similar utilities. The pharmaceutical compositions can be prepared by combining a Fc region-containing polypeptide and/or a conjugate with an appropriate physiologically acceptable carrier, diluent or excipient, and may be formulated into preparations in solid, semi-solid, liquid or gaseous forms, such as tablets, capsules, powders, granules, ointments, solutions, suppositories, injections, inhalants, gels, microspheres, and aerosols. In addition, other pharmaceutically active ingredients (including other small molecules as described elsewhere herein) and/or suitable excipients such as salts, buffers and stabilizers may, but need not, be present within the composition.

Administration may be achieved by a variety of different routes, including oral, parenteral, nasal, intravenous, intradermal, subcutaneous or topical. Preferred modes of administration depend upon the nature of the condition to be treated or prevented. Particular embodiments include systemic administration, for example, by intravenous (IV) infusion.

Carriers can include, for example, pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as polysorbate 20 (TWEEN^{™}) polyethylene glycol (PEG), and poloxamers (PLURONICS^{™}), and the like.

In certain conjugate compositions, the BBB-moiety and the agent are each, individually or as a pre-existing conjugate, bound to or encapsulated within a particle, *e.g*., a nanoparticle, bead, lipid formulation, lipid particle, or liposome, *e.g*., immunoliposome. For instance, in particular embodiments, a BBB-moiety is bound to the surface of a particle, and an agent is bound to the surface of the particle and/or encapsulated within the particle. In some of these and related embodiments, the BBB-moiety and the agent are covalently or operatively linked to each other only via the particle itself (*e.g*., nanoparticle, liposome), and are not covalently linked to each other in any other way; that is, they are bound individually to the same particle. In other embodiments, the BBB-moiety and the agent are first covalently or non-covalently conjugated to each other, as described herein (*e.g*., via a linker molecule), and are then bound to or encapsulated within a particle (*e.g*., liposome, nanoparticle). In specific embodiments, the particle is a liposome, and the composition comprises one or more BBB-moieties, one or more agents, and optionally one or more Fc region-containing polypeptides, and a mixture of lipids to form a liposome (*e.g*., phospholipids, mixed lipid chains with surfactant properties). In some aspects, the BBB-moiety and the agent are individually mixed with the lipid/liposome mixture, such that the formation of liposome structures operatively links the BBB-moiety and the agent without the need for covalent conjugation. In other aspects, the BBB-moiety and the agent are first covalently or non-covalently conjugated to each other, as described herein, and then mixed with lipids to form a liposome. The BBB-moiety, the agent, or the conjugate may be entrapped in microcapsules prepared, for example, by coacervation techniques or by interfacial polymerization (for example, hydroxymethylcellulose or gelatin-microcapsules and poly-(methylmethacylate)microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980). The particle(s) or liposomes may further comprise other therapeutic or diagnostic agents, such as cytotoxic agents.

The precise dosage and duration of treatment is a function of the disease being treated and may be determined empirically using known testing protocols or by testing the compositions in model systems known in the art and extrapolating therefrom. Controlled clinical trials may also be performed. Dosages may also vary with the severity of the condition to be alleviated. A pharmaceutical composition is generally formulated and administered to exert a therapeutically useful effect while minimizing undesirable side effects. The composition may be administered one time, or may be divided into a number of smaller doses to be administered at intervals of time. For any particular subject, specific dosage regimens may be adjusted over time according to the individual need.

Typical routes of administering these and related pharmaceutical compositions thus include, without limitation, oral, topical, transdermal, inhalation, parenteral, sublingual, buccal, rectal, vaginal, and intranasal. The term parenteral as used herein includes subcutaneous injections, intravenous, intramuscular, intrasternal injection or infusion techniques. Pharmaceutical compositions according to certain embodiments of the present invention are formulated so as to allow the active ingredients contained therein to be bioavailable upon administration of the composition to a patient. Compositions that will be administered to a subject or patient may take the form of one or more dosage units, where for example, a tablet may be a single dosage unit, and a container of a herein described Fc region-containing polypeptides and/or conjugate in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). The composition to be administered will typically contain a therapeutically effective amount of a conjugate described herein, for treatment of a disease or condition of interest.

A pharmaceutical composition may be in the form of a solid or liquid. In one embodiment, the carrier(s) are particulate, so that the compositions are, for example, in tablet or powder form. The carrier(s) may be liquid, with the compositions being, for example, an oral oil, injectable liquid or an aerosol, which is useful in, for example, inhalatory administration. When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent. When the pharmaceutical composition is in the form of a capsule, for example, a gelatin capsule, it may contain, in addition to materials of the above type, a liquid carrier such as polyethylene glycol or oil.

The pharmaceutical composition may be in the form of a liquid, for example, an elixir, syrup, solution, emulsion or suspension. The liquid may be for oral administration or for delivery by injection, as two examples. When intended for oral administration, preferred composition contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

A liquid pharmaceutical composition intended for either parenteral or oral administration should contain an amount of a conjugate and/or an Fc region-containing polypeptide such that a suitable dosage will be obtained. Typically, this amount is at least 0.01% of the agent of interest in the composition. When intended for oral administration, this amount may be varied to be between 0.1 and about 70% of the weight of the composition. Certain oral pharmaceutical compositions contain between about 4% and about 75% of the agent of interest. In certain embodiments, pharmaceutical compositions and preparations according to the present invention are prepared so that a parenteral dosage unit contains between 0.01 to 10% by weight of the agent of interest prior to dilution.

The pharmaceutical composition may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device.

The pharmaceutical composition may be intended for rectal administration, in the form, for example, of a suppository, which will melt in the rectum and release the drug. The composition for rectal administration may contain an oleaginous base as a suitable nonirritating excipient. Such bases include, without limitation, lanolin, cocoa butter, and polyethylene glycol.

The pharmaceutical composition may include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule. The pharmaceutical composition in solid or liquid form may include an agent that binds to the conjugate or agent and thereby assists in the delivery of the compound. Suitable agents that may act in this capacity include monoclonal or polyclonal antibodies, one or more proteins or a liposome.

The pharmaceutical composition may consist essentially of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols may be delivered in single phase, bi-phasic, or tri-phasic systems in order to deliver the active ingredient(s). Delivery of the aerosol includes the necessary container, activators, valves, subcontainers, and the like, which together may form a kit. One of ordinary skill in the art, without undue experimentation may determine preferred aerosols.

The compositions described herein may be prepared with carriers that protect the Fc region-containing polypeptides and/or conjugates against rapid elimination from the body, such as time release formulations or coatings. Such carriers include controlled release formulations, such as, but not limited to, implants and microencapsulated delivery systems, and biodegradable, biocompatible polymers, such as ethylene vinyl acetate, polyanhydrides, polyglycolic acid, polyorthoesters, polylactic acid and others known to those of ordinary skill in the art.

The pharmaceutical compositions may be prepared by methodology well known in the pharmaceutical art. For example, a pharmaceutical composition intended to be administered by injection may comprise one or more of salts, buffers and/or stabilizers, with sterile, distilled water so as to form a solution. A surfactant may be added to facilitate the formation of a homogeneous solution or suspension. Surfactants are compounds that non-covalently interact with the Fc region-containing polypeptide and/or conjugate so as to facilitate dissolution or homogeneous suspension of the same in the aqueous delivery system.

The compositions may be administered in a therapeutically effective amount, which will vary depending upon a variety of factors including the activity of the specific compound employed; the metabolic stability and length of action of the compound; the age, body weight, general health, sex, and diet of the patient; the mode and time of administration; the rate of excretion; the drug combination; the severity of the particular disorder or condition; and the subject undergoing therapy. Generally, a therapeutically effective daily dose is (for a 70 kg mammal) from about 0.001 mg/kg (*i.e*., ∼ 0.07 mg) to about 100 mg/kg (*i.e*., ∼ 7.0 g); preferably a therapeutically effective dose is (for a 70 kg mammal) from about 0.01 mg/kg (*i.e*., ∼ 0.7 mg) to about 50 mg/kg (*i.e*., ∼ 3.5 g); more preferably a therapeutically effective dose is (for a 70 kg mammal) from about 1 mg/kg (*i.e*., ∼ 70 mg) to about 25 mg/kg (*i.e*., ∼ 1.75 g).

Compositions described herein may also be administered simultaneously with, prior to, or after administration of one or more other therapeutic agents, as described herein. For instance, in one embodiment, the Fc region-containing polypeptides and/or conjugate is administered with an anti-inflammatory agent. Anti-inflammatory agents or drugs include, but are not limited to, steroids and glucocorticoids (including betamethasone, budesonide, dexamethasone, hydrocortisone acetate, hydrocortisone, hydrocortisone, methylprednisolone, prednisolone, prednisone, triamcinolone), nonsteroidal anti-inflammatory drugs (NSAIDS) including aspirin, ibuprofen, naproxen, methotrexate, sulfasalazine, leflunomide, anti-TNF medications, cyclophosphamide and mycophenolate.

Such combination therapy may include administration of a single pharmaceutical dosage formulation, which contains a compound of the invention (i.e., conjugate, an Fc-region containing polypeptide) and one or more additional active agents, as well as administration of compositions comprising conjugates of the invention and each active agent in its own separate pharmaceutical dosage formulation. For example, a conjugate and/or Fc-region containing polypeptide as described herein and optionally another active agent can be administered to the patient together in a single oral dosage composition such as a tablet or capsule, or each agent administered in separate oral dosage formulations. Similarly, a conjugate and/or Fc-region containing polypeptide as described herein and optionally another active agent can be administered to the patient together in a single parenteral dosage composition such as in a saline solution or other physiologically acceptable solution, or each agent administered in separate parenteral dosage formulations. Where separate dosage formulations are used, the compositions comprising conjugates and one or more additional active agents can be administered at essentially the same time, i.e., concurrently, or at separately staggered times, i.e., sequentially and in any order; combination therapy is understood to include all these regimens.

Also included are kits, for example, patient care kits, comprising one or more containers filled with one or more of the therapeutic compositions, Fc region-containing polypeptides and/or conjugates described herein. In some embodiments, the kits include written instructions on how to use such compositions, for example, in the treatment of one or more diseases.

Certain embodiments therefore include a patient care kit, comprising: (a) a polypeptide that comprises an immunoglobulin Fc region; and (b) a conjugate, as described herein. In some kits, (a) and (b) are in separate compositions, and are optionally defined as described herein. In some kits, (a) and (b) are in the same composition, optionally as a pharmaceutical or therapeutic composition as described herein.

The kits herein may also include a one or more additional therapeutic agents or other components suitable or desired for the indication being treated,. An additional therapeutic agent may be contained in a second container, if desired. Examples of additional therapeutic agents include, but are not limited to anti-inflammatory agents, antibacterial agents, antiviral agents, etc.

The kits herein can also include one or more syringes (e.g., injectable syringes) or other components necessary or desired to facilitate an intended mode of delivery (e.g., stents, implantable depots, etc.).

The various embodiments described herein can be combined to provide further embodiments. All of the U.S. patents, U.S. patent application publications, U.S. patent application, foreign patents, foreign patent application and non-patent publications referred to in this specification and/or listed in the Application Data Sheet are incorporated herein by reference, in their entirety. Aspects of the embodiments can be modified, if necessary to employ concepts of the various patents, application and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

## Claims

1. A pharmaceutical composition, comprising:
(a) a polypeptide that comprises an immunoglobulin Fc region; and
(b) a conjugate, comprising a blood-brain barrier (BBB)-transport moiety linked to (i) a therapeutic antibody or antigen binding fragment thereof or (ii) a therapeutic Fc-fusion polypeptide.

2. The pharmaceutical composition of claim 1, wherein (a) is an antibody, which optionally differs from the antibody of part (b), preferably wherein the variable region of the antibody of (a) does not substantially bind to a human antigen.

3. The pharmaceutical composition of claim 1 or 2, wherein (a) comprises a human immunoglobulin Fc region, preferably wherein the human immunoglobulin Fc region comprises, consists, or consists essentially of a sequence from Table A1, including combinations, variants, and fragments thereof more preferably wherein the human immunoglobulin Fc region comprises, consists, or consists essentially of an Fc region derived from human IgA1, human IgA2, human IgD, human IgE, human IgG1, human IgG2, human IgG3, human IgG4, or human IgM, optionally as defined by the sequences in Table A1.

4. The pharmaceutical composition of any one of the preceding claims, wherein (b) is a human IgA1 antibody, a human IgA2 antibody, a human IgD antibody, a human IgE antibody, a human IgG1 antibody, a human IgG2 antibody, a human IgG3 antibody, a human IgG4 antibody, or a human IgM antibody, or a fragment thereof that comprise an Fc region or a portion of an Fc region.

5. The pharmaceutical composition of any one of the preceding claims, wherein the BBB-transport moiety is selected from one or more of:
a p97 (melanotransferrin) polypeptide,
a Receptor Associated Protein (RAP),
an aprotinin peptide or an analog thereof,
a protein transduction domain (PTD),
a human low-density lipoprotein receptor (hLDLR) binding peptide or an analog thereof,
an antibody or natural ligand that binds to a BBB-associated receptor, and
glutathione (GSH).

6. The pharmaceutical composition of claim 5,
wherein the p97 polypeptide comprises, consists, or consists essentially of a sequence in Table B1 or B2, or an active variant or fragment thereof, preferably wherein the p97 polypeptide comprises DSSHAFTLDELR (SEQ ID NO:14) or is a soluble human p97 polypeptide;
or wherein the RAP comprises or consists of a sequence in Table B3, or an active variant or fragment thereof;
or wherein the aprotinin peptide comprises or consists of a sequence in Table B4, or an active variant or fragment thereof;
or wherein the PTD comprises or consists of a sequence in Table B5, or an active variant or fragment;
or wherein the hLDLR binding peptide comprises or consists of a sequence in Table B6, or an active variant or fragment;
or wherein the BBB-associated receptor is selected from one or more of the insulin receptor, the transferrin receptor, the leptin receptor, lipoprotein receptors such as the lipoprotein receptor-related protein (LRP-1) receptor, insulin-like growth factor (IGF) receptors such as IGF1R and IGF2R, the low-density lipoprotein receptor, the diptheria toxin receptor, and TMEM 30A (Flippase);
or wherein the BBB-associated receptor ligand is selected from one or more of insulin, transferrin and transferrin fragments, lactoferrin and lactoferrin fragments, apolipoprotein A (Apo A), apolipoprotein B (Apo B), apolipoprotein E (Apo E), and diptheria toxin (including non-toxic mutants thereof such as CRM45 and CRM197).

7. The pharmaceutical composition of any one of the preceding claims, wherein the antibody or antigen-binding fragment of (b) comprises an immunoglobulin Fc region.

8. The pharmaceutical composition of any one of the preceding claims, wherein the antibody or antigen-binding fragment of (b) specifically binds to anyone of the following:
(I) specifically binds to one or more of human Her2/neu, Her1/EGFR, TNF-α, B7H3 antigen, CD20, VEGF, CD52, CD33, CTLA-4, tenascin, alpha-4 (α4) integrin, IL-23, amyloid-β such as Aβ₍₁₋₄₂₎, Huntingtin, CD25, nerve growth factor (NGF), TrkA, or *α*-synuclein;
(II) specifically binds to a cell surface receptor;
(III) specifically binds to a ligand of a cell surface receptor;
(IV) specifically binds to a cancer-associated antigen,
preferably wherein (IV) the cancer-associated antigen, or cancer antigen, is selected from one or more of human Her2/neu, Her1/EGF receptor (EGFR), Her3, A33 antigen, B7H3, CD5, CD19, CD20, CD22, CD23 (IgE Receptor), C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin,
more preferably wherein the antibody is selected from trastuzumab, cetuximab, daclizumab, tanezumab, 3F8, 8H9, abagovomab, adecatumumab, afutuzumab, alemtuzumab, alacizumab (pegol), amatuximab, apolizumab, bavituximab, bectumomab, belimumab, bevacizumab, bivatuzumab (mertansine), brentuximab vedotin, cantuzumab (mertansine), cantuzumab (ravtansine), capromab (pendetide), catumaxomab, citatuzumab (bogatox), cixutumumab, clivatuzumab (tetraxetan), conatumumab, dacetuzumab, dalotuzumab, detumomab, drozitumab, ecromeximab, edrecolomab, elotuzumab, enavatuzumab, ensituximab, epratuzumab, ertumaxomab, etaracizumab, farletuzumab, FBTA05, figitumumab, flanvotumab, galiximab, gemtuzumab, ganitumab, gemtuzumab (ozogamicin), girentuximab, glembatumumab (vedotin), ibritumomab tiuxetan, icrucumab, igovomab, indatuximab ravtansine, intetumumab, inotuzumab ozogamicin, ipilimumab (MDX-101), iratumumab, labetuzumab, lexatumumab, lintuzumab, lorvotuzumab (mertansine), lucatumumab, lumiliximab, mapatumumab, matuzumab, milatuzumab, mitumomab, mogamulizumab, moxetumomab (pasudotox), nacolomab (tafenatox), naptumomab (estafenatox), narnatumab, necitumumab, nimotuzumab, nivolumab, Neuradiab^{®} (with or without radioactive iodine), NR-LU-10, ofatumumab, olaratumab, onartuzumab, oportuzumab (monatox), oregovomab, panitumumab, patritumab, pemtumomab, pertuzumab, pritumumab, racotumomab, radretumab, ramucirumab, rilotumumab, rituximab, robatumumab, samalizumab, sibrotuzumab, siltuximab, tabalumab, taplitumomab (paptox), tenatumomab, teprotumumab, TGN1412, ticilimumab, tremelimumab, tigatuzumab, TNX-650, tositumomab, TRBS07, tucotuzumab (celmoleukin), ublituximab, urelumab, veltuzumab, volociximab, votumumab, and zalutumumab, and fragments, variants, and derivatives of the foregoing;
(V) specifically binds to an antigen associated with at least one nervous system disorder;
(VI) specifically binds to an antigen associated with pain;
(VII) specifically binds to an antigen associated with autoimmune disorder, optionally an autoimmune disorder of the nervous system or CNS; or
(VIII) specifically binds to a pro-inflammatory cytokine or chemokine, or a receptor thereof,
preferably wherein the pro-inflammatory cytokine or chemokine is selected from TNF-α, TNF-β, FasL, CD27L, CD30L, CD40L, Ox40L, 4-1BBL, TRAIL, TWEAK, and Apo3L, IL-1α, IL-1β, IL-2, interferon-y (IFN-γ), IFN-α/β, IL-6, IL-8, IL-12, IL-15, IL-17, IL-18, IL-21, LIF, CCL5, GROα, MCP-1, MIP-1α, MIP-1β, macrophage colony stimulating factor (MCSF), granulocyte macrophage colony stimulating factor (GM-CSF), CXCL2, and CCL2,
more preferably wherein the antibody is adalimumab (Humira^{®}), certolizumab pegol (Cimzia^{®}), golimumab (Cimzia^{®}), infliximab (Remicade^{®}), D2E7, CDP 571, or CDP 870, or an antigen-binding fragment or variant thereof,
or wherein the receptor is selected from TNF receptor (TNFR), an IL-1 receptor (IL-1R), and an IL-6 receptor (IL-6R).

9. The pharmaceutical composition of claim 8, wherein the antigen of (V), (VI) or (VII) is selected from one or more of alpha-4 (α4) integrin, CD20, CD52, IL-12, IL-23, the p40 subunit of IL-12 and IL-23, and the axonal regrowth and remyelination inhibitors Nogo-A and LINGO, IL-23, amyloid-β optionally Aβ(1-42), Huntingtin, CD25, nerve growth factor (NGF), neurotrophic tyrosine kinase receptor type 1 (TrkA; the high affinity catalytic receptor for NGF), and α-synuclein.

10. The pharmaceutical composition of any one of the preceding claims, wherein the Fc-fusion polypeptide is selected from etanercept, abatercept, aflibercept, alefacept, belatacept, rilonacept, and romiplastin.

11. The pharmaceutical composition of any one of the preceding claims, which is substantially endotoxin-free.

12. The pharmaceutical composition of any one of the preceding claims for use in therapeutic treatment.

13. The pharmaceutical composition for use according to claim 12,
wherein (a) and (b) are administered systemically, optionally intravenously;
or wherein (a) and (b) are administered separately, wherein either (a) is administered prior to (b), preferably (a) is administered about, at least about, or no more than about 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 200, 220, 240, 260, 280, or 300 minutes before (b), in particular (a) is administered about, at least about, or no more than about 1, 2, 3, 4, 5, 6, 7, 8, 9, 10, 11, 12, 16, 24, 30, 36, 42, 48, 60, 72, 84, or 96 hours before (b);or (a) and (b) are administered together.

14. The pharmaceutical composition for use according to claims 12 or 13, wherein systemic administration of (a) and (b) increases delivery of (a) across the BBB and into tissues of the CNS relative to systemic administration of (a) alone.

15. The pharmaceutical composition for use according to anyone of claims 12 to 14 for anyone of the following treatments:
for treating cancer of the central nervous system (CNS), optionally the brain, particularly for treating primary cancer of the CNS, optionally the brain;
for treating a metastatic cancer of the CNS, optionally the brain;
for treating a glioma, meningioma, pituitary adenoma, vestibular schwannoma, primary CNS lymphoma, neuroblastoma, or primitive neuroectodermal tumor (medulloblastoma), wherein optionally the glioma is an astrocytoma, oligodendroglioma, ependymoma, or a choroid plexus papilloma;
for treating glioblastoma multiforme, optionally wherein the glioblastoma multiforme is a giant cell gliobastoma or a gliosarcoma;
for treating a degenerative or autoimmune disorder of the central nervous system (CNS), optionally wherein the degenerative or autoimmune disorder of the CNS is Alzheimer's disease, Huntington's disease, Parkinson's disease, or multiple sclerosis (MS);
for treating pain , optionally wherein the pain is acute pain, chronic pain, neuropathic pain, and/or central pain;
for treating an inflammatory condition,
optionally wherein the inflammatory condition is associated with a cancer of the CNS, optionally a malignant meningitis, or wherein the inflammatory condition has a central nervous system component,
preferably wherein the inflammatory condition is one or more of meningitis, myelitis, encephalomyelitis, arachnoiditis, sarcoidosis, granuloma, drug-induced inflammation, Alzheimer's disease, stroke, HIV-dementia, encephalitis, parasitic infection, an inflammatory demyelinating disorder, a CD8+ T Cell-mediated autoimmune disease of the CNS, Parkinson's disease, myasthenia gravis, motor neuropathy, Guillain-Barre syndrome, autoimmune neuropathy, Lambert-Eaton myasthenic syndrome, paraneoplastic neurological disease, paraneoplastic cerebellar atrophy, non-paraneoplastic stiff man syndrome, progressive cerebellar atrophy, Rasmussen's encephalitis, amyotrophic lateral sclerosis, Sydeham chorea, Gilles de la Tourette syndrome, autoimmune polyendocrinopathy, dysimmune neuropathy, acquired neuromyotonia, arthrogryposis multiplex, optic neuritis, stroke, traumatic brain injury (TBI), spinal stenosis, acute spinal cord injury, and spinal cord compression, in particular wherein the inflammatory condition is associated with an infection of the central nervous system,
wherein the infection optionally is a bacterial infection caused by one or more of *group B streptococci* (*e.g.,* subtypes III), *Streptococcus pneumoniae* (*e.g.,* serotypes 6, 9, 14, 18 and 23), *Escherichia coli* (*e.g.,* carrying K1 antigen), *Listeria monocytogenes* (*e.g.,* serotype IVb), neisserial infection such as *Neisseria meningitidis* (meningococcus), staphylococcal infection, heamophilus infection such as *Haemophilus influenzae* type B, *Klebsiella, Mycobacterium tuberculosis, Treponema pallidum,* or *Borrelia burgdorferi,* , or wherein the infection optionally is a viral infection caused by one or more of an enterovirus, herpes simplex virus type 1 or 2, human T-lymphotrophic virus, varicella zoster virus, mumps virus, human immunodeficiency virus (HIV), or lymphocytic choriomeningitis virus (LCMV).

16. A patient care kit, comprising:
(a) a polypeptide that comprises an immunoglobulin Fc region; and
(b) a conjugate, comprising a blood-brain barrier (BBB)-transport moiety linked to (i) a therapeutic antibody or antigen binding fragment thereof or (ii) a therapeutic Fc-fusion polypeptide.

17. The patient care kit of claim 16, wherein (a) and (b) are in separate pharmaceutical compositions, and are optionally defined according to any one of claims 1-11; or
wherein (a) and (b) are in the same composition, optionally as a pharmaceutical composition according to any one of claims 1-11.
